# EUROPEAN PATENT SPECIFICATION

(11) **EP 1 068 339 B1**
(45) Date of publication and mention of the grant of the patent: **23.07.2008**
(21) Application number: 99911542.1
(22) Date of filing: 29.03.1999
(51) Int. Cl.: C12N 15/63, C12N 15/31, C07K 14/245, C12N 15/62, C12P 21/02

(54) **COMPOSITIONS AND METHODS FOR PROTEIN SECRETION**
ZUSAMMENSETZUNGEN UND VERFAHREN ZUR PROTEIN-SEKRETION
COMPOSITIONS ET METHODES DE SECRETION PROTEIQUE

(30) Priority: 01.04.1998 US 53197; 28.05.1998 US 85761
(43) Date of publication of application: 17.01.2001
(73) Proprietor: The Governors of the University of Alberta, Edmonton, Alberta T6G 2J9 (CA)
(72) Inventor: WEINER, Joel, Hirsch, Edmonton, Alberta T6J 2C2 (CA); TURNER, Raymond, Joseph, Calgary, Alberta T2K 0W1 (CA)
(74) Representative: Harding, Charles Thomas
(86) International application number: PCT/CA1999/000272
(87) International publication number: WO 1999/051753

(56) References cited:
- SETTLES, M. ET AL.: "Sec-independent protein translocation by the maize Hcf106 protein" SCIENCE., vol. 278, 21 November 1997 (1997-11-21), pages 1467-1470, XP002113153 cited in the application
- NIVIERE, V. ET AL.: "Site-directed mutagenesis of the hydrogenase signal peptide consensus box prevents export of a beta-lactamase fusion protein" JOURNAL OF GENERAL MICROBIOLOGY, vol. 138, 1992, pages 2173-2183, XP002113154 ISSN: 0001-2961
- BERKS, B.: "A common export pathway for proteins binding redox cofactors ?" MOLECULAR MICROBIOLOGY., vol. 22, 1996, pages 393-404, XP002113155 cited in the application
- SANTINI C L ET AL: "A novel sec - independent periplasmic protein translocation pathway in Escherichia coli." EMBO JOURNAL, (1998 JAN 2) 17 (1) 101-12., XP002113156
- WEINER J H ET AL: "A novel and ubiquitous system for membrane targeting and secretion of cofactor-containing proteins." CELL, (1998 APR 3) 93 (1) 93-101., XP002113157
- SARGENT F ET AL: "Overlapping functions of components of a bacterial Sec - independent protein export pathway." EMBO JOURNAL, (1998 JUL 1) 17 (13) 3640-50., XP002113158
- DALBEY R E ET AL: "Protein translocation into and across the bacterial plasma membrane and the plant thylakoid membrane" TIBS TRENDS IN BIOCHEMICAL SCIENCES, vol. 24, no. 1, January 1999 (1999-01), page 17-22 XP004155514 ISSN: 0968-0004
- SAMBASIVARAO ET AL.: "Investigation of Escherichia coli dimethyl sulfoxide reductase assembly and processing in strains defective for the sec-independent protein translocation system membrane targeting and translocation" J. BIOL. CHEM., vol. 276, no. 23, 8 June 2001 (2001-06-08), pages 20167-20174,

## Description

### FIELD OF THE INVENTION

The present invention relates to compositions and methods for secretion of functional proteins in a soluble form by host cells. In particular, the invention relates to proteins involved in targeting expression of a protein of interest extracellularly and to the periplasm, thus facilitating generation of a functional soluble protein.

### BACKGROUND OF THE INVENTION

Proteins having clinical or industrial value may be obtained using techniques which facilitate their synthesis in bacterial or in eukaryotic cell cultures. However, once synthesized, there are often problems in recovering these recombinant proteins in substantial yields and in a useful form. For example, recombinant proteins expressed in bacteria often accumulate in the bacterial cytoplasm as insoluble aggregates known as inclusion bodies [Marston, (1986) Biochem. J. 240:1-12; Schein (1989) Biotechnology 7:1141-1149]. Similarly, recombinant transmembrane proteins which contain both hydrophobic and hydrophilic regions are intractable to solubilization.

While transmembrane recombinant proteins and recombinant proteins which are expressed in the cytoplasm may be solubilized by use of strong denaturing solutions (e. g., urea, guanidium salts, detergents, Triton, SDS detergents, etc.), solubilization efficiency is nevertheless variable and there is no general method of solubilization which works for most proteins. Additionally, many proteins which are present at high concentrations precipitate out of solution when the solubilizing agent is removed. Yet a further drawback to solubilization of recombinant proteins is that denaturing chemicals (e. g., guanidium salts and urea) contain reactive primary amines which swamp those of the protein, thus interfering with the protein's reactive amine groups.

Thus, what is needed is a method for producing soluble proteins.

### SUMMARY OF THE INVENTION

The present invention provides a recombinant polypeptide comprising at least a portion of amino acid sequence of SEQ ID NO: 47 and at least a portion of amino acid sequence of SEQ ID NO: 7.

This invention further provides an isolated nucleic acid sequence encoding at least a portion of amino acid sequence of SEQ ID NO: 47 and at least a portion of amino acid sequence of SEQ ID NO: 7. In one preferred embodiment, the nucleic acid sequence is contained on a recombinant expression vector. In a more preferred embodiment, the expression vector is contained within a host cell.

Also provided by the present invention is a nucleic acid sequence that hybridizes under stringent conditions to a nucleic acid sequence encoding an amino acid sequence of SEQ ID NO: 7.

The invention additionally provides a method for expressing a nucleotide sequence of interest in a host cell to produce a soluble polypeptide sequence, the nucleotide sequence of interest when expressed in the absence of an operably linked nucleic acid sequence encoding a twin-arginine signal amino acid sequence produces an insoluble polypeptide, comprising: a) providing: i) the nucleotide sequence of interest encoding the insoluble polypeptide, wherein the insoluble polypeptide comprises a cofactor; ii) the nucleic acid sequence encoding the twin-arginine signal amino acid sequence; and iii) the host cell, wherein the host cell comprises at least a portion of an amino acid sequence of SEQ ID NO: 47 and at least a portion of an amino acid sequence of SEQ ID NO: 7 wherein both said at least a portion of the amino acid sequences together have a biological activity comprising targeting of a fully-folded protein which contains a twin-arginine signal amino acid sequence to a cell membrane and translocating the fully-folded protein which contains twin-arginine signal amino acid sequence to a periplasm and/or translocating the fully-folded protein which contains a twin-arginine signal amino acid sequence to an extracellular space; b) operably linking the - nucleotide sequence of interest to the nucleic acid sequence encoding the twin-arginine signal amino acid sequence to produce a linked polynucleotide sequence; and c) introducing the linked polynucleotide sequence into the host cell under conditions such that the fused polynucleotide sequence is expressed and the soluble polypeptide is produced.

Without intending to limit the location of the insoluble polypeptide, in one preferred embodiment, the insoluble polypeptide is comprised in an inclusion body. In another preferred embodiment, the insoluble polypeptide comprises a cofactor. In a more preferred embodiment, the cofactor is selected from the group consisting of iron-sulfur clusters, molybdopterin, polynuclear copper, tryptophan, tryptophylquinone, and flavin adenine dinucleotide.

Without limiting the location of the soluble polypeptide to any particular location, in one preferred embodiment, the soluble polypeptide is comprised in periplasm of the host cell. In an alternative preferred embodiment, the host cell is cultured in medium, and the soluble polypeptide is contained in the medium.

The methods of the invention are not intended to be limited to any particular cell. However, in one preferred embodiment, the cell is *Escherichia coli*. In a more preferred embodiment, the *Escherichia coli* cell is D-43.

It is not intended that the invention be limited to a particular twin-arginine signal amino acid sequence. In a preferred embodiment, the twin-arginine signal amino acid sequence is selected from the group consisting of SEQ ID NO: 41 and SEQ ID NO: 42.

The invention further provides a method for expressing a nucleotide sequence of interest encoding an amino acid sequence of interest in a host cell, comprising: a) providing: i) the host cell; ii) the nucleotide sequence of interest; iii) a first nucleic acid sequence encoding twin-arginine signal amino acid sequence; and iv) a second nucleic acid sequence encoding at least a portion of amino acid sequence of SEQ ID NO: 47, and at least a portion of amino acid sequence of SEQ ID NO: 7, wherein said at least a portion of the amino acid sequence encoded by said second nucleic acid has a biological activity comprising targeting of a fully-folded protein which contains a twin-arginine signal amino acid sequence to a cell membrane and translocating the fully-folded protein which contains twin-arginine signal amino acid sequence to a periplasm, and/or translocating the fully-folded protein which contains a twin-arginine signal amino acid sequence to an extracellular space; b) operably fusing the nucleotide sequence of interest to the first nucleic acid sequence encoding a twin-arginine signal amino acid sequence to produce a fused polynucleotide sequence; and c) introducing the fused polynucleotide sequence and the second nucleic acid sequence into the host cell under conditions such that the at least a portion of an amino acid sequence of SEQ ID NO: 47 and at least a portion of an amino acid sequence of SEQ ID NO: 7 are expressed, and the fused polynucleotide sequence is expressed to produce a fused polypeptide sequence comprising the twin-arginine signal amino acid sequence and the amino acid sequence of interest.

The location of the expressed amino acid sequence of interest is not intended to be limited to any particular location. However, in one preferred embodiment, the expressed amino acid sequence of interest is contained in periplasm of the host cell. In a particularly preferred embodiment, the expressed amino acid sequence of interest is soluble. Also without intending to limit the location of the expressed amino acid sequence of interest, in an alternative preferred embodiment, the host cell is cultured in medium, and the expressed amino acid sequence of interest is contained in the medium. In a particularly preferred embodiment, the expressed amino acid sequence of interest is soluble. In a particularly preferred embodiment, the second nucleic acid sequence encodes an amino acid sequence comprising SEQ ID NO: 47 and SEQ ID NO: 7.

### BRIEF DESCRIPTION OF THE DRAWINGS

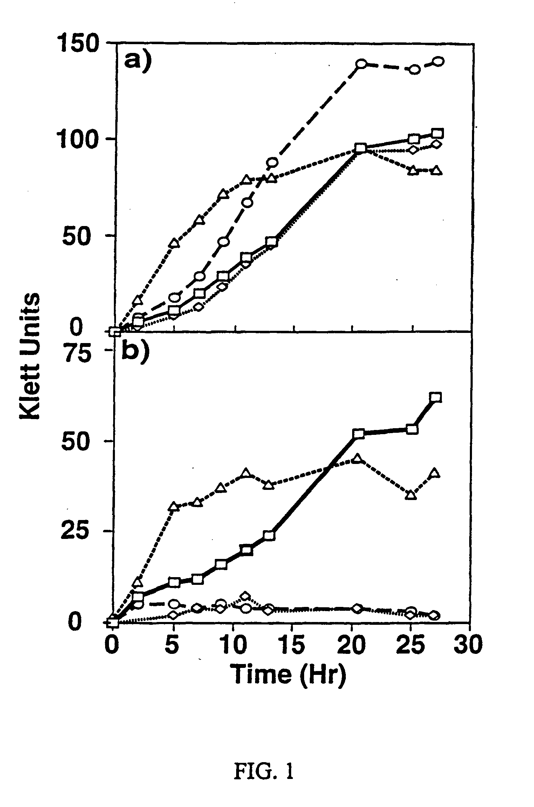

Figure 1 shows anaerobic growth of strain a) HB101 and b) D-43 in the presence of various electron acceptors: (Δ) 40 mM nitrate, (□) 35 mM fumarate, (○) 100 mM TMAO or (0) 70 mM DMSO.

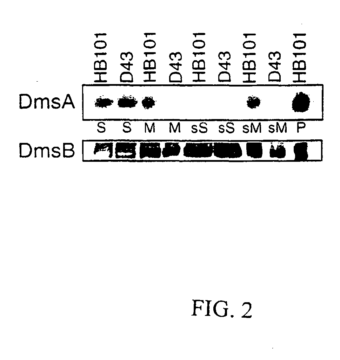

Figure 2 shows a Western blot analysis of washed membranes and soluble fractions of HB101 and D-43 harboring pDMS160 expressing DmsABC.

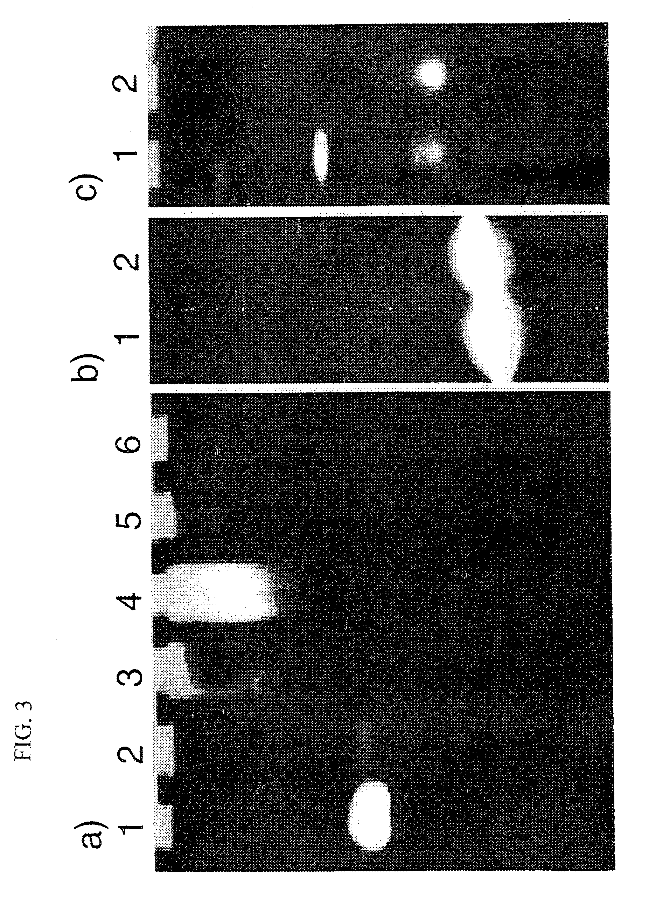

Figure 3 shows A) Nitrate-stained polyacrylamide gel containing periplasmic proteins, membrane proteins and cytoplasmic proteins from HB101 and D-43, B) Nitrite-stained polyacrylamide gel containing periplasmic proteins from HB101 and D-43, and C) TMAO-stained polyacrylamide gel containing periplasmic proteins from HB101 and D-43.

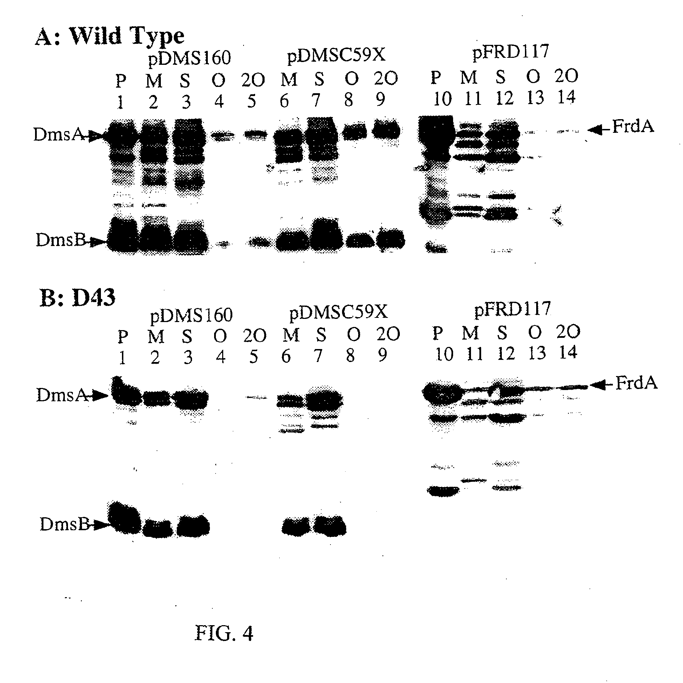

Figure 4 shows the results of a Western blot analysis of the cellular localization of DmsAB in A) HB101 expressing either native DmsABC (pDMS160), DmsABΔC (pDMSC59X), or FrdABΔCD, and B) equivalent lanes as in Figure 4A. but with the same plasmids in D-43.

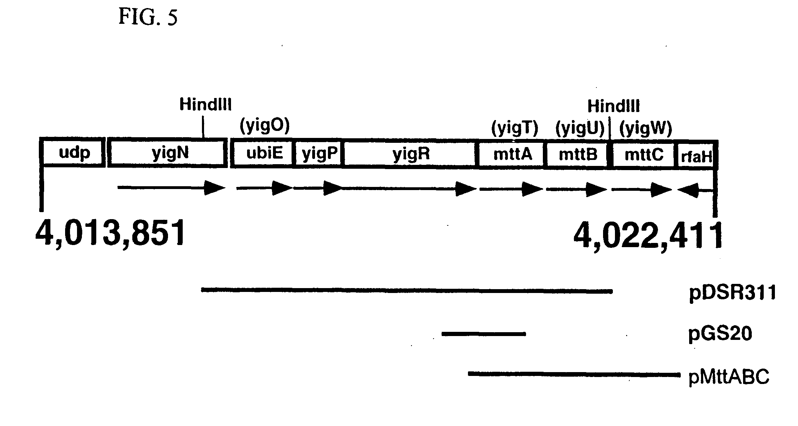

Figure 5 shows a gene map of contig AE00459 noting the positions of the ORFs and the clones used in this investigation.

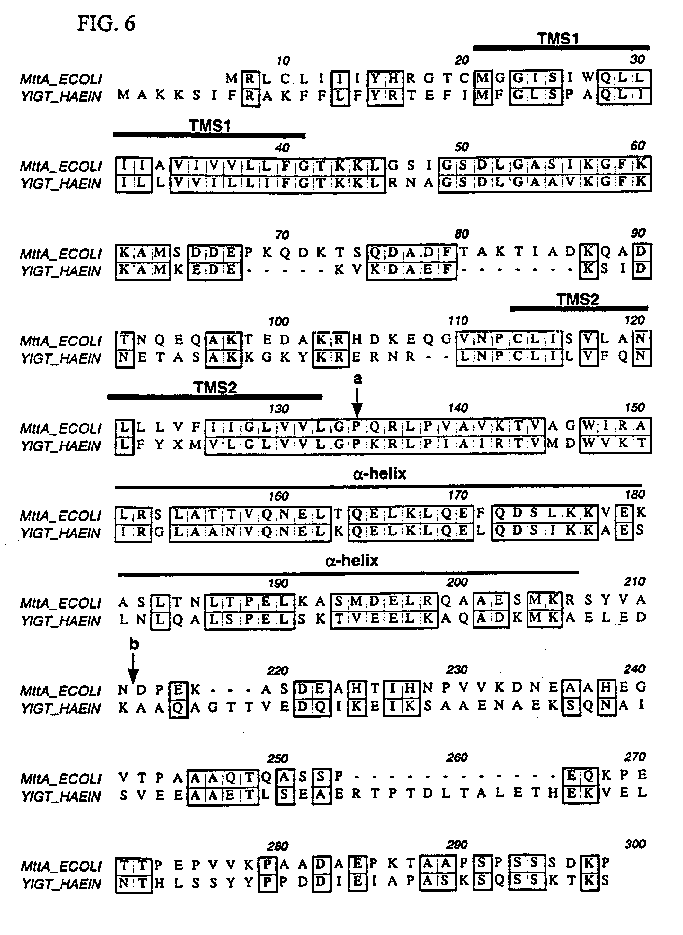

Figure 6 shows the amino acid sequence (SEQ ID NO:1) of MttA aligned with the amino acid sequence of YigT of *Haemophilus influenzae* (SEQ ID NO:2).

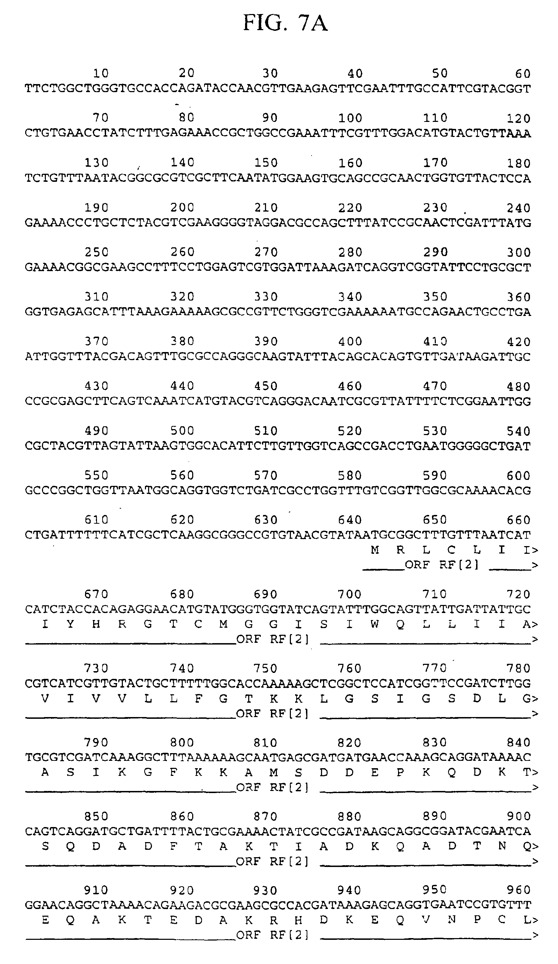

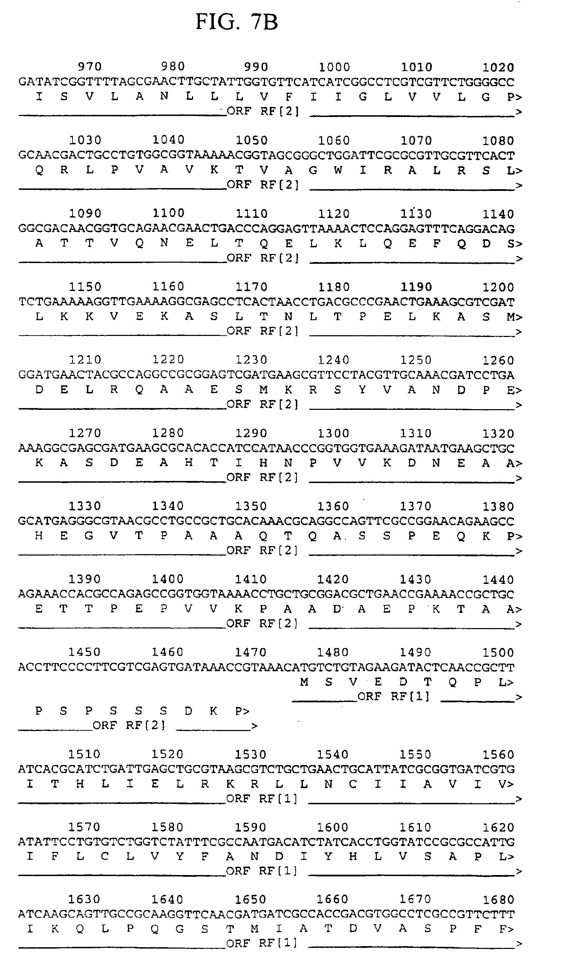

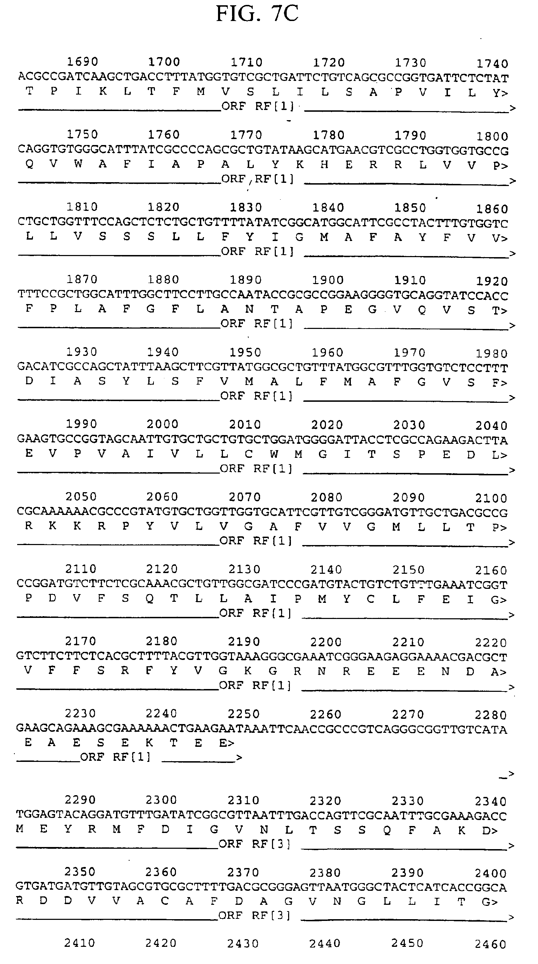

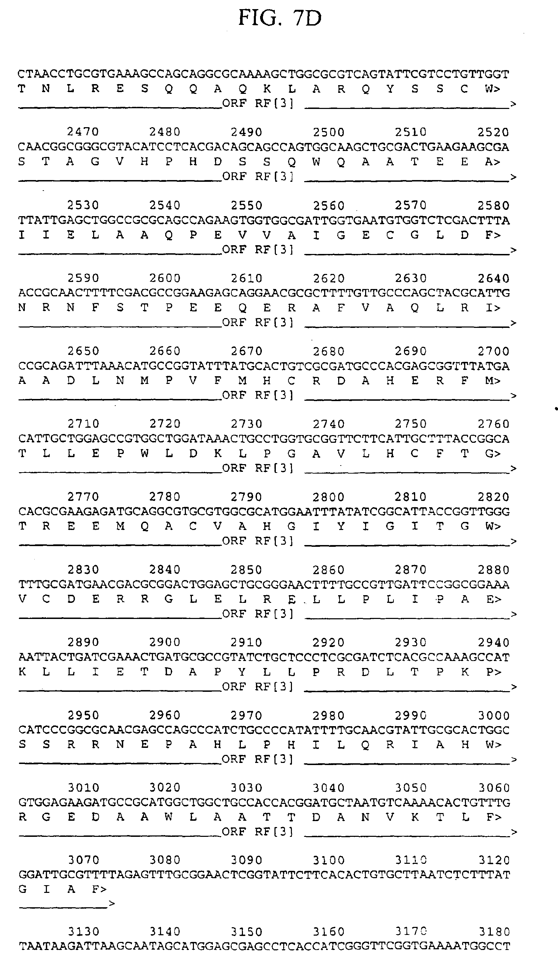

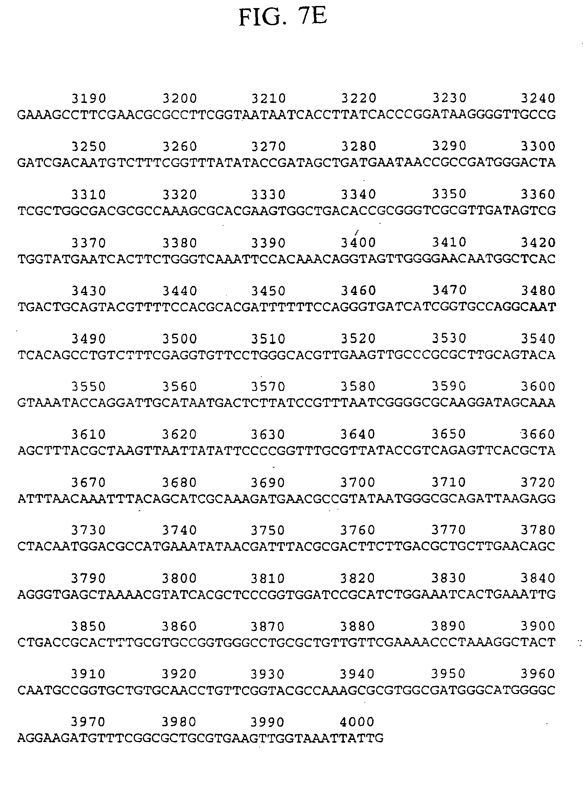

Figure 7 shows the nucleotide sequence (SEQ ID NO:3) of the *mttABC* operon which contains the nucleotide sequence of the three open reading frames, ORF RF[3] nucleotides 5640-6439 (SEQ ID NO:4), ORF RF[2] nucleotides 6473-7246 (SEQ ID NO:5), and ORF RF[1] nucleotides 7279-8070 (SEQ ID NO:6) which encode the amino acid sequences of MttA (SEQ ID NO:1), MttB (SEQ ID NO:7) and MttC (SEQ ID NO:8), respectively.

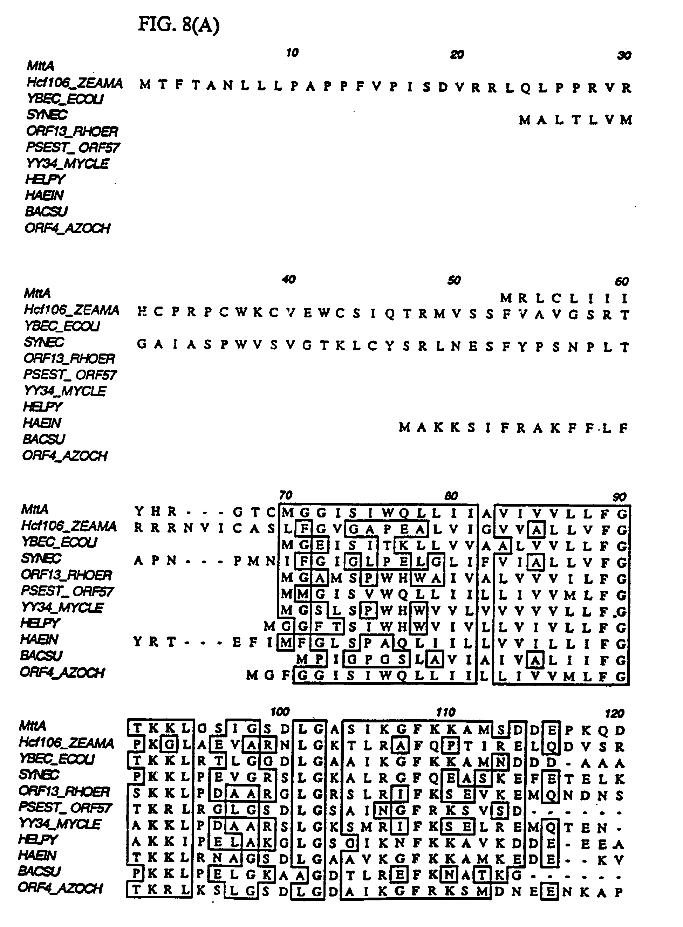

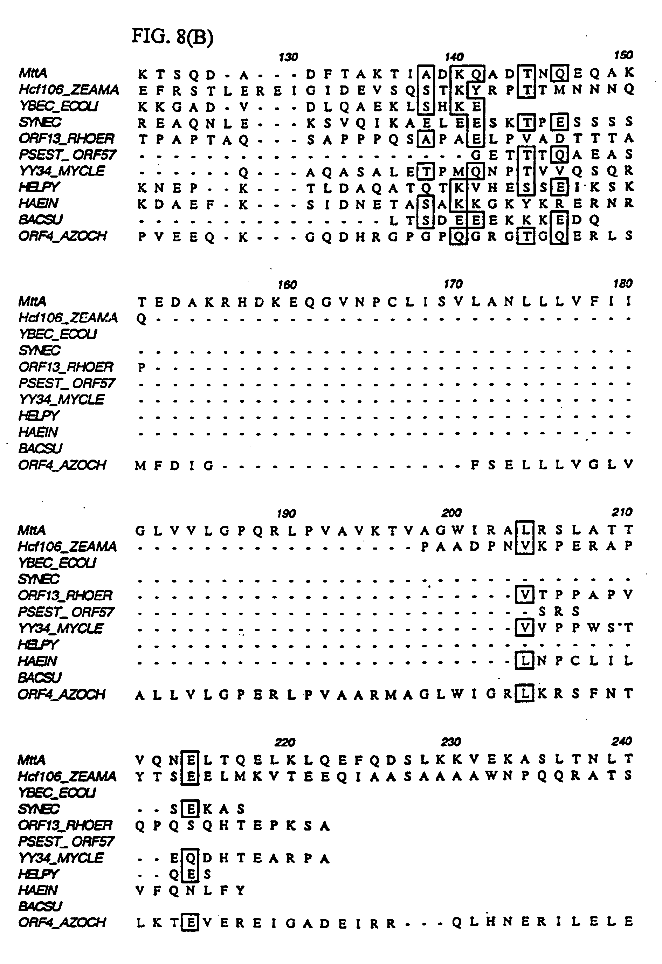

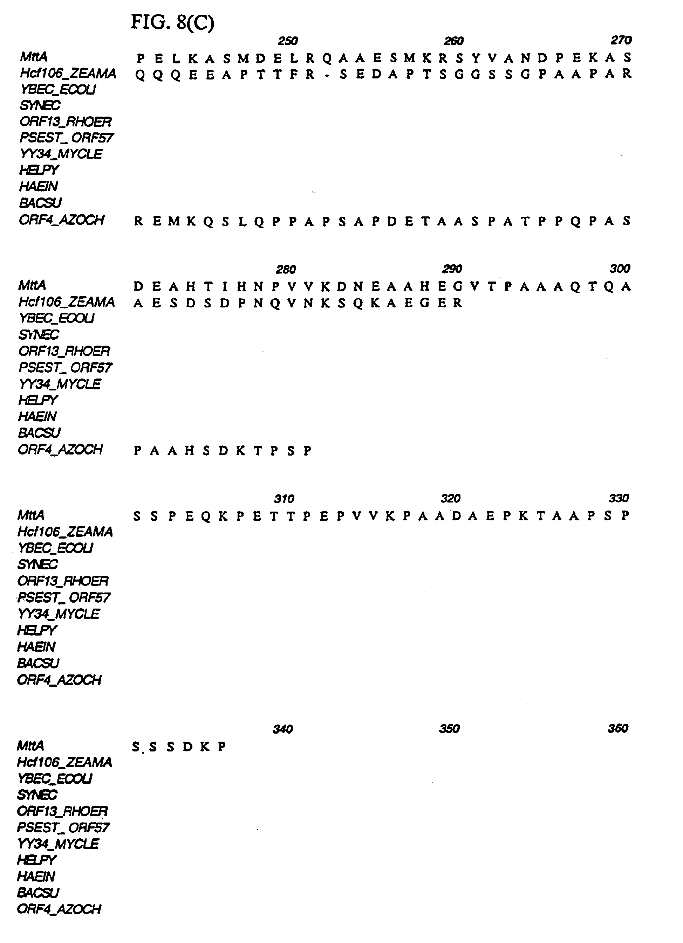

Figure 8 shows an alignment of the amino acid sequence of the *E. coli* MttA sequence (SEQ ID NO:1) with amino acid sequences of Hcf106-ZEAMA (SEQ ID NO:9), YBEC-ECOLI (SEQ ID NO:10), SYNEC (SEQ ID NO:11), ORF13-RHOER (SEQ ID NO:12), PSEST-ORF57 (SEQ ID NO:13), YY34-MYCLE (SEQ ID NO:14), HELPY (SEQ ID NO:15), HAEIN (SEQ ID NO:16), BACSU (SEQ ID NO:17), and ORF4-AZOCH (SEQ ID NO:18).

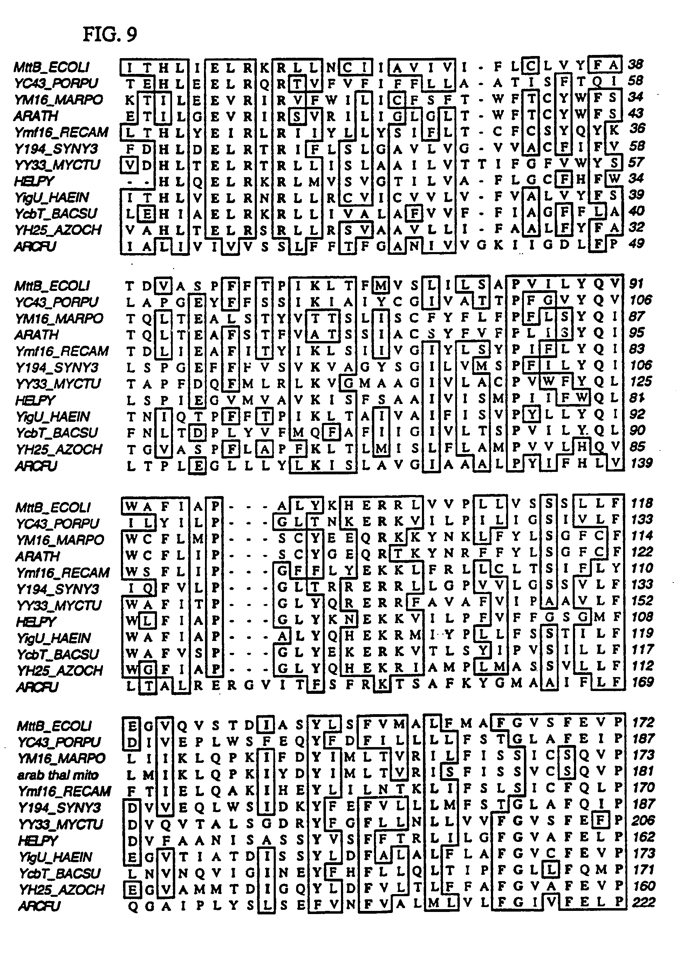

Figure 9 shows an alignment of the amino acid sequence of the *E. coli* MttB sequence (SEQ ID NO:7) with amino acid sequences of YC43-PROPU (SEQ ID NO:19), YM16-MARPO (SEQ ID NO:20), ARATH (SEQ ID NO:21), Ymfl6-RECAM (SEQ ID NO:22), Y194-SYNY3 (SEQ ID NO:23), YY33-MYCTU (SEQ ID NO:24), HELPY (SEQ ID NO:25), YigU-HAEIN (SEQ ID NO:26), YcbT-BACSU (SEQ ID NO:27), YH25-AZOCH (SEQ ID NO:28) and ARCFU (SEQ ID NO:29).

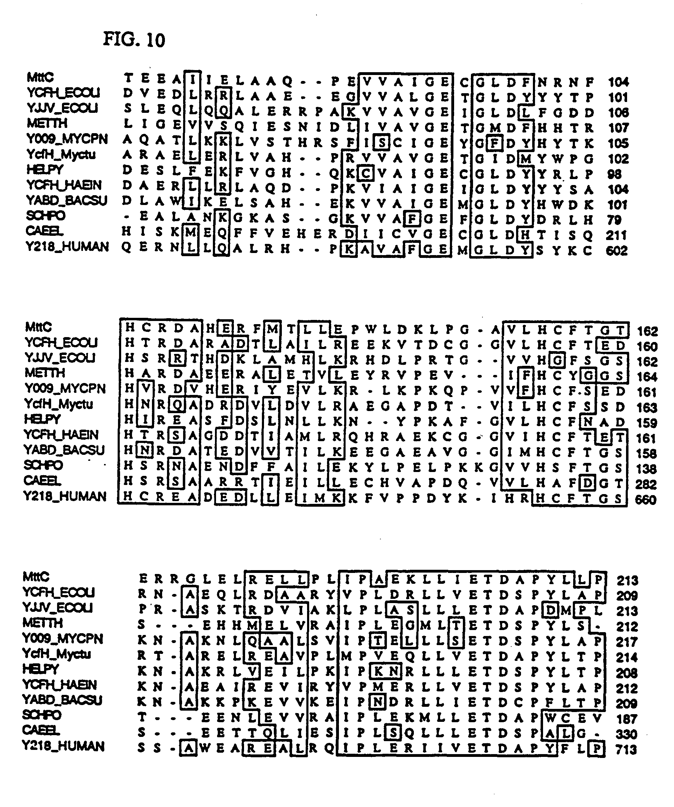

Figure 10 shows an alignment of the amino acid sequence of the *E. coli* MttC sequence (SEQ ID NO:8) with amino acid sequences of YCFH-ECOLI (SEQ ID NO:30). YJJV-ECOLI (SEQ ID NO:31), METTH (SEQ ID NO:32), Y009-MYCPN (SEQ ID NO:33). YcfH-Myctu (SEQ ID NO:34), HELPY (SEQ ID NO:35), YCFH-HAEIN (SEQ ID NO:36), YABC-BACSU (SEQ ID NO:37), SCHPO (SEQ ID NO:38), CAEEL (SEQ ID NO:39) and Y218-HUMAN (SEQ ID NO:40).

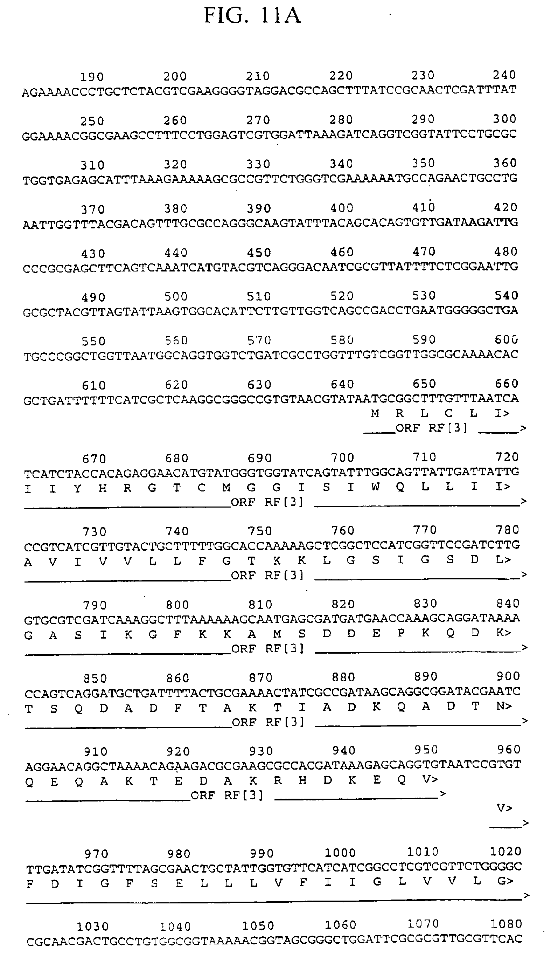

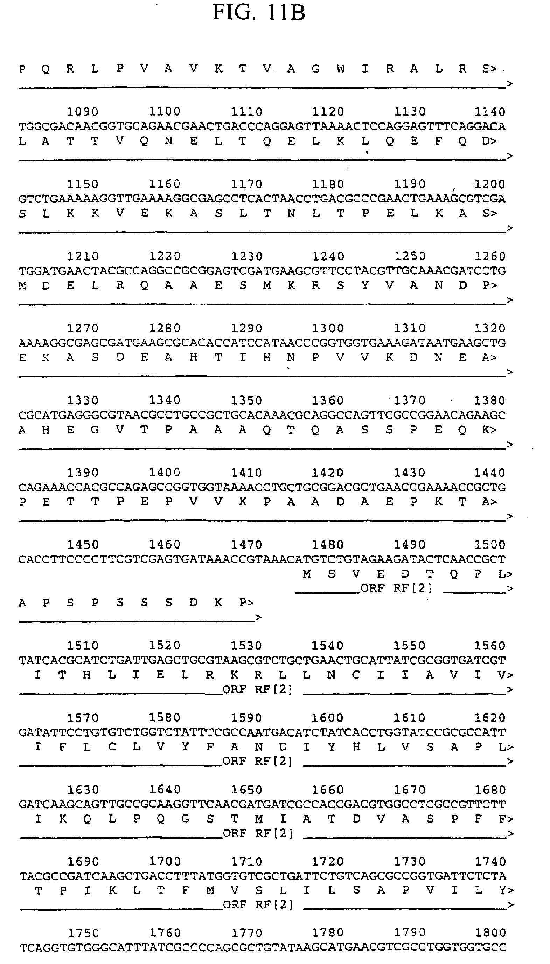

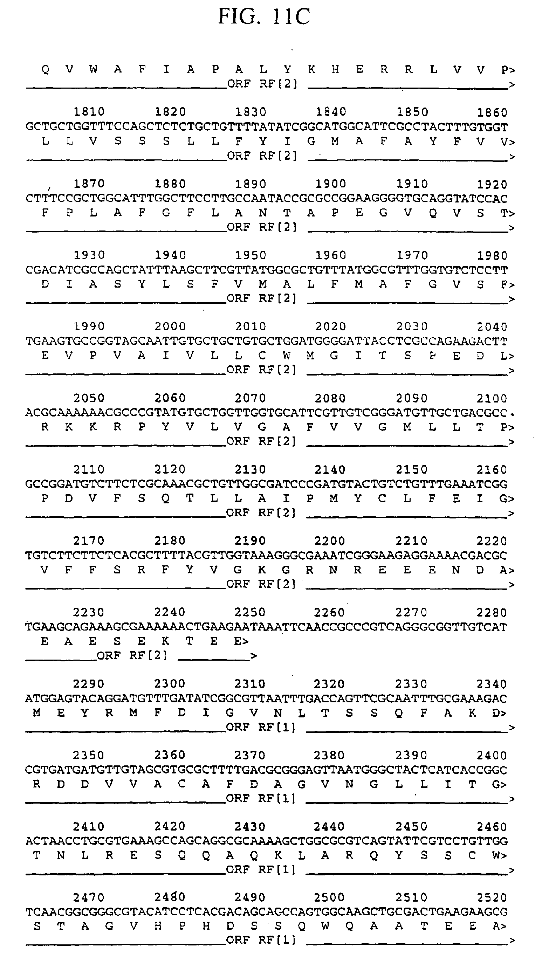

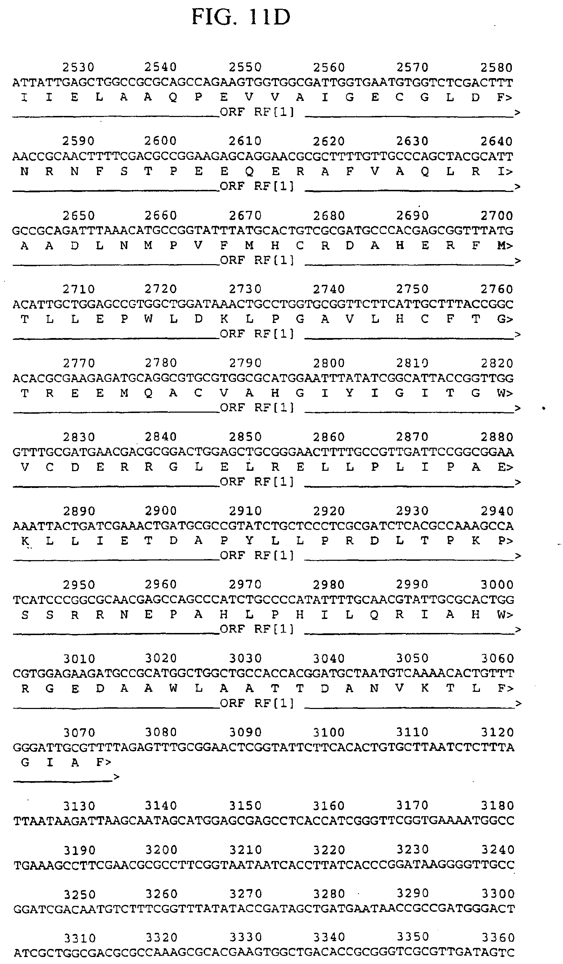

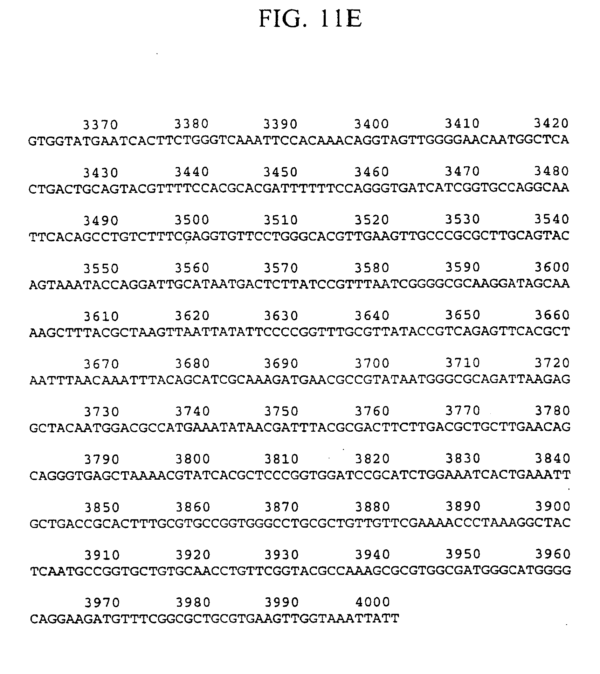

Figure 11 shows the nucleotide sequence (SEQ ID NO:45) of the *mttABC* operon which contains the *mttA1* nucleotide sequence (SEQ ID NO:46) (from nucleic acid number 642 to nucleic acid number 953) encoding the amino acid sequence of MttA1 (SEQ ID NO:47), and the *mttA2* nucleotide sequence (SEQ ID NO:48) (from nucleic acid number 558 to nucleic acid number 1472) encoding the amino acid sequence of MttA2 (SEQ ID NO:49).

### DEFINITIONS

To facilitate understanding of the invention, a number of terms are defined below.

The term "foreign gene" refers to any nucleic acid (*e.g*., gene sequence) which is introduced into a cell by experimental manipulations and may include gene sequences found in that cell so long as the introduced gene contains some modification (*e.g.*, a point mutation, the presence of a selectable marker gene, *etc*.) relative to the naturally-occurring gene.

The term "gene" refers to a DNA sequence that comprises control and coding sequences necessary for the production of RNA or a polypeptide. The polypeptide can be encoded by a full length coding sequence or by any portion of the coding sequence.

The terms "gene of interest" and "nucleotide sequence of interest" refer to any gene or nucleotide sequence, respectively, the manipulation of which may be deemed desirable for any reason, by one of ordinary skill in the art. Such nucleotide sequences include, but are not limited to, coding sequences of structural genes (*e.g*., reporter genes, selection marker genes, oncogenes, drug resistance genes, growth factors, *etc*.), and of regulatory genes (*e.g*., activator protein 1 (AP1), activator protein 2 (AP2), Sp1, *etc*.). Additionally, such nucleotide sequences include non-coding regulatory elements which do not encode an mRNA or protein product, such as for example, a promoter sequence, an enhancer sequence, *etc*.

As used herein the term "coding region" when used in reference to a structural gene refers to the nucleotide sequences which encode the amino acids found in the nascent polypeptide as a result of translation of an mRNA molecule. The coding region is bounded, in eukaryotes, on the 5' side by the nucleotide triplet "ATG" which encodes the initiator methionine and on the 3' side by one of the three triplets which specify stop codons (*i.e*., TAA, TAG, TGA).

Transcriptional control signals in eukaryotes comprise "promoter" and "enhancer" elements. Promoters and enhancers consist of short arrays of DNA sequences that interact specifically with cellular proteins involved in transcription [Maniatis, et al., Science 236:1237 (1987)]. Promoter and enhancer elements have been isolated from a variety of eukaryotic sources including genes in yeast, insect and mammalian cells and viruses (analogous control elements, i.e., promoters, are also found in prokaryotes). The selection of a particular promoter and enhancer depends on what cell type is to be used to express the protein of interest. Some eukaryotic promoters and enhancers have a broad host range while others are functional in a limited subset of cell types [for review see Voss, et al., Trends Biochem. Sci., 11:287 (1986) and Maniatis, et al., Science 236:1237 (1987)].

The term "wild-type" refers to a gene or gene product which has the characteristics of that gene or gene product when isolated from a naturally occurring source. A wild-type gene is that which is most frequently observed in a population and is thus arbitrarily designed the "normal" or "wild-type" form of the gene. In contrast, the term "modified" or "mutant" refers to a gene or gene product which displays modifications in sequence and or functional properties (*i*.*e*., altered characteristics) when compared to the wild-type gene or gene product. It is noted that naturally-occurring mutants can be isolated; these are identified by the fact that they have altered characteristics when compared to the wild-type gene or gene product.

The term "expression vector" as used herein refers to a recombinant DNA molecule containing a desired coding sequence and appropriate nucleic acid sequences necessary for the expression of the operably linked coding sequence in a particular host cell. Nucleic acid sequences necessary for expression in prokaryotes include a promoter, optionally an operator sequence, a ribosome binding site and possibly other sequences. Eukaryotic cells are known to utilize promoters, enhancers, and termination and polyadenylation signals.

The terms "targeting vector" or "targeting construct" refer to oligonucleotide sequences comprising a gene of interest flanked on either side by a recognition sequence which is capable of homologous recombination of the DNA sequence located between the flanking recognition sequences into the chromosomes of the target cell or recipient cell. Typically, the targeting vector will contain 10 to 15 kb of DNA homologous to the gene to be recombined: this 10 to 15 kb of DNA is generally divided more or less equally on each side of the selectable marker gene. The targeting vector may contain more than one selectable maker gene. When more than one selectable marker gene is employed, the targeting vector preferably contains a positive selectable marker (*e.g*., the *neo* gene) and a negative selectable marker (*e.g*., the Herpes simplex virus *tk* (HSV-*tk*) gene). The presence of the positive selectable marker permits the selection of recipient cells containing an integrated copy of the targeting vector whether this integration occurred at the target site or at a random site. The presence of the negative selectable marker permits the identification of recipient cells containing the targeting vector at the targeted site (*i*.*e*., which has integrated by virtue of homologous recombination into the target site); cells which survive when grown in medium which selects against the expression of the negative selectable marker do not contain a copy of the negative selectable marker. Integration of a replacement-type vector results in the insertion of a selectable marker into the target gene. Replacement-type targeting vectors may be employed to disrupt a gene resulting in the generation of a null allele (*i.e.,* an allele incapable of expressing a functional protein; null alleles may be generated by deleting a portion of the coding region, deleting the entire gene, introducing an insertion and/or a frameshift mutation, etc.) or may be used to introduce a modification (*e.g*., one or more point mutations) into a gene.

The terms "in operable combination", "in operable order" and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

As used herein, the terms "vector" and "vehicle" are used interchangeably in reference to nucleic acid molecules that transfer DNA segment(s) from one cell to another.

The term "recombinant DNA molecule" as used herein refers to a DNA molecule which is comprised of segments of DNA joined together by means of molecular biological techniques.

The term "recombinant protein" or "recombinant polypeptide" as used herein refers to a protein molecule which is expressed using a recombinant DNA molecule.

The term "transfection" as used herein refers to the introduction of a transgene into a cell. The term "transgene" as used herein refers to any nucleic acid sequence which is introduced into the genome of a cell by experimental manipulations. A transgene may be an "endogenous DNA sequence," or a "heterologous DNA sequence" (*i*.*e*., "foreign DNA"). The term "endogenous DNA sequence" refers to a nucleotide sequence which is naturally found in the cell into which it is introduced so long as it does not contain some modification (*e*.*g*., a point mutation, the presence of a selectable marker gene, etc.) relative to the naturally-occurring sequence. The term "heterologous DNA sequence" refers to a nucleotide sequence which is not endogenous to the cell into which it is introduced. Heterologous DNA includes a nucleotide sequence which is ligated to, or is manipulated to become ligated to, a nucleic acid sequence to which it is not ligated in nature, or to which it is ligated at a different location in nature. Heterologous DNA also includes a nucleotide sequence which is naturally found in the cell into which it is introduced and which contains some modification relative to the naturally-occurring sequence. Generally, although not necessarily, heterologous DNA encodes RNA and proteins that are not normally produced by the cell into which it is introduced. Examples of heterologous DNA include reporter genes, transcriptional and translational regulatory sequences, DNA sequences which encode selectable marker proteins (*e*.*g*., proteins which confer drug resistance), *etc*. Yet another example of a heterologous DNA includes a nucleotide sequence which encodes a ribozyme which is found in the cell into which it is introduced, and which is ligated to a promoter sequence to which it is not naturally ligated in that cell.

Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, biolistics (*i*.*e*., particle bombardment) and the like.

The term "stable transfection" or "stably transfected" refers to the introduction and integration of a transgene into the genome of the transfected cell. The term "stable transfectant" refers to a cell which has stably integrated one or more transgenes into the genomic DNA.

As used herein the term "portion" when in reference to a gene refers to fragments of that gene. The fragments may range in size from 5 nucleotide residues to the entire nucleotide sequence minus one nucleic acid residue. Thus, "an oligonucleotide comprising at least a portion of a gene" may comprise small fragments of the gene or nearly the entire gene.

The term "portion" when used in reference to a protein (as in a "portion of a given protein") refers to fragments of that protein. The fragments may range in size from four amino acid residues to the entire amino acid sequence minus one amino acid.

The term "isolated" when used in relation to a nucleic acid, as in "an isolated oligonucleotide" refers to a nucleic acid sequence that is identified and separated from at least one contaminant nucleic acid with which it is ordinarily associated in its natural source. Isolated nucleic acid is nucleic acid present in a form or setting that is different from that in which it is found in nature. In contrast, non-isolated nucleic acids are nucleic acids such as DNA and RNA which are found in the state they exist in nature. For example, a given DNA sequence (e.g., a gene) is found on the host cell chromosome in proximity to neighboring genes; RNA sequences, such as a specific mRNA sequence encoding a specific protein, are found in the cell as a mixture with numerous other mRNAs which encode a multitude of proteins. However, isolated nucleic acid sequences encoding MttA1, MttA2, MttB or MttC polypeptides include, by way of example, such nucleic acid sequences in cells ordinarily expressing MttA1, MttA2, MttB or MttC polypeptides, respectively, where the nucleic acid sequences are in a chromosomal or extrachromosomal location different from that of natural cells, or are otherwise flanked by a different nucleic acid sequence than that found in nature. The isolated nucleic acid or oligonucleotide may be present in single-stranded or double-stranded form. When an isolated nucleic acid or oligonucleotide is to be utilized to express a protein, the oligonucleotide will contain at a minimum the sense or coding strand (*i.e*., the oligonucleotide may be single-stranded). Alternatively, it may contain both the sense and anti-sense strands (*i.e*., the oligonucleotide may be double-stranded).

As used herein, the term "purified" or "to purify" refers to the removal of undesired components from a sample. For example, where recombinant MttA1, MttA2, MttB or MttC polypeptides are expressed in bacterial host cells, the MttA1, MttA2, MttB or MttC polypeptides are purified by the removal of host cell proteins thereby increasing the percent of recombinant MttA1, MttA2, MttB or MttC polypeptides in the sample.

As used herein, the term "substantially purified" refers to molecules, either nucleic or amino acid sequences, that are removed from their natural environment, isolated or separated, and are at least 60% free, preferably 75% free, and more preferably 90% free from other components with which they are naturally associated. An "isolated polynucleotide" is therefore a substantially purified polynucleotide.

The term "recombinant DNA molecule" as used herein refers to a DNA molecule which is comprised of segments of DNA joined together by means of molecular biological techniques.

The term "recombinant protein" or "recombinant polypeptide" as used herein refers to a protein molecule which is expressed using a recombinant DNA molecule.

The term "homology" when used in relation to nucleic acids refers to a degree of complementarity. There may be partial homology or complete homology (*i.e*., identity). A partially complementary sequence is one that at least partially inhibits a completely complementary sequence from hybridizing to a target nucleic acid is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe (*i*.*e*., an oligonucleotide which is capable of hybridizing to another oligonucleotide of interest) will compete for and inhibit the binding (*i.e.*, the hybridization) of a completely homologous sequence to a target under conditions of low stringency. This is not to say that conditions of low stringency are such that non-specific binding is permitted; low stringency conditions require that the binding of two sequences to one another be a specific (*i.e*., selective) interaction. The absence of non-specific binding may be tested by the use of a second target which lacks even a partial degree of complementarity (*e.g*., less than about 30% identity); in the absence of non-specific binding the probe will not hybridize to the second non-complementary target.

Low stringency conditions when used in reference to nucleic acid hybridization comprise conditions equivalent to binding or hybridization at 42°C in a solution consisting of 5X SSPE (43.8 g/l NaCl, 6.9 g/l NaH₂PO₄•H₂O and 1.85 g/l EDTA. pH adjusted to 7.4 with NaOH), 0.1% SDS, 5X Denhardt's reagent [50X Denhardt's contains per 500 ml: 5 g Ficoll (Type 400, Pharmacia), 5 g BSA (Fraction V; Sigma)] and 100 µg/ml denatured salmon sperm DNA followed by washing in a solution comprising 5X SSPE, 0.1 % SDS at 42°C when a probe of about 500 nucleotides in length is employed.

High stringency conditions when used in reference to nucleic acid hybridization comprise conditions equivalent to binding or hybridization at 42°C in a solution consisting of 5X SSPE (43.8 g/l NaCl, 6.9 g/l NaH₂PO₄•H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42°C when a probe of about 500 nucleotides in length is employed.

When used in reference to nucleic acid hybridization the art knows well that numerous equivalent conditions may be employed to comprise either low or high stringency conditions: factors such as the length and nature (DNA, RNA, base composition) of the probe and nature of the target (DNA, RNA, base composition, present in solution or immobilized, etc.) and the concentration of the salts and other components (*e.g*., the presence or absence of formamide, dextran sulfate, polyethylene glycol) are considered and the hybridization solution may be varied to generate conditions of either low or high stringency hybridization different from, but equivalent to, the above listed conditions.

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of ribonucleotides along the mRNA chain, and also determines the order of amino acids along the polypeptide (protein) chain. The DNA sequence thus codes for the RNA sequence and for the amino acid sequence.

"Nucleic acid sequence" and "nucleotide sequence" as used interchangeably herein refer to an oligonucleotide or polynucleotide, and fragments or portions thereof, and to DNA or RNA of genomic or synthetic origin which may be single- or double-stranded, and represent the sense or antisense strand.

"Amino acid sequence" and "polypeptide sequence" are used interchangeably herein to refer to a sequence of amino acids.

The term "antisense sequence" as used herein refers to a deoxyribonucleotide sequence whose sequence of deoxyribonucleotide residues is in reverse 5' to 3' orientation in relation to the sequence of deoxyribonucleotide residues in a sense strand of a DNA duplex. A "sense strand" of a DNA duplex refers to a strand in a DNA duplex which is transcribed by a cell in its natural state into a "sense mRNA." Sense mRNA generally is ultimately translated into a polypeptide. Thus an "antisense" sequence is a sequence having the same sequence as the non-coding strand in a DNA duplex. The term "antisense RNA" refers to a ribonucleotide sequence whose sequence is complementary to an "antisense" sequence. Alternatively, the term "antisense RNA" is used in reference to RNA sequences which are complementary to a specific RNA sequence (*e.g*., mRNA). Antisense RNA may be produced by any method, including synthesis by splicing the gene(s) of interest in a reverse orientation to a viral promoter which permits the synthesis of a coding strand. Once introduced into a cell, this transcribed strand combines with natural mRNA produced by the cell to form duplexes. These duplexes then block either the further transcription of the mRNA or its translation. In this manner, mutant phenotypes may be generated. The term "antisense strand" is used in reference to a nucleic acid strand that is complementary to the "sense" strand. The designation (-) (*i.e*., "negative") is sometimes used in reference to the antisense strand, with the designation (+) sometimes used in reference to the sense (*i.e*., "positive") strand.

The term "biologically active" when made in reference to MttA1, MttA2, MttB or MttC refers to a MttA1, MttA2, MttB or MttC molecule, respectively, having biochemical functions of a naturally occurring MttA1, MttA2, MttB or MttC. Biological activity of MttA1, MttA2, MttB or MttC is determined, for example, by restoration of wild-type targeting of proteins which contain twin-arginine signal amino acid sequence to cell membranes and/or translocation of such proteins to the periplasm in cells lacking MttA. MttB or MttC activity (*i.e*., MttA1, MttA2, MttB or MttC null cells). Cells lacking MttA1, MttA2. MttB or MttC activity may be produced using methods well known in the art (*e.g*.. point mutation and frame-shift mutation) [Sambasivarao et al (1991) J. Bacteriol. 5935-5943: Jasin et al (1984) J. Bacteriol. 159:783-786]. Complementation is achieved by transfecting cells which lack MttA1, MttA2, MttB or MttC activity with an expression vector which expresses MttA1, MttA2, MttB or MttC, a homolog thereof, or a portion thereof. Details concerning complementation of cells which contain a point mutation in MttA1, MttA2 is provided in Example 6 herein.

As used herein "soluble" when in reference to a protein produced by recombinant DNA technology in a host cell is a protein which exists in solution; if the protein contains a twin-arginine signal amino acid sequence the soluble protein is exported to the periplasmic space in gram negative bacterial hosts and is secreted into the culture medium by eukaryotic cells capable of secretion or by bacterial host possessing the appropriate genes (*i.e*., the *kil* gene). Thus, a soluble protein is a protein which is not found in an inclusion body inside the host cell. Alternatively, a soluble protein is a protein which is not found integrated in cellular membranes. In contrast, an insoluble protein is one which exists in denatured form inside cytoplasmic granules (called an inclusion body) in the host cell. Alternatively, an insoluble protein is one which is present in cell membranes, including but not limited to, cytoplasmic membranes, mitochondrial membranes, chloroplast membranes, endoplasmic reticulum membranes, *etc*.

A distinction is drawn between a soluble protein (*i.e.*, a protein which when expressed in a host cell is produced in a soluble form) and a "solubilized" protein. An insoluble recombinant protein found inside an inclusion body or found integrated in a cell membrane may be solubilized (*i.e.*, rendered into a soluble form) by treating purified inclusion bodies or cell membranes with denaturants such as guanidine hydrochloride, urea or sodium dodecyl sulfate (SDS). These denaturants must then be removed from the solubilized protein preparation to allow the recovered protein to renature (refold). Not all proteins will refold into an active conformation after solubilization in a denaturant and removal of the denaturant. Many proteins precipitate upon removal of the denaturant. SDS may be used to solubilize inclusion bodies and cell membranes and will maintain the proteins in solution at low concentration. However, dialysis will not always remove all of the SDS (SDS can form micelles which do not dialyze out); therefore, SDS-solubilized inclusion body protein and SDS-solubilized cell membrane protein is soluble but not refolded.

A distinction is also drawn between proteins which are soluble ( *i*.*e*., dissolved) in a solution devoid of significant amounts of ionic detergents (*e*.*g*., SDS) or denaturants (*e.g*., urea, guanidine hydrochloride) and proteins which exist as a suspension of insoluble protein molecules dispersed within the solution. A soluble protein will not be removed from a solution containing the protein by centrifugation using conditions sufficient to remove cells present in a liquid medium (*e.g*., centrifugation at 5,000 x g for 4-5 minutes).

### DESCRIPTION OF THE INVENTION

The present invention exploits the identification of proteins involved in a Sec-independent protein translocation pathway which are necessary for the translocation of proteins which contain twin-arginine signal amino acid sequences to the periplasm of gram negative bacteria, and into the extracellular media of cells which do not contain a periplasm (*e.g.*, gram positive bacteria, eukaryotic cells, *etc.),* as well as for targeting such proteins to cell membranes. The proteins of the invention are exemplified by the Membrane Targeting and Translocation proteins MttA1 (103 amino acids), MttA2 (161 amino acids), MttB (258 amino acids) and MttC (264 amino acids) of *E. coli* which are encoded by the *mttABC* operon. The invention further exploits the presence of a large number of proteins which are widely distributed in organisms extending from archaebacteria to higher eukaryotes.

The well characterized Sec-dependent export system translocates an unfolded string of amino acids to the periplasm and folding follows as a subsequent step in the periplasm and mediated by chaperones and disulfide rearrangement. In contrast to the Sec-dependent export pathway, the proteins of the invention translocate fully-folded as well as cofactor-containing proteins from the cytoplasm into the bacterial periplasm and are capable of translocating such proteins into extracellular medium. Such translocation offers a unique advantage over current methodologies for protein purification. Because the composition of culture medium can be manipulated, and because the periplasm contains only about 3% of the proteins of gram negative bacteria, expressed proteins which are translocated into the extracellular medium or into the periplasm are more likely to be expressed as functional soluble proteins than if they were translocated to cellular membranes or to the cytoplasm. Furthermore, translocation to the periplasm or to the extracellular medium following protein expression in the cytoplasm allows the expressed protein to be correctly folded by cytoplasmic enzymes prior to its translocation, thus allowing retention of the expressed protein's biological activity.

The *mttABC* operon disclosed herein is also useful in screening compounds for antibiotic activity by identifying those compounds which inhibit translocation of proteins containing twin-arginine signal amino acid sequences in bacteria. For example, DMSO reductase has been found to be essential for the pathogenesis of *Salmonella* [Bowe and Heffron (1994) Methods in Enzymology 236:509-526]. Thus, compounds which inhibit targeting of DMSO reductase to *Salmonella* could result in conversion of a virulent bacterial strain to an avirulent nonpathogenic variant.

The invention is further described under (A) *mttA, mttB, and mttC* nucleotide sequences, (B) MttA, MttB, and MttC polypeptides, and (C) Methods for expressing polypeptides to produce soluble proteins.

### A. mttA, mttB, and mttC nucleotide sequences

The present invention discloses the nucleic acid sequence of the *mttA*1 (SEQ ID NO:46), *mttA*2 (SEQ ID NO:48), mttB (SEQ ID NO:5) and *mtt*C (SEQ ID NO:6) genes which form part of the *mttABC* operon (SEQ ID NO:45) shown in Figure 11. Data presented herein demonstrates that the MttA2 polypeptide encoded by *mttA2* functions in targeting proteins which contain twin-arginine signal amino acid sequences to cell membranes, and in translocating such proteins to the periplasm of gram negative bacteria and to the extracellular medium of cells which do not contain a periplasm (*e.g*., gram positive bacteria and eukaryotic cells). Data presented herein further shows that the MttB and MttC polypeptides which are encoded by *mttB* and *mttC,* respectively, also serve the same functions as MttA2. This conclusion is based on the inventors' finding that *mttA1, mttA2, mttB* and *mttC* form an operon which is expressed as a single polycistronic mRNA.

The function of MttB and MttC may be demonstrated by *in vivo* homologous recombination of chromosomal *mttB* and *mttC* by using knockouts in the *mttBC* operon by utilizing insertion of mini-MudII as previously described [Taylor et al. (1994) J. Bacteriol. 176:2740-2742]. Alternatively, the function of MttB and MttC may also be demonstrated as previously described [Sambasivarao et al (1991) J. Bacteriol. 5935-5943; Jasin et al (1984) J. Bacteriol. 159:783-786]. Briefly, the *mttABC* operon (Figure 11) is cloned into pTZ18R and pBR322 vectors. In pBR322, the HindIII site in *mttB* is unique. The pBR322 containing *mttB* is then modified by insertion of a kanamycin gene cartridge at this unique site, while the unique Nrul fragment contained in *mttC* are replaced by a kanamycin cartridge. The modified plasmids are then be homologously recombined with chromosomal *mttB* and *mttC* in *E. coli* cells which contain either a *recBC* mutation or a *recD* mutation. The resulting recombinant are transferred by P1 transduction to suitable genetic backgrounds for investigation of the localization of protein expression. The localization (*e.g*., cytoplasm, periplasm, cell membranes, extracellular medium) of expression of proteins which contain twin-arginine signal amino acid sequences is compared using methods disclosed herein (*e.g*., functional enzyme activity and Western blotting) between homologously recombined cells and control cells which had not been homologously recombined. Localization of expressed proteins which contain twin-arginine signal amino acid sequences in extracellular medium or in the periplasm of homologously recombined cells as compared to localization of expression in other than the extracellular medium and the periplasm (*e.g.*, in the cytoplasm, in the cell membrane, *etc.)* of control cells demonstrates that the wild-type MttB or MttC protein whose function had been modified by homologous recombination functions in translocation of the twin argining containing proteins to the extracellular medium or to the periplasm.

The present invention contemplates any nucleic acid sequence which encodes one or more of MttA1, MttA2, MttB and MttC polypeptide sequences or variants or homologs thereof. These nucleic acid sequences are used to make recombinant molecules which express the MttA1, MttA2, MttB and MttC polypeptides. For example, one of ordinary skill in the art would recognize that the redundancy of the genetic code permits an enormous number of nucleic acid sequences which encode the MttA1, MttA2, MttB and MttC polypeptides. Thus, codons which are different from those shown in Figure 7 may be used to increase the rate of expression of the nucleotide sequence in a particular prokaryotic or eukaryotic expression host which has a preference for particular codons. Additionally, alternative codons may also be used in eukaryotic expression hosts to generate splice variants of recombinant RNA transcripts which have more desirable properties (*e.g*., longer or shorter half-life) than transcripts generated using the sequence depicted in Figure 7. In addition, different codons may also be desirable for the purpose of altering restriction enzyme sites or, in eukaryotic expression hosts, of altering glycosylation patterns in translated polypeptides.

The nucleic acid sequences of the invention may also be used for *in vivo* homologous recombination with chromosomal nucleic acid sequences. Homologous recombination may be desirable to, for example, delete at least a portion of at least one of chromosomal *mttA1, mttA2, mttB* and *mttC* nucleic acid sequences, or to introduce a mutation in these chromosomal nucleic acid sequence as described below.

Variants of the nucleotide sequences which encode MttA1, MttA2, MttB and MttC and which are shown in Figure 7 and Figure 11 are also included within the scope of this invention. These variants include, but are not limited to, nucleotide sequences having deletions, insertions or substitutions of different nucleotides or nucleotide analogs.

This invention is not limited to the *mttA1, mttA2, mttB and mttC* sequences (SEQ ID NOs:46, 48. 5 and 6, respectively) but specifically includes nucleic acid homologs which are capable of hybridizing to the nucleotide sequence encoding MttA1, MttA2, MttB and MttC (Figures 11 and 7), and to portions, variants and homologs thereof. Those skilled in the art know that different hybridization stringencies may be desirable. For example, whereas higher stringencies may be preferred to reduce or eliminate non-specific binding between the nucleotide sequences of Figure 7 and other nucleic acid sequences, lower stringencies may be preferred to detect a larger number of nucleic acid sequences having different homologies to the nucleotide sequence of Figure 7.

Portions of the nucleotide sequence encoding MttA1, mttA2, MttB and MttC of Figures 11 and 7 are also specifically contemplated to be within the scope of this invention. It is preferred that the portions have a length equal to or greater than 10 nucleotides and show greater than 50% homology to nucleotide sequences encoding MttA1, mttA2, MttB and MttC of Figures 11 and 7.

The present invention further contemplates antisense molecules comprising the nucleic acid sequence complementary to at least a portion of the polynucleotide sequences encoding MttA1, mttA2, MttB and MttC (Figures 11 and 7).

The scope of this invention further encompasses nucleotide sequences containing the nucleotide sequence of Figures 11 and 7, portions, variants, and homologs thereof, ligated to one or more heterologous sequences as part of a fusion gene. Such fusion genes may be desirable, for example, to detect expression of sequences which form part of the fusion gene. Examples of a heterologous sequence include the reporter sequence encoding the enzyme β-galactosidase or the enzyme luciferase. Fusion genes may also be desirable to facilitate purification of the expressed protein. For example, the heterologous sequence of protein A allows purification of the fusion protein on immobilized immunoglobulin. Other affinity traps are well known in the art and can be utilized to advantage in purifying the expressed fusion protein. For example, pGEX vectors (Promega, Madison WI) may be used to express the MttA1, MttA2, MttB and MttC polypeptides as a fusion protein with glutathione S-transferase (GST). In general, such fusion proteins are soluble and can easily be purified from lysed cells by adsorption to glutathione-agarose beads followed by elution in the presence of free glutathione. Proteins made in such systems are designed to include heparin, thrombin or factor XA protease cleavage sites so that the cloned polypeptide of interest can be released from the GST moiety at will.

The nucleotide sequences which encode MttA1, MttA2, MttB and MttC (Figures 11 and 7), portions, variants, and homologs thereof can be synthesized by synthetic chemistry techniques which are commercially available and well known in the art. The nucleotide sequence of synthesized sequences may be confirmed using commercially available kits as well as from methods well known in the art which utilize enzymes such as the Klenow fragment of DNA polymerase I, Sequenase^{®}, *Taq* DNA polymerase, or thermostable T7 polymerase. Capillary electrophoresis may also be used to analyze the size and confirm the nucleotide sequence of the products of nucleic acid synthesis. Synthesized sequences may also be amplified using the polymerase chain reaction (PCR) as described by Mullis [U.S. Patent No. 4,683,195] and Mullis et al. [U.S. Patent No. 4,683,202], the ligase chain reaction [LCR; sometimes referred to as "Ligase Amplification Reaction" (LAR)] described by Barany, Proc. Natl. Acad. Sci., 88:189 (1991); Barany, PCR Methods and Applic., 1:5 (1991); and Wu and Wallace, Genomics 4:560 (1989).

It is readily appreciated by those in the art that the *mttA1, mttA2, mttB* and *mttC* nucleotide sequences of the present invention may be used in a variety of ways. For example, fragments of the sequence of at least about 10 bp, more usually at least about 15 bp, and up to and including the entire (*i.e.*, full-length) sequence can be used as probes for the detection and isolation of complementary genomic DNA sequences from any cell. Genomic sequences are isolated by screening a genomic library with all or a portion of the nucleotide sequences which encode MttA1, MttA2, MttB and MttC (Figures 11 and 7). In addition to screening genomic libraries, the *mttA1, mttA2, mttB* and *mttC* nucleotide sequences can also be used to screen cDNA libraries made using RNA.

The *mttA1, mttA2, mttB* and *mttC* nucleotide sequences of the invention are also useful in directing the synthesis of MttA1, MttA2, MttB, and MttC, respectively. The MttA1, MttA2, MttB, and MttC polypeptides find use in producing antibodies which may be used in, for example, detecting cells which express MttA1, MttA2, MttB and MttC. These cells may additionally find use in directing expression of recombinant proteins to cellular membranes or to the periplasm, extracellular medium. Alternatively, cells containing at least one of MttA1, MttA2, MttB and MttC may be used to direct expression of recombinant proteins which are engineered to contain twin-arginine signal amino acid sequences, or of wild-type proteins which contain twin-arginine signal amino acid sequences, to the periplasm or extracellularly (as described below), thus reducing the likelihood of formation of insoluble proteins.

### B. MttA, MttB, and MttC polypeptides

This invention discloses the amino acid sequence of MttA1 (SEQ ID NO:47), and MttA2 (SEQ ID NO:49) which are encoded by the *mttA1* and *mttA2* genes, respectively. Data presented herein demonstrates that the protein MttA2 targets twin arginine containing proteins (*i.e.*, proteins which contain twin-arginine signal amino acid sequences), as exemplified by the proteins dimethylsulfoxide (DMSO) reductase (DmsABC) to the cell membrane (Examples 2 and 5). The function of MttA2 in membrane targeting of twin arginine containing proteins was demonstrated by isolating a pleiotropic-negative mutant in *mttA2* which prevents the correct membrane targeting of *Escherichia coli* dimethylsulfoxide reductase and results in accumulation of DmsA in the cytoplasm. DmsABC is an integral membrane molybdoenzyme which normally faces the cytoplasm and the DmsA subunit has a twin-arginine signal amino acid sequence. The mutation in *mttA2* changed proline 25 to leucine in the encoded MttA2, and was complemented by a DNA fragment encoding the *mttA2* gene.

Data presented herein further demonstrates that MttA2 also functions in selectively translocating twin arginine containing proteins, as exemplified by nitrate reductase (NapA) and trimethylamine N-oxide reductase (TorA), to the periplasm (Example 4). The mutation in the *mttA2* gene resulted in accumulation of the periplasmic proteins NapA and TorA in the cytoplasm and cell membranes. In contrast, proteins with a sec-dependent leader, as exemplified by nitrite reductase (NrfA), or which contain a twin-arginine signal amino acid sequence and which assemble spontaneously in the membrane, as exemplified by trimethylamine N-oxide (TMAO), were not affected by this mutation (Examples 2 and 4).

The isolation of mutant D-43 which contained a mutant *mttA2* gene was unexpected. The assembly of multisubunit redox membrane proteins in bacteria and eukaryotic organelles has been assumed to be a spontaneous process mediated by protein-protein interactions between the integral anchor subunit(s) and the extrinsic subunit(s) [Latour and Weiner (1987) J. Gen. Microbiol. 133:597-607; Lemire et al. (1983) J. Bacteriol. 155:391-397]. It has previously been shown that the extrinsic subunits of fumarate reductase, FrdAB, can be reconstituted to form the holoenzyme with the anchor subunits, FrdCD, in vitro without any additional proteins [Lemire et al. (1983) J. Bacteriol. 155:391-397]. Because the architecture of DMSO reductase is similar to that of fumarate reductase, it seemed likely that this protein assembled in a similar manner. However, data presented herein demonstrates that this was not the case. Thus, the isolation of mutant D-43 was unexpected and it suggests that the assembly of DmsABC needs auxiliary proteins for optimal efficiency. Alternatively, the assembly of DmsABC may be an evolutionary vestige related to the soluble periplasmic DMSO reductase found in several organisms [McEwan (1994) Antonie van Leeuwenhoek 66:151-164; McEwan et al. (1991) Biochem. J. 274:305-307].

Without limiting the invention to a particular mechanism, MttA2 is predicted to be a membrane protein with two transmembrane segments and a long periplasmic α-helix. Proline 25 is located after the second transmembrane helix and immediately preceding the long periplasmic α-helix suggesting the essential nature of this region of MttA2. Interestingly, the smallest complementing DNA fragment, pGS20, only encoded the amino terminal two thirds of MttA2. This suggests that the carboxy terminal globular domain is not necessary or can be substituted by some other activity. This conclusion is further supported by the observation that the carboxy terminal third of MttA2 is also the least conserved region of MttA2. While the amino terminal of MttA2 is homologous to YigT of Settles et al. (1997) Science 278:1467-1470, the YigT sequence was not correct throughout its length. Data presented herein shows that proteins which were homologous to MttA1 and MttA2 were identified by BLAST searches in a wide variety of archaebacteria, eubacteria, cyanobacteria and plants, suggesting that the sec-independent translocation system of which MttA1 and MttA2 are members is very widely distributed in nature.

The invention further discloses the amino acid sequence of MttB (SEQ ID NO:7) and MttC (SEQ ID NO:8). Without limiting the invention to any particular mechanism, MttB is an integral membrane protein with six transmembrane segments and MttC is a membrane protein with one or two transmembrane segments and a large cytoplasmic domain. Proteins homologous to MttB were identified by BLAST searches in a wide variety of archaebacteria, eubacteria, cyanobacteria and plants, suggesting that the protein translocation system of which MttB is a member is very widely distributed in nature. The MttC protein was even more widely dispersed with homologous proteins identified in archaebacteria, mycoplasma, eubacteria, cyanobacteria, yeast, plants, *C*. *elegans* and humans. In all cases the related proteins were of previously unknown function.

Without limiting the invention to any particular mechanism, the predicted topology of the MttABC proteins suggests that the large cytoplasmic domain of MttC serves a receptor function for twin arginine containing proteins, with the integral MttB protein serving as the pore for protein transport. Based on the observation that the MttA2 can form a long α-helix, this protein is predicted to play a role in gating the pore.

The present invention specifically contemplates variants and homologs of the amino acid sequences of MttA1, MttA2, MttB and MttC. A "variant" of MttA1, MttA2, MttB and MttC is defined as an amino acid sequence which differs by one or more amino acids from the amino acid sequence of MttA1 (SEQ ID NO:47), MttA2 (SEQ ID NO:49), MttB (SEQ ID NO:7) and MttC (SEQ ID NO:8), respectively. The variant may have "conservative" changes, wherein a substituted amino acid has similar structural or chemical properties, e.g., replacement of leucine with isoleucine. More rarely, a variant may have "nonconservative" changes, *e.g*., replacement of a glycine with a tryptophan. Similar minor variations may also include amino acid deletions or insertions (*i*.*e*., additions), or both. Guidance in determining which and how many amino acid residues may be substituted, inserted or deleted without abolishing biological or immunological activity may be found using computer programs well known in the art, for example, DNAStar software.

For example, MttA1, MttA2, MttB and MttC variants included within the scope of this invention include MttA1, MttA2, MttB and MttC polypeptide sequences containing deletions, insertion or substitutions of amino acid residues which result in a polypeptide that is functionally equivalent to the MttA1, MttA2, MttB and MttC polypeptide sequences of Figure 11 and Figure 7. For example, amino acids may be substituted for other amino acids having similar characteristics of polarity, charge, solubility, hydrophobicity, hydrophilicity and/or amphipathic nature. Alternatively, substitution of amino acids with other amino acids having one or more different characteristic may be desirable for the purpose of producing a polypeptide which is secreted from the cell in order to, for example, simplify purification of the polypeptide.

The present invention also specifically contemplates homologs of the amino acid sequences of MttA1, MttA2, MttB and MttC. An oligonucleotide sequence which is a "homolog" of MttA1 (SEQ ID NO:47), MttA2 (SEQ ID NO:49), MttB (SEQ ID NO:7) and MttC (SEQ ID NO:8) is defined herein as an oligonucleotide sequence which exhibits greater than or equal to 50% identity to the sequence of MttA1 (SEQ ID NO:47), MttA2 (SEQ ID NO:49), MttB (SEQ ID NO:7) and MttC (SEQ ID NO:8), respectively, when sequences having a length of 20 amino acids or larger are compared. Alternatively, a homolog of MttA1 (SEQ ID NO:47), MttA2 (SEQ ID NO:49), MttB (SEQ ID NO:7) and MttC (SEQ ID NO:8) is defined as an oligonucleotide sequence which encodes a biologically active MttA1, MttA2, MttB and MttC amino acid sequence, respectively.

The MttA1, MttA2, MttB and MttC polypeptide sequence of Figures 11 and 7 and their functional variants and homologs may be made using chemical synthesis. For example, peptide synthesis of the MttA1, MttA2, MttB and MttC polypeptides, in whole or in part, can be performed using solid-phase techniques well known in the art. Synthesized polypeptides can be substantially purified by high performance liquid chromatography (HPLC) techniques, and the composition of the purified polypeptide confirmed by amino acid sequencing. One of skill in the art would recognize that variants and homologs of the MttA1, MttA2. MttB and MttC polypeptide sequences can be produced by manipulating the polypeptide sequence during and/or after its synthesis.

MttA1, MttA2, MttB and MttC and their functional variants and homologs can also be produced by an expression system. Expression of MttA1, MttA2, MttB and MttC may be accomplished by inserting the nucleotide sequence encoding MttA1, MttA2, MttB and MttC (Figures 11 and 7), its variants, portions, or homologs into appropriate vectors to create expression vectors, and transfecting the expression vectors into host cells.

Expression vectors can be constructed using techniques well known in the art [Sambrook et al. (1989) Molecular Cloning, A Laboratory Manual, Cold Spring Harbor Press, Plainview NY; Ausubel et al. (1989) Current Protocols in Molecular Biology, John Wiley & Sons, New York NY]. Briefly, the nucleic acid sequence of interest is placed in operable combination with transcription and translation regulatory sequences. Regulatory sequences include initiation signals such as start (*i.e*., ATG) and stop codons, promoters which may be constitutive (*i*.*e*., continuously active) or inducible, as well as enhancers to increase the efficiency of expression, and transcription termination signals. Transcription termination signals must be provided downstream from the structural gene if the termination signals of the structural gene are not included in the expression vector. Expression vectors may become integrated into the genome of the host cell into which they are introduced, or are present as unintegrated vectors. Typically, unintegrated vectors are transiently expressed and regulated for several hours (*eg*., 72 hours) after transfection.

The choice of promoter is governed by the type of host cell to be transfected with the expression vector. Host cells include bacterial, yeast, plant, insect, and mammalian cells. Transfected cells may be identified by any of a number of marker genes. These include antibiotic (*e.g*., gentamicin, penicillin, and kanamycin) resistance genes as well as marker or reporter genes (*e.g*., β-galactosidase and luciferase) which catalyze the synthesis of a visible reaction product.

Expression of the gene of interest by transfected cells may be detected either indirectly using reporter genes, or directly by detecting mRNA or protein encoded by the gene of interest. Indirect detection of expression may be achieved by placing a reporter gene in tandem with the sequence encoding one or more of MttA1, MttA2, MttB and MttC under the control of a single promoter. Expression of the reporter gene indicates expression of the tandem one or more MttA1, MttA2, MttB and MttC sequence. It is preferred that the reporter gene have a visible reaction product. For example, cells expressing the reporter gene β-galactosidase produce a blue color when grown in the presence of X-Gal, whereas cells grown in medium containing luciferin will fluoresce when expressing the reporter gene luciferase.

Direct detection of MttA1, MttA2, MttB and MttC expression can be achieved using methods well known to those skilled in the art. For example, mRNA isolated from transfected cells can be hybridized to labelled oligonucleotide probes and the hybridization detected. Alternatively, polyclonal or monoclonal antibodies specific for MttA1, MttA2, MttB and MttC can be used to detect expression of the MttA1, MttA2, MttB and MttC polypeptide using enzyme-linked immunosorbent assay (ELISA), radioimmunoassay (RIA) and fluorescent activated cell sorting (FACS).

Those skilled in the art recognize that the MttA1, MttA2, MttB and MttC polypeptide sequences of the present invention are useful in generating antibodies which find use in detecting cells that express MttA1, MttA2, MttB and MttC or proteins homologous thereto. Such detection is useful in the choice of host cells which may be used to target recombinant twin arginine containing protein expression to cellular membranes or to the periplasm or to the extracellular medium. Additionally, such detection is particularly useful in selecting host cells for cytoplasmic or extracellular expression of recombinant twin arginine containing proteins by disrupting the function of at least one of MttA1, MttA2, MttB and MttC as described below.

### C. Methods for expressing polypeptides to produce soluble proteins

This invention contemplates methods for targeting expression (*e*.*g*., to the periplasm, extracellular medium) of any gene of interest (*e.g*., to the cytoplasm, extracellular medium) thus reducing the likelihood of expression of insoluble recombinant polypeptides, *e*.*g*., in inclusion bodies. The methods of the invention are premised on the discovery of three proteins, MttA1, MttA2, MttB and MttC which function as part of a Sec-independent pathway, and which target expression of twin arginine containing proteins to cell membranes and which direct translocation of such proteins to the periplasm of gram negative bacteria and to the extracellular medium of cells which do not contain a periplasm. This discovery makes possible methods for expression of any gene of interest such that the expressed polypeptide is targeted to the periplasm or extracellular medium thereby allowing its expression in a soluble form and thus facilitating its purification. The methods of the invention contemplate expression of any recombinant polypeptide as a fusion polypeptide with a twin-arginine signal amino acid sequence as the fusion partner. Such expression may be accomplished by introducing a nucleic acid sequence which encodes the fusion polypeptide into a host cell which expresses wild-type MttA1, MttA2, MttB or MttC, or variants or homologs thereof, or which is engineered to express MttA1, MttA2, MttB or MttC, or variants or homologs thereof. While expressly contemplating the use of the methods of the invention for the expression of any polypeptide of interest, the methods disclosed herein are particularly useful for the expression of cofactor-containing proteins. The methods of the invention are further described under (i) Cofactor-containing proteins, (ii) Expression of fusion proteins containing twin-arginine signal amino acid sequences, and (iii) Construction of host cells containing deletions or mutations in at least a portion of the genes *mttA1, MttA2, mttB* and *mttC.*

### i. Cofactor-containing proteins

A strong correlation has been reported between possession of a twin-arginine signal amino acid sequence in the preprotein and the presence of a redox cofactor in the mature protein; approximately 40 out of 135 preprotein amino acid sequences which contain a twin-arginine signal amino acid sequence have been found by Berks [Berks (1996) Molecular Microbiology 22 393-104; http://www.blackwell-science.com/products/journals/ contents/berks.htm] to result in a mature protein which binds, or can be inferred to bind, a redox cofactor.

The cofactors associated with a twin-arginine signal amino acid sequence include, but are not limited to, iron-sulfur clusters, at least two variants of the molybdopterin cofactor, certain polynuclear copper sites, the tryptophan tryptophylquinone (TTQ) cofactor, and flavin adenine dinucleotide (FAD). A representative selection of bacterial twin-arginine signal amino acid sequences is shown in Table 1.

**TABLE 1**

| | | | Evidence | Length |
|---|---|---|---|---|
| 1. PERIPLASMIC PROTEINS-BINDING IRON-SULFUR CLUSTERS | | | | |
| A. MauM family ferredoxins | | | | |
| *P. denitrificans* | MauM | | VH | 46 |
| *E. coli* | NapG | | VH | 41 |

| B. '16Fe' ferredoxin superfamily | | | | |
|---|---|---|---|---|
| *E. coli* | NrfC | MTWSRRQFLTGVGVLAAVSGTAGRVVA | VH | 27 |
| *D. vulgaris* | Hmc2 | MDRRRFLTLLGSAGLTATVATAGTAKA | VH | 27 |

| C. High potential iron protein (HiPIP) | | | | |
|---|---|---|---|---|
| *T*. *ferroaxidans* | Iro | | EX | 37 |

| D. Periplasmically-located \|Fe\| hydrogenase small subunits | | | | |
|---|---|---|---|---|
| *D. vulgaris* | HydB | | VH | 34 |

| E. Peripllasmically-located \|NiFe\| hydrogenase small subunits | | | | |
|---|---|---|---|---|
| *E*. *coli* | HyaA | | EX | 45 |
| *+M. mazei* | VhoG | | EX | 48 |
| *D. gigas* | HynB | | EX | 50 |
| *E*. *coli* | HybA | MNRRNFIKAASCGALLTGALPSVSHA | VH | 26 |

| F. Membrane-anchored Rieske proteins | | | | |
|---|---|---|---|---|
| *P. denitrificans* | FbcF | | | |
| +*Synechocystis* | PetC | | | |
| +*S. acidocaldarius* | SoxF | MD**RR**T**FL**RLYLLVGAAIAVAPVIKPALDYVGY | | |

| **II. PERIPLASMIC PROTEINS BINDING THE MOLYBDOPTERIN COFACTOR** | | | | |
|---|---|---|---|---|
| **A. Molybdopterin guanine dinucleotide-binding proteins, some of which also bind an iron-sulfur cluster** | | | | |
| *R. sphaeroides* | DmsA | | EX | 42 |
| *E. coli* | BisZ | MTL**TRR**E**F**I**K**HSGIAAGALVVTSAAPLPAWA | VH | 31 |
| *T. pantotropha* | NapA | MTIS**RR**DL**LK**AQAAGIAAMAANIPLSSQAPA | VH | 31 |
| *W. succinogenes* | FdhA | | EX | 32 |
| *E*. *coli* | DmsA | | EX | 45 |
| *H. influenzae* | DmsA | MSNFNQIS**RR**D**F**V**K**ASSAGAALAVSNLTLPFN VMA | VH | 35 |
| *S. typhimurium* | PhsA | MSI**SRR**S**FL**QGVGIGCSACALGAFPPGALA | VH | 30 |

| **B. Molybdopterin cytosine dinucleotide-binding proteins** | | | | |
|---|---|---|---|---|
| *P. diminuta* | IorB | | VH | 37 |
| *A. polyoxogenes* | Ald | | EX | 44 |

| **III. PERIPLASMIC ENZYMES WITH POLYNUCLEAR COPPER SITES** | | | | |
|---|---|---|---|---|
| **A. Nitrous oxide reductases** | | | | |
| *P. stutzeri* | NosZ | | EX | 50 |

| **B. Multicopper oxidase superfamily** | | | | |
|---|---|---|---|---|
| *P. syringae* | CopA | | VH | 32 |
| *E. coli* | Sufl | MSLSRRQFIQASGIALCAGAVPLKASA | VH | 27 |

| **IV. METHYLAMINE DEHYDROGENASE SMALL SUBUNITS (TRYPTOPHAN TRYPTOPHYLQUINONE COFACTOR)** | | | | |
|---|---|---|---|---|
| *M. extorquens* | MauA | | EX | 57 |

| **V. PERIPLASMIC PROTEINS BINDING FLAVIN ADENINE DINUCLEOTIDE** | | | | |
|---|---|---|---|---|
| *C*. *vinosum* | FccB | MTLN**RR**D**F**I**K**TSGAAVAAVGILGFPHLAFG | EX | 30 |
| +*B. sterolicum* | ChoB | | EX | 45 |

A more complete listing of bacterial twin-arginine signal amino acid sequences is available at http://www. blackwell-science, com/products/journals/mole. htm. Amino acids with identity to the most preferred (S/T)-RR-x-F-L-K consensus motif are indicated in bold. Signal sequences are from Proteobacterial preproteins except where indicated (+). 'Evidence' indicates the method used to determine the site of protease processing: EX, experimentally determined; VH, inferred using the algorithm of von Heijne (1987). [1] van der Palen et al. (1995); [2] Richterich et al. (1993); [3] Hussain et al. (1994); [4] Rossi et al. (1993); [5] Kusano et al. (1992); [6] Voordouw et czl. (1989); [7] Menon et al. (1990); [8] Deppenmeier et al. (1995); [9] Li et al. (1987); [10] Menon et al. (1994); [11] Kurowski and Ludwig (1987); [12] Mayes and Barber (1991) : [13] Castresana et al, (1995); [14] Hilton and Rajagopalan (1996); [15] Campbell and Campbell (1996); [16] Berks et al. (1995a); [17] Bokranz et al. (1991); [18] Bilous et al. (1988); [19] Fleischmann et al. (1995); [20] Heinzinger et al. (1995); [21] Lehmann et al. (1995); [22] Tamaki et al. (1989); [23] Viebrock and Zumft (1988); [24] Mellano and Cooksey (1988); [25] Plunkett (1995); [26] Chistoserdov and Lidstrom (1991); [27] Dolata et al. (1993); [28] Ohta et al. (1991).

In contrast to twin-arginine signal amino acid sequences, Sec signal sequences are associated with periplasmic proteins binding other redox cofactors, i, e., iron porphyrins (including the cytochromes c), mononuclear type I or II copper centers, the dinuclear Cu_{A} center, and the pyrrolo-quinoline quinone (PQQ) cofactor.

Currently the assembly of cofactor-containing proteins is limited to the cytoplasm because the machinery to insert the cofactor is located in this compartment. The present invention offers the advantage of providing methods for periplasmic and extracellular expression of cofactor-containing proteins which contain a twin-arginine signal amino acid sequence, thus facilitating their purification in a functional and soluble form.

### ii. Expression of fusion proteins containing twin-arginine signal amino acid sequences

The methods of the invention exploit the inventors' discovery of proteins MttA1, MttA2, MttB and MttC which are involved in targeting expression of proteins which contain a twin-arginine amino acid signal sequence to cell membranes and in translocation of such proteins to the periplasm of gram negative bacteria and the extracellular medium of cell that do not contain a periplasm. The term"twin-arginine signal amino acid sequence"as used herein means an amino acid sequence of between 2 and about 200 amino acids, more preferably between about 10 and about 100 amino acids, and most preferably between about 25 and about 60 amino acids, and which comprises the amino acid sequence, from the N-terminal to the C-terminal, A-B-C-D-E-F-G, wherein the amino acid at position B is Arg, and the amino acid at position C is Arg. The amino acid at positions A, D, E, F, and G can be any amino acid. However, the amino acid at position A preferably is Gly, more preferably is Glu, yet more preferably is Thr, and most preferably is Ser. The amino acid at position D preferably is Gln, more preferably is Gly, yet more preferably is Asp, and most preferably is Ser. The amino acid at position E preferably is Leu and more preferably is Phe. The amino acid at position F preferably is Val, more preferably is Met, yet more preferably is Ile, and most preferably is Leu. The amino acid at position G preferably is Gln, more preferably is Gly and most preferably is Lys, In one preferred embodiment, the twin-arginine amino acid signal sequence is Ser-Arg-Arg-Ser-Phe-Leu-Lys (SEQ ID NO: 41). In yet another preferred embodiment, the twin-arginine amino acid signal sequence is Thr-Arg-Arg-Ser-Phe-Leu-Lys (SEQ ID NO: 42).

The invention contemplates expression of wild-type polypeptide sequences which contain a twin-arginine amino acid signal sequence as part of a preprotein. To date, 135 polypeptide sequences have been reported to contain a twin-arginine amino acid signal sequence motif [Berks (1996) Molecular Microbiology 22 393-104; http://www. blackwell-science. com/products/journals/contents/berks.htm].

The invention further contemplates expression of recombinant polypeptide sequences which are engineered to contain a twin-arginine amino acid signal sequence as part of a fusion protein. Fusion protein containing one or more twin-arginine amino acid signal sequences may be made using methods well known in the art. For example, one of skill in the art knows that nucleic acid sequences which encode a twin-arginine amino acid signal sequence may be operably ligated in frame (directly, or indirectly in the presence of intervening nucleic acid sequences) to a nucleotide sequence which encodes a polypeptide of interest. The ligated nucleotide sequence may then be inserted in an expression vector which is introduced into a host cell for expression of a fusion protein containing the polypeptide of interest and the twin-arginine amino acid signal sequence.

Fusion proteins containing twin-arginine amino acid signal sequences are expected to be targeted to the periplasm or extracellular medium by the MttA1, MttA2, MttB and MttC proteins of the invention and by variants and homologs thereof; Keon and Voordouw [Keon and Voordouw (1996) Anaerobe 2:231-238] have reported that a fusion protein containing *E*. *coli* alkaline phosphatase (phoA) linked to a signal amino acid sequence from the Hmc complex of *Desulfovibrio vulgaris* subsp. *vulgaris* was exported to *E. coli* periplasm. Similarly, a fusion protein containing a hydrogenase signal peptide to β-lactamase from which the signal peptide had been removed led to export in *E. coli* under both aerobic and anaerobic conditions [Niviere et al. (1992) J. Gen. Microbiol. 138:2173-2183].

Fusion proteins which contain twin-arginine amino acid signal sequences are also expected to be cleaved to generate a mature protein from which the twin-arginine amino acid signal sequences has been cleaved. Two signal peptidases have so far been identified in *E*. *coli*: Signal peptidase I and signal peptidase II. The signal peptidase II which has a unique cleavage site involving a cystine residue at the cleavage site [Bishop et al. (1995) J. Biol. Chem. 270:23097-23103] is believed not to participate in cleavage of twin-arginine amino acid signal sequences. Rather, signal peptidase I, which cleaves Sec signal sequences has been suggested by Berks to cleave twin-arginine amino acid signal sequences. Berks also suggested that signal peptidase I has the same recognition site in Sec signal sequences as in twin-arginine amino acid signal sequences [Berks (1996)]. This suggestion was based on (a) the "-1/-3" rule for Sec signal peptidase in which the major determinant of signal peptidase processing is the presence of amino acids with small neutral side-chains at positions -1 and -3 relative to the site of cleavage, and (b) the good agreement between the cleavage site of twin-arginine amino acid signal sequences as determined using the "-1/-3" rule (with the invariant arginine at the N-terminus of the signal sequence, i.e., position B in the A-B-C-D-E-F-G sequence, designated as position zero) and the experimentally determined amino terminus of the mature protein [Berks (1996)]. Evidence presented herein (Example 9) further confirms cleavage of twin-arginine amino acid signal sequences to release a mature protein which lacks the twin-arginine amino acid signal sequence.

### iii. Construction of host cells containing deletions or mutations in at least a portion of the genes mttA, mttB and mttC

The function of any portion of *E*. *coli* MttA1, MttA2, MttB and MttC polypeptides and variants and homologs thereof, as well as the function of any polypeptide which is encoded by a nucleotide sequence that is a variant or homolog of the *mttA1, MttA2, mttB* and *mttC* sequences disclosed herein may be demonstrated in any host cell by *in vivo* homologous recombination of chromosomal sequences which are variants or homologs of *mttA1, MttA2. mttB* and *mttC* using previously described methods [Sambasivarao et al (1991) J. Bacteriol. 5935-5943; Jasin et al (1984) J. Bacteriol. 159:783-786]. Briefly, the nucleotide sequence whose function is to be determined is cloned into vectors, and the gene is mutated. *e.g*., by insertion of a nucleotide sequence within the coding region of the gene. The plasmids are then homologously recombined with chromosomal variants or homologs of *mttA1, MttA2, mttB* or *mttC* sequences in order to replace the chromosomal variants or homologs of *mttA1, MttA2, mttB* or *mttC* genes with the mutated genes of the vectors. The effect of the mutations on the localization of proteins containing twin-arginine amino acid signal sequences is compared between the wild-type host cells and the cells containing the mutated *mttA1, MttA2, mttB* or *mttC* genes. The localization (*e*.*g*., cytoplasm, periplasm, cell membranes, extracellular medium) of expressed twin arginine containing proteins is compared using methods disclosed herein (*e*.*g*., functional enzyme activity and Western blotting) between homologously recombined cells and control cells which had not been homologously recombined. Localization of expressed twin arginine containing proteins extracellularly, in the periplasm, or in the cytoplasm of homologously recombined cells as compared to localization of expression in cell membranes of control cells demonstrates that the wild-type MttA1, MttA2, MttB or MttC protein whose function had been modified by homologous recombination functions in targeting expression of the twin arginine containing protein to the cell membrane. Similarly, accumulation of expressed twin arginine containing proteins in extracellular medium, in the cytoplasm, or in cell membranes of homologously recombined cells as compared to periplasmic localization of the expressed twin arginine containing protein in control cells which had not been homologously recombined indicates that the protein (i. e.. MttA1, MttA2, MttB or MttC) whose function had been modified by homologous recombination functions in translocation of the twin arginine containing protein to the periplasm.

### EXPERIMENTAL

The following examples serve to illustrate certain preferred embodiments and aspects of the present invention and are not to be construed as limiting the scope thereof. The strains and plasmids used in this investigation are listed in Table 2.

**TABLE 2**

| **Bacteria and Plasmids used in this Investigation** | | |
|---|---|---|
| Strain/Plasmid | Genotype or Gene Combinations Present | Reference/Source |
| HB101 | *F-, hsdS20(r-_{B}m-_{B}), leu, supE44, aral4,galK2, lacY1, proA2, rpsL20, xyl-5, mtl-1, recA13, mcrB* | Boyer and Roulland-Dussoix, 1969 |
| TG1 | K 12Δ*(lac-pro) sup EF' traD36 proAB lacl^{q}* Δ*lacZM15* | Amersham Corp. |
| D43 | HB101; *mttA* | Bilous and Weiner, 1985 |
| pBR322 | cloning vector Tet^{r}, Amp^{r} | Pharmacia |
| pTZ18R | cloning vector Amp^{r}, *lacZ* | Pharmacia |
| pJBS633 | *blaM* fusion vector | Broome-Smith and Spratt, 1986 |
| pFRD84 | *frdABCD* cloned into pBR322 | Lemire *et al*., 1982 |
| pFRD117 | Δ*ftdCD* version of pFRD84 | Lemire *et al*., 1982 |
| pDMS160 | *dmsABC* cloned into pBR322 | Rothery and Weiner, 1991 |
| pDMS223 | *dmsABC* operon in pTZ18R | Rothery and Weiner, 1991 |
| pDMSL71 | *dmsABC::blaM* in pJBS633 fusion after residue 12 | Weiner *et al*., 1993 |
| pDMSL5 | *dmsABC::blaM* in pJBS633 fusion after residue 216 | Weiner *et al*., 1993 |
| pDMSL29 | *dmsABC::blaM* in pJBS633 fusion after residue 229 | Weiner *et al*., 1993 |
| pDMSL4 | *dmsABC::blaM* in pJBS633 fusion after residue 267 | Weiner *et al*., 1993 |
| pDMSC59X | *dmsC* truncate after residue 59 | Sambasivarao and Weiner, 1991 |
| pDSR311 | *yigO,P, R, T* and *U* in pBR322 | This investigation |
| pGS20 | b3835', b3836, b3837, and b3838' in pBR322 | This investigation |
| pTZmttABC | region of ORF's b3836, b3838, yigU, yigW, cloned into pTZ18R | This investigation |
| pBRmttABC | region of ORF's b3836, b3838, yigU, yigW, cloned into pBR322 | This investigation |
| pTZb3836 | ORF b3836 cloned into pTZ18R | This investigation |
| pBRb3836 | ORF b3836 cloned into pBR322 | This investigation |

### EXAMPLE 1 (REFERENCE)

### Isolation And Properties of D-43 Mutants Defective In DmsABC Targeting

DMSO reductase is a "twin arginine" trimeric enzyme composed of an extrinsic membrane dimer with catalytic, DmsA, and electron transfer, DmsB, subunits bound to an intrinsic anchor subunit, DmsC. The DmsA subunit has a "twin arginine" leader but it has been exhaustively shown that the DmsA and DmsB subunits face the cytoplasm [Rothery and Weiner (1996) Biochem. 35:3247-3257; Rothery and Weiner (1993) Biochem, 32:5855-5861; Sambasivarao et al. (1990) J. Bacteriol. 172:5938-5948; Weiner et al. (1992) Biochem. Biophys, Acta 1102:1-18; Weiner et al. (1993) J. Biol. Chem. 268:3238-3244].

In order to isolate a *E. coli* mutant defective in membrane targeting of DmsABC, plieotropic mutants which were unable to grow on DMSO were produced by nitrosoguanidine mutagenesis of HB101 and the growth rates on DMSO of both the mutants and HB101 were determined. Mutant D-43, which grew anaerobically on fumarate and nitrate, nevertheless failed to grow on DMSO or TMAO. These results are further described in the following sections.

### A. Isolation of mutant

Nitrosoguanidine mutagenesis and ampicillin enrichment were as described by Miller (1992) in A Short Course in Bacterial Genetics, Cold Spring Harbor Laboratory Press. Sixteen mutants were isolated that were defective for anaerobic growth on DMSO but grew with nitrate or fumarate as the alternate electron acceptor. Each of the mutants was transformed with pDMS160 [Rothery and Weiner (1991) Biochem. 30:8296-8305] carrying the entire *dms* operon and again tested for growth on DMSO. All of the transformants failed to grow on DMSO, When tested for DMSO reductase activity 14 of the 16 transformants lacked measurable enzyme activity. Two of the mutants expressed high levels of DMSO reductase activity but the activity was localized in the cytoplasm rather than the membrane fraction. One of these mutants, D-43, was chosen for further study.

### B. Anaerobic growth rates of HB101 and D-43

For growth experiments, bacteria were initially grown aerobically overnight at 37°C in LB plus 10 µg/ml⁻¹ vitamin B1. A 1% inoculum was added to 150 ml of minimal salts medium containing 0.8% (w/v) glycerol. 10 µg/ml⁻¹ each of proline, leucine, vitamin B1 and 0.15% peptone and supplemented with either DMSO 70 mM, fumarate 35 mM, nitrate 40 mM, or trimethylamine N-oxide (TMAO) 100mM. Cultures were grown anaerobically at 37°C in Klett flasks and the turbidity monitored in a Klett spectrophotometer with a No, 66 filter.

The rates of anaerobic growth of strains HB101 and D-43 with a range of electron acceptors and a nonfermentable carbon source, glycerol, were compared. The results are shown in Figure 1.

All the terminal electron acceptors tested supported the growth of the parent HB101 (Figure 1a). In contrast, only nitrate and fumarate stimulated the growth rate of the mutant (Figure 1b). However, even in the presence of nitrate and furnarate the growth yield was half that of strain HB101. The reduced growth rate may reflect the pleiotropic effects of the mutation of various metabolic reactions needed for optimal growth in addition to the terminal electron transfer reaction. Only DmsABC supports growth on DMSO whereas both DmsABC and the periplasmic TMAO reductase support growth on TMAO [Sambasivarao and Weiner (1991) J. Bacteriol. 173:5935-5943]. The observation that D-43 is unable to grow on either DMSO or TMAO indicates that both of these enzymes were non-functional.

### EXAMPLE 2 (REFERENCE)

### DmsA Is Not Anchored To the Membrane In D-43

Previous studies have exhaustively shown that DmsABC is localized on the cytoplasmic membrane of wild-type *E. coli* stains with the DmsAB subunits anchored to the cytoplasmic surface [Rothery and Weiner (1996) Biochem. 35:3247-3257; Rothery and Weiner (1993) Biochem. 32:5855-5861; Sambasivarao et al. (1990) J. Bacteriol. 172:5938-5948; Weiner et al. (1992) Biochem. Biophys. Acta 1102:1-18; Weiner et al, (1993) J. Biol.Chem. 268:3238-3244]. In order to determine he localization of DmsABC in D-43 mutants, cell fractions were assayed for the presence of DmsA and DmsB by immunoblot analysis, and for DMSO reductase activity as follows.

### A. Functional enzyme activity assays

Cell fractions were assayed for DMSO reductase activity by measuring the DMSO-dependent oxidation of reduced benzyl viologen at 23°C [Bilous and Weiner (1985) J. Bacteriol. 162:1151-1155]. This assay is dependent only on the presence of DmsAB.

To test the localization of DmsABC in D-43, enzyme activity in the soluble fraction and membrane band fraction of HB101/pDMS160 and of D-43/pDMS160 was determined. 250 ml anaerobic cultures of HB101/pDMS160 and D-43/pDMS160 were grown on Gly/Fum medium. HB101/pDMS160 yielded 114 mg total protein, 3240 units of membrane-bound TMAO reductase activity, and 2900 units of soluble activity. D-43/pDMS160 yielded 99 mg total protein, 320 units were membrane-bound and 4000 units were soluble. Thus, although the total DmsABC activity was lower in D-43, (4300 total units compared to 6200 for HB101/pDMS160) the vast majority was not targeted to the membrane. This suggested that D-43 was defective in targeting to the membrane rather than in a biosynthetic step.

### B. Western blot analysis of DmsA and DmsB

To determine the cellular locations of DmsA and DmsB by Western blots. D-43/pDMS160 and HB101/pDMS160 were grown anaerobically on Gly/fumerate medium at 37°C in 19 1 batches [Bilous and Weiner (1985) J. Bacteriol. 162:1151-1155]. Cultures were grown for 24hr, at 37°C and the cells harvested and membranes prepared by French pressure cell lysis at 16,000 psi followed by differential centrifugation as previously described [Rothery and Weiner (1991) Biochem. 30:8296-8305]. The crude membranes were washed twice with lysis buffer (50 mM MOPS, 5 mM EDTA pH 7.0). DmsABC was purified as described by Simala-Grant and Weiner (1996) Microbiology 142:3231-3229. For the determination of subunit anchoring to the membrane, membrane preparations were first washed with lysis buffer and then with lysis buffer containing 1 M NaCI. The osmotic shock procedure of Weiner and Heppel (1971) J. Biol. Chem. 246:6933-6941) was used to isolate the periplasmic fraction tested for fumarate and DMSO reductase polypeptides.

For Western blot analysis, antibodies to purified DmsA and DmsB were used [Sambasivarao et al. (1990) J. Bacteriol. 172:5938-5948]. Typically, samples were separated on 10% (w/v) SDS-PAGE and then blotted onto nitrocellulose. The protein bands were detected using the enhanced chemiluminescence detection system from Amersham and goat anti-rabbit 1gG (H+L) horseradish peroxidase conjugate. The results are shown in Figure 2.

Figure 2 shows a Western blot of washed membranes and soluble fractions of HB101 and D-43 harboring pDMS160 expressing DmsABC. The blot was probed with either purified anti-DmsA or anti-DmsB. S; soluble fraction, M; Washed membranes, sM; salt washed membranes, sS; soluble fraction from the salt washed membranes, P; purified DmsABC. Figure 2 clearly shows that DmsA is not targeted to the membrane in D-43. The DmsA polypeptide was expressed and was present in the cytoplasm at levels equivalent to the wild-type. Equivalent samples probed with anti-DmsB demonstrated that significant amounts of DmsB were targeted to the membrane. Membrane incorporation of DmsC in the absence of DmsAB is lethal [Turner et al. (1997) Prof. Engineering 10:285-290] and the presence of DmsB on the membrane may overcome the lethality normally associated with incorporation of DmsC in the absence of the catalytic subunits.

### EXAMPLE 3 (REFERENCE)

### DmsC Is Anchored To the Membrane In D-43

Because polyclonal antibodies against DmsC could not successfully be raised [Sambasivarao et al. (1990) J. Bacteriol. 172:5938-5948; Turner et al. (1997) Pro., Engineering 10:285-290], three BlaM (β-lactamase) fusions were used to determine whether the anchor subunit is translated and correctly inserted into the membranes of D-43 [Weiner et al. (1993) J. Biol. Chem. 268:3238-3244]. These fusions were located after amino acid positions 216, 229 and 267 of DmsC. Fusion 216 was localized to the periplasm and mediated very high resistance. Fusions 229 and 267 were localized to the seventh and eighth transmembrane helices and mediated intermediate levels of resistance [Weiner et al. (1993) J. Biol. Chem. 268:3239-3244]. The minimal inhibitory concentrations of ampicillin. for each of these fusions expressed in D-43 under anaerobic growth conditions, were the same or within one plate dilution of the wild-type values. Additionally, Western blots, using antibody directed against BlaM, of cell fractions of membrane, cytoplasmic and osmotic shock fluids of D-43/pDMSL29 (fusion at amino acid 229) showed DmsC-BlaM in the membrane fractions (results not shown). These data suggest that the DmsC protein is translated and inserted into the membrane and has the same topology as that found in wild-type *E. coli* cells.

### EXAMPLE 4 (REFERENCE)

### Enzyme Activity Of Nitrate Reductase and Trimethylamine N-Oxide Reductase With A Twin Arginine Signal Sequence Is Not Targeted To the Periplasm Of D-43 While Enzyme Activity of Nitrite Reductase With A Sec-Signal Sequence Is Present In the Periplasm Of D-43

In order to determine whether the mutation in D-43 (which resulted in failure to anchor DmsA. and DmsB to the cell membrane as described above) selectively prevented membrane targeting of proteins with a twin-arginine signal amino acid sequence, the enzyme activity of periplasmic enzymes having a twin-arginine signal amino acid sequence (*i*.*e*., nitrate reductase (NapA) and trimethylamine N-oxide reductase (TorA)) and of a periplasmic enzyme having a Sec-leader sequence (*i.e*., nitrite reductase (NrfA)) was determined in the periplasm of D-43 and HB101.

*E. coli* can reduce nitrate to ammonia using two periplasmic electron transfer chains, the Nap and Nrf pathways [Grove et al. (1996) Mol. Microbiol. 19:467-481; Cole (1996) FEMS Microbiol. Letts. 136:1-11]. The catalytic subunit of the periplasmic nitrate reductase, NapA. is a large molybdoprotein with similarity to DmsA and is synthesized with a twin-arginine signal amino acid sequence. NrfA, the periplasmic nitrite reductase, is not a molybdoprotein but a c-type cytochrome and contains a *Sec*-leader peptide. Accumulation of both of these redox enzymes in the periplasm of strain D-43 was assayed by staining the periplasmic proteins separated by PAGE with reduced methyl viologen in the presence of nitrate and nitrite as follows.

Periplasmic proteins were released from washed bacterial suspensions as described by McEwan et al. (1984) Arch. Microbiol. 137:344-349 except that the EDTA concentration was 5 mM. The periplasmic fraction was dialyzed against two changes of a 20-fold excess of 10 mM Na+/K+ phosphate, pH 7.4 to remove sucrose and excess salt, freeze dried and dissolved in 10 mM phosphate pH 7.4 to a protein concentration of about 15 mg/ml⁻¹. Protein concentrations were determined by the Folin phenol method described previously [Newman and Cole (1978) J. Gen. Microbiol. 106:1-12]. The periplasmic proteins were separated on a 7.5% non-denaturing polyacrylamide gel. After electrophoresis, the 18 cm square gel was immersed in 5 µg ml⁻¹ methyl viologen containing 5 mM nitrate. Dithionite was added to keep the viologen reduced; bands of activity were detected as transparent areas against a dark purple background. The same protocol was used to detect periplasmic nitrite and TMAO reductase activity but 5 mM nitrate was replaced by 2.5 mM nitrite or 5 mM TMAO, respectively. The results are shown in Figure 3.

Figure 3a shows A nitrate-stained polyacrylamide gel containing periplasmic proteins, membrane proteins and cytoplasmic proteins from HB101 and D-43. Lanes 1) and 2) contain periplasmic proteins from HB101 and D-43, respectively. Lanes 3) and 4) contain membrane proteins from HB101 and D-43, respectively and lanes 5) and 6) contain soluble cytoplasmic proteins from HB101 and D-43, respectively. Figure 3b shows nitrite-stained polyacrylamide gel containing periplasmic proteins from 1) HB101 and 2) D-43. Approximately 30 µg of protein was loaded into each lane. Figure 3c shows TMAO-stained polyacrylamide gel containing periplasmic proteins from 1) HB101 and 2) D-43.

The results in Figure 3 show that nitrate reductase activity due to NapA was present in the periplasmic proteins extracted from the parental strain HB101 but was not observed in periplasmic proteins prepared from strain D-43 (Figure 3a). In contrast, activity of NrfA. the c-type cytochrome nitrite reductase, was similar in periplasmic proteins prepared from both HB101 and D-43 (Figure 3b). Significantly, the nitrate reductase activity was higher in membranes prepared from strain D-43 than in membranes prepared from the parental strain HB101, suggesting that NapA protein was "stuck" in the membrane fraction. No nitrate reductase activity was detected in soluble cytoplasmic proteins prepared from either strain (data not shown).

Additionally, the rate of electron transfer from physiologic electron donors to NrfA was measured by assaying the rate of nitrite reduction by a suspension of whole cells in the presence of formate or glycerol. The effects of the mutation on periplasmic nitrite reductase activity provided a key control to test whether MttA2 plays a major role in protein targeting. Nrf activity can be assessed in two ways: by detecting the activity of the terminal nitrite reductase which is a c-type cytochrome secreted by the Sec pathway and assembled in the periplasm (Figure 3b) [Thony-Meyer and Kunzler (1997) Eur. J. Biochem. 246:794-799], and by measuring the rate of nitrite reduction by washed bacteria in the presence of the physiologic substrate, formate. Only the latter activity requires the membrane-bound iron-sulfur protein, NrfC, which is synthesized with an N-terminal twin-arginine signal amino acid sequence.

The rate of nitrite reduction in suspensions of strain HB101 was 34 µmol nitrite reduced/min⁻¹/ml⁻¹ while that measured with suspensions of D-43 was 11 µmol nitrite reduced/min⁻¹/ml⁻¹. These results show that cytochrome *c*₅₅₂ was correctly targeted in the mutant and able to catalyse nitrite reduction with dithionite-reduced methyl viologen as the artificial electron donor, but strain D-43 was deficient in formate-dependent nitrite reductase activity.

Loss of electron transport to NrfA from physiologic electron donors, but not from reduced methyl viologen was probably due to the presence of a twin-arginine signal amino acid sequence motif in either NrfC, which is a protein essential for the transfer of electrons from quinones to NrfA [Hussain et al. (1996) Mol. Microbiol. 12:153-163] or in FdnG which contributes to the transfer of electrons from formate to nitrite [Darwin et al. (1993) J. Gen. Microbiol. 139:1829-1840].

Trimethylamine N-oxide reductase (TorA) is another periplasmic terminal reductase related to DmsA [Mejean et al. (1994) Mol. Microbiol. 11:1169-1179] which contains a twin-arginine signal amino acid sequence. In strain D-43 this enzyme activity was not observed in the periplasmic protein fraction (Figure 3c).

### EXAMPLE 5

### MttA2 Protein Targets DmsAB To The Membrane And Does Not Translocate DmsAB To The Periplasm

In order to determine whether MttA2 is involved in targeting DmsAB to the membrane rather than in the translocation of DmsAB to the periplasm, and whether the role of DmsC is to prevent translocation of DmsAB to the periplasm, the intracellular location was examined in HB101 and D-43 for the DmsA and DmsB subunits expressed from a plasmid encoding the wild-type DmsABC operon as well as a truncated form lacking the anchor subunit DmsC. The results are shown in Figure 4.

Figure 4 shows a Western blot of DmsAB. Figure 4A shows HB101 expressing either native DmsABC (pDMS160), DmsABΔC (pDMSC59X), or FrdABΔCD. Figure 4B shows equivalent lanes as in Figure 4A, with the same plasmids in D-43. P; purified or enriched sample protein of either DmsABC or FrdAB, M; washed membranes. S; soluble fraction. O; osmotic shock fraction, 20; 2 fold osmotic shock fraction. Purified FrdAB was obtained from HB101/pFRD84 expressing high levels of the wild-type enzyme and purified by the method of [Dickie and Weiner (1979) Can. J. Biochem. 57:813-821; Lemire and Weiner (1986) Meth. Enzymol. 126:377-386]. All lanes had the equivalent concentration of protein loaded.

As shown in Figure 4A, (compare lanes 8 and 9 to lanes 4 and 5) significant amounts of DmsA and DmsB accumulated in the periplasm only when the DmsC subunit was absent. As a control for this experiment, plasmids carrying the intact *frdABCD* (pFRD84) (not shown) and truncated *frdAB* (pFRD 117) [Lemire et al. (1982) J. Bacteriol. 152:1126-1131] lacking the anchor subunits of fumarate reductase were also expressed. As fumarate reductase does not have a twin-arginine signal amino acid sequence and assembles spontaneously in the membrane [Latour and Weiner (1987) J. Gen. Microbiol. 133:597-607] neither a Mtt mutation, nor loss of the anchor subunits, FrdC and FrdD, should result in secretion of FrdAB into the periplasm. This was confirmed (lanes 13 and 14). In Figure 4B the same experiment is shown for strain D-43. As expected neither DmsA nor DmsB accumulated in the periplasm.

These results demonstrate that MttA is not involved in the translocation of DmsAB to the periplasm but in targeting them to the membrane. These results also suggest that the role of DmsC is to prevent translocation of DmsAB to the periplasm.

### EXAMPLE 6

### Plasmid Complementation Of D-43 And Sequencing Of The mttA Region

Complementation of the D-43 mutant with plasmid pDMS160 (which carries the wild-type DmsABC operon) was carried out to determine whether the mutation was located within or outside the DmsABC structural gene.

### A. Plasmid complementation of mutant D-43

For initial complementation experiments, an *E. coli* DNA library was prepared by Hindlll digestion of an *E. coli* HB101 chromosomal DNA preparation and ligated into the Hindll site of pBR322. The ligation mixture was transformed directly into D-43. The transformants were grown anaerobically on glycerol/DMSO (Gly/DMSO) plates and incubated anaerobically at 37°C for 72 hr. The complementing clone identified form this library, pDSR311, was isolated and restriction mapped. The map was compared with the integrated *E. coli* restriction map version 6 [Berlyn et al. (1996) Edition 9 in Escherichia coli and Salmonella 2:1715-1902, ASM Press, Washington DC].

A second gene bank was prepared using random 5-7 kb Sau3a fragments of *E*. *coli* W1485 ligated into the BamHI site of pBR322. This *E. coli* gene bank was a gift from Dr. P. Miller. Parke-Davis Pharmaceuticals, Ann Arbor, MI. D-43 was transformed with 2 µg of this library and transformants were plated onto Luria-Bertani (LB) broth plates containing 100 µg/ml⁻¹ ampicillin. After overnight growth at 37°C the cells were washed off the plates into 5 ml of LB broth and 20 µl of this suspension was diluted with 10 ml of Minimal A medium [Miller (1992) in A Short Course in Bacterial Genetics, Cold Spring Harbor Laboratory Press] containing 100 µg/ml⁻¹ ampicillin and 10 µg/ml⁻¹ vitamin B1, proline and leucine and grown aerobically at 37°C for 16 hr. The cells were washed twice in phosphate buffered saline (PBS) and samples were serially diluted into PBS buffer. Each dilution (100 µl) was plated on Gly/DMSO plates and incubated anaerobically at 37°C for 72 hr. Colonies were further tested for anaerobic growth in 9 ml screw-top test tubes containing Gly/DMSO broth medium.

The location of the complementing clones in the *E*. *coli* chromosome obtained from both libraries was confirmed by DNA sequencing the ends of the clones using primers which flanked the HindIII and BamHI sites of pBR322. Subclones of the complementing clones from each of the libraries were constructed utilizing standard cloning methods [Sambrook *et al.* (1989)] and ligated into the cloning vector pTZ18R. DNA from subclones was restriction mapped to verify the insert. Positive subclones were tested for anaerobic growth in Gly/DMSO and Gly/Fumarate broth medium.

A single clone, pDSR311, which allowed growth on Gly/DMSO was identified. Through restriction map analysis and sequencing the ends of the insert. the clone was mapped to the 88 min region of the chromosome, within contig AE00459 covering the 4,013,851-4.022,411 bp region of the sequence of Blattner et al. [Blattner et al. (1997) Science 277:1453-1462]. The clone contained the previously undefined open reading frames *yigO, P, R, T*, and *U* (based on the original *yig* nomenclature for unidentified ORFs) (Figure 5).

All attempts to use available restriction sites to subclone this region into ORF groups *yigOP, yigR, yigRTU,* and *yigTU* were unsuccessful. Therefore, a second library consisting of *E. coli* chromosomal DNA which had been partially-digested with Sau3a was ligated into BamHI- digested pBR322. This library generated a number of complementing clones. The smallest was pGS20 which encoded the 3' end of *yigR* and approximately three quarters of *yigT* as shown in Figure 5. This suggested that the products of the putative genes *yigTUW* were responsible for DmsA targeting to the membrane and Nap translocation to the periplasm and these genes were renamed *mttABC* (membrane targeting and translocation). This region was cloned from wild-type HB101 utilizing PCR as follows.

For PCR cloning of the *mttABC* region, the chromosomal DNA template for PCR was prepared from HB101. Bacteria from 1.5 ml of an overnight culture were pelleted in an Eppendorf tube and resuspended in 100 µl of water. The cells were frozen and thawed three times, pelleted by centrifugation and 5 µl of the supernatant was used as the PCR template.

The region of the putative *mttABC* operon was cloned utilizing PCR. The 5' primer was located at the end of the coding sequence for *yigR*(b3835) (position 5559-5573 of contig AE00459) and included the intervening sequence between *yigtR* and *mttA*. The 3' primer hybridized immediately after the stop codon of *mttC* (position 8090-8110). The primers contained the restriction sites EcoRI and SaII to facilitate cloning into the phagemid pTZ18R and recombinants were screened in *e. coli* strain TGI. The ends of the clones were sequenced to verify the region cloned.

Clones of the ORF region *mttABC* were subcloned utilizing standard cloning methods [Sambrook *et al.* (1989)] and ligated into the vector pBR322. Positive clones and subclones were transformed into D-43 and tested for anaerobic growth in Gly/DMSO and Gly/Fumarate broth medium.

The clone of *mttABC* was able to complement the D-43 mutation only when cloned into the lower copy number plasmid pBR322 (pBRmttABC) and no complementation (or growth) was observed when *mttABC* was cloned into the high copy number plasmid pTZ 18R (pTZmttABC).

The D-43 mutant could not be complemented with plasmid pDMS160 carrying the wild-type DmsABC operon suggesting that the mutation mapped outside the structural genes. Interestingly, the mutant expressed nearly normal levels of DMSO reductase activity but the activity was soluble rather than membrane-bound. This was surprising given that the membrane anchor, DmsC, was expressed in these cells (see below) and this suggested that the mutant was defective in membrane targeting or assembly.

### B. Sequencing the mttA region

We compared the sequence of clone pGS20 with the identical region of strain D-43 by PCR sequencing of both strands as follows. Chromosomal DNA from strains HB101 and D-43 was prepared as above. The 976 bp region which complements the D-43 mutation was amplified, the PCR products were sequenced directly and the DNA sequences of both strains were compared to the published sequence of *E. coli* [Blattner *et al*. (1997)]. As Taq DNA polymerase was used for PCR, two different reaction products, resulting from separately prepared templates, were sequenced to identify any mutations which may have resulted from the PCR reaction. Both strands were sequenced in the region of any identified mutations.

We identified only one nucleotide change altering a C to a T at position 743 of pGS20. When this region was compared to the sequence of contig AE00459 in the *E. coli* genome sequence [Blattner et al. (1997) Science 277:1453-1462], it appeared that the mutation mapped within the proposed ORF termed b3837. This ORF did not have a normal *E. coli* codon usage and so we determined the DNA sequence of this region of AE00459. Several differences were identified and a revised ORF map of this contig is shown in Figure 5. This revision resulted in several changes: ORF b3836, b3837 and b3838 are no longer observed and are replaced by a polypeptide which is very similar throughout its length to the YigT protein of *H.influenzae* [Fleischmann et al. (1995) Science 269:496-512] (Figure 6).

Figure 6 shows the sequence (SEQ ID NO:1) of *E. coli* wild-type MttA aligned with YigT of *Haemophilus influenzae* (Fleischmann *et al*., 1995) (SEQ ID NO:2). The two potential transmembrane segments are denoted as TMS1 and TMS2, respectively. a) denotes the position of the mutation in MttA which changes proline 25 to leucine. b) denotes the termination of MttA in clone pGS20. The potential α-helical region is indicated.

The mutation in D-43 resulted in the mutation of proline 25 of MttA2 to leucine. Interestingly, clone pGS20 did not encode the entire MttA polypeptide but terminated at amino acid 205. The MttA protein is composed of 277 amino acids and has a mass of -30.6 kDa. Without limiting the invention to any particular mechanism, the MttA protein has two potential transmembrane helices between residues 15-34 and 107-126. The most likely orientation is with the amino and carboxyl termini exposed to the periplasm. Residues 150 to 200 are predicted to form a very long α-helix. The mutation in D-43 altered the proline immediately after the second transmembrane helix and could disrupt this structure of the protein.

### C. Proteins homologous to the MttA protein

A database search of sequences which are related to *mttA* (*i.e*., *mttA1* and *mttA2*) identified a large family of related proteins whose function was previously unknown. In addition to the *Zea mays* protein of Settles et al. (1997) Science 278:1467-1470. related sequences were identified by BLAST searches in *Azotobacter chroococcum, Bacillus subtilis, Heamophilus influenzae, Helicobacter pylori, Mycobacterium leprae, Mycobacterium tuberculosis, Pseudomonas stutzerii, Rhodococcus erythropolis, and Synechocystis PCC6803* as well as the Ybec sequence of *E. coli* (Figure 8).

### EXAMPLE 7

### E. coli mttB And mttC Form An Operon With mttA

### A. The mttABC operon

Examination of the DNA sequence adjacent to *mttA* suggested that the upstream gene, *yigR*, encodes an aminoglycosyl transferase (BLAST search of the non-redundant data base). A potential transcription terminator at position 5590-5610 of contig AE00459 [Blattner et al. (1997) Science 277:1453-1462] separates *yigR* from *mttA.*

To test whether the adjacent genes *mttB* and *mttC* form an operon with *mttA,* mRNA was isolated from aerobically grown HB101 and RT-PCR was used with a primer within *mttC* to make a cDNA product. This cDNA was then amplified by PCR with primers within *mttA* and *mttB* giving the expected product of 270 bp., and *mttA* and *mttC* giving a product of 1091 bp. confirming a single polycistronic mRNA for the *mttA, mttB*, and *mttC* genes. To ensure that the PCR products were not the result of contaminating chromosomal DNA, the mRNA preparation was extensively digested with DNase prior to PCR and a control omitting the RT-PCR step did not give any products after PCR amplification.

The nucleotide sequence (SEQ ID NO:45) of the *mttABC* operon is shown in Figure 11. Figure 7 also shows the nucleotide sequence of the three open reading frames, ORF RF[3], ORF RF[2] and ORF RF[1], and the encoded amino acid sequences of MttA (SEQ ID NO:1), MttB (SEQ ID NO:7) and MttC (SEQ ID NO:8), respectively.

### B. Proteins homologous to the MttB and MttC proteins

A database search of sequences which are related to *mttB* and *mttC* identified a large family of related proteins which are organized contiguously in several organisms. In all cases the function of these proteins was previously unknown.

The nucleotide sequence of *mttB* (SEQ ID NO:)5 is shown in Figure 7. *mttB* encodes an integral membrane protein of 258 amino acids with six predicted transmembrane segments. A large number of related sequences was identified in a BLAST search extending from the archaebacteria (*Archeoglobus fulgidus*), through the eubacteria (*Azotobacter chroococcum, Bacillus subtilis, Heamophilus influenzae, Helicobacter pylori, Mycobacterium laprae, Mycobacterium tuberculosis)*, cyanobacteria (*Synechocystis PCC6803)* to mitochondria of algae (*Reclimonas americana, Chondrus crispus*) and plants (*Arabidopsis thalania, Marchantia polymorpha*) as well as chloroplasts of *Porphyra purpurea* and *Odentella sinensis* (Figure 9).

The nucleotide sequence of the neighboring gene *mttC* (SEQ ID NO:6) is shown in Figure 7. *mttC* encodes a polypeptide of 264 amino acids which is predicted to have at least one potential transmembrane segment (residues 24-41). The most likely orientation of this protein results in a large cytoplasmic domain extending from residue 41 to 264. Without limiting the invention to any particular mechanism, there is the possibility of a second transmembrane domain at residues 165-182. This possibility may be confirmed by a *blaM* gene fusion analysis. Like MttA and MttB, the MttC protein also is a member of a very large family of homologous proteins which includes two homologous sequences in *E. coli* (Ycfh and Yjjv) as well as homologous sequences in archaebacteria (*Methanobacterium thermoautotrophicum*), Mycoplasma (*Mycoplasma pneumoniae and Mycoplasma gentitaluium*), eubacteria (*Bacillus subtillis, Heamophilus influenzae*, *Helicobacter pylori, Mycobacterium tuberculosis*), cyanobacteria (*Synechocytis PCC6803*), yeast (*Schizosaccharomyces pombe and Saccharomyces cerevisae*), *C. elegans* and humans (Figure 10). The human protein is notable in having a 440 amino acid extension at the amino terminus which is not found in the other proteins. This extension is not related to MttA or MttB.

### EXAMPLE 8

### Construction of host cells containing a deletion of at least a portion of the genes mttA, mttB and mttC

The function of MttA, MttB and MttC proteins in a host cell is demonstrated by *in vivo* homologous recombination of chromosomal *mttA, mttB* and *mttC* as previously described [Sambasivarao et al (1991) J. Bacteriol. 5935-5943; Jasin et al (1984) J. Bacteriol. 159:783-786]. Briefly, the *mttABC* operon is cloned into vectors, and the gene whose function is to be determined (*i.e., mttA, mttB* or *mttC*) is mutated, *e.g.,* by insertion of a nucleotide sequence within the coding region of the gene. The plasmids are then homologously recombined with chromosomal *mttA, mttB* or *mttC* sequences in order to replace the chromosomal *mttA, mttB* or *mttC* genes with the mutated genes of the vectors. The effect of the mutations on the localization of proteins containing twin-arginine amino acid signal sequences is compared between the wild-type host cells and the cells containing the mutated *mttA, mttB* or *mttC* genes. These steps are further described as follows.

### A. Construction of plasmids carrying deletions or insertions in mttA, mttB and mttC genes

The *mttABC* operon (Figure 11) is cloned into pTZ18R and pBR322 vectors. In pBR322, the HindIII site in *mttB* is unique. The pBR322 containing *mttB* is then modified by insertion of a kanamycin gene cartridge at this unique site, while the unique NruI fragment contained in *mttC* is replaced by a kanamycin cartridge.

### B. Homologous recombination and P1 transduction

The modified plasmids are homologously recombined with chromosomal *mttA, mttB* and *mttC* in *E. coli* cells which contain either a *recBC* mutation or a *recD* mutation. The resulting recombinant is transferred by P1 transduction to suitable genetic backgrounds for investigation of the localization of protein expression. The localization (*e.g.,* cytoplasm, periplasm, cell membranes, extracellular medium) of expression of twin arginine containing proteins is compared using methods disclosed herein (*e.g.,* functional enzyme activity and Western blotting) between homologously recombined cells and control cells which had not been homologously recombined. Localization of expressed twin arginine containing proteins extracellularly, in the periplasm, or in the cytoplasm of homologously recombined cells as compared to localization of expression in cell membranes of control cells demonstrates that the wild-type MttA, MttB or MttC protein whose function had been modified by homologous recombination functions in targeting expression of the twin arginine containing protein to the cell membrane. Similarly, accumulation of expressed twin arginine containing proteins in extracellular medium, in the cytoplasm, or in cell membranes of homologously recombined cells as compared to periplasmic localization of the expressed twin arginine containing protein in control cells which had not been homologously recombined indicates that the protein (*i*.*e*., MttA, MttB or MttC) whose function had been modified by homologous recombination functions in translocation of the twin arginine containing protein to the periplasm.

### EXAMPLE 9

### Wild-type and mutant twin-arginine amino acid signal sequences of preDmsA are cleaved to release mature DmsA

In this Example, the following numbering system for DmsA has been used: the mature protein starts at Val 46; the leader extends from Metl to Ala 45 and the double Arg signal is at residues 15-21. In order to determine whether preproteins which contain twin-arginine amino acid signal sequences are cleaved to release a mature polypeptide as suggested by Berks [Berks (1996)], the two alanine amino acids at the -1 and -3 positions of the twin-arginine amino acid signal sequences of wild-type DmsA preprotein were replaced with asparagine, and cleavage of both the wild-type and the mutated twin-arginine amino acid signal sequences was investigated.

### A. Cell culture conditions

Cells were grown anaerobically in Luria Broth [Sambrook (1989)] and these cultures were used for a 1% inoculum into glycerol minimal medium with 0.167% peptone and vitamin B1, proline, leucine at final concentrations of 0.005%.

All manipulations of plasmids and strains were carried out as described by Sambrook *et al.* (1989)].

The upstream untranslated region of DmsA was examined using software from the Center for Biological Analysis (http://www.cbs.dtu.dk/) to identify potential leader peptidase I cleavage sites. This analysis indicated that mutation of both Ala43 and Ala45 was needed to inhibit cleavage. An additional secondary cleavage site with low probability was identified between Thr36 and Leu37. The two Ala mutated in this study were Ala43 and Ala45 which are underlined in the following DmsA leader sequence (SEQ ID NO:43) that contains the twin-arginine amino acid signal sequence: Mutants were generated by site-directed mutagenesis of single stranded DNA of plasmid pDMS223 [Rothery and Weiner (1991) Biochemistry 30:8296-8305] using the Sculptor kit (Amersham) and mutagenic primers to generate the mutants A43N and A43N,A45N. The mutagenic primer (SEQ ID NO:44) 5'-TTAGTCGGATTAATCACAATGTCGATAGCG-3' was used. Mutant DNA was subcloned into pDMS160 [Rothery and Weiner (1991)] using BgIII and EcoRI restriction sites, and resequenced to confirm the mutation.

### B. Expression studies

Samples were removed from the cultures after 30-48 hours of anaerobic growth, the cells pelleted by centrifugation at 9500g for 10 min., resuspended and everted envelopes prepared by French Press lysis. The cytoplasm and membrane fractions were separated by differential centrifugation. Membranes were washed twice with 50mM MOPS pH7.0 prior to use. Membrane proteins were solubilized with 1% SDS and polyacrylamide gel electrophoresis was performed using the Bio-Rad minigel system with a discontinuous SDS buffer system [Laemmli (1970) Nature 227:680-685]. Western blotting was performed using affinity purified DmsA antibody with the ECL Western blotting detection reagents from Amersham Life Science.

The results (data not shown) demonstrated cleavage of both the preDmsA proteins which contained alanine and which contained asparagine in the twin-arginine amino acid signal sequence to release mature DmsA. These results suggest that twin-arginine amino acid signal sequences are cleaved by signal peptidase I which also cleaves Sec signal sequences. Alternatively, a signal peptidase which is different from signal peptidase I and signal peptidase II, and which has different specificity may be operative. This possibility is investigated by N-terminal amino acid sequencing.

### C. N-terminal amino acid sequencing

N-terminal amino acid sequencing is carried out as previously described [Bilous et al (1988) Molec. Microbiol. 2:785-795] in order to determine the cleavage site in preDmsA and other preproteins which contain twin-arginine amino acid signal sequences, *e*.*g*., preTorA, and preNapA. A signal peptidase I temperature sensitive mutant is used to determine if preDmsA. preTorA and preNapA are cleaved at the restrictive temperature. Amino terminal sequences are determined by automated Edman degradation on an Applied Biosystems Model 470A gas phase sequenator. Subunits are separated by SDS PAGE and electroblotted onto polyvinylidene fluoride membranes and electroeluted as described by Cole et al. [J. Bacteriol. 170:2448-2456 (1988)].

The above-presented data shows that mttA1, MttA2, mttB and mttC encode proteins MttA1, MttA2, MttB and MttC which are essential in a Sec-independent pathway, and which function in targeting twin arginine containing proteins to cell membranes and in translocating twin arginine containing proteins to the periplasm and extracellular medium. The above-disclosed data further demonstrates that disruption of the function of any one or more of MttA1, MttA2, MttB and MttC results in translocation of twin arginine containing proteins to the periplasm, to extracellular medium, or to cellular compartments other than those compartments in which the twin arginine containing proteins are translocated in cells containing wild-type MttA1, MttA2, MttB and MttC. These results demonstrate that mttA1, MttA2, MttB and mttC are useful in translocating twin arginine containing proteins to the periplasm and extracellular medium. Such translocation is particularly useful in generating soluble proteins in a functional form, thus facilitating purification of such proteins and increasing their recovery.

## Claims

1. A method for expressing a nucleotide sequence of interest encoding an amino acid sequence of interest in a host cell, comprising:
a) providing:
i) said host cell;
ii) said nucleotide sequence of interest;
iii) a first nucleic acid sequence encoding twin-arginine signal amino acid sequence; and
iv) a second nucleic acid sequence encoding at least a portion of an amino acid sequence of (SEQ ID NO: 47, and at least a portion of amino acid sequence of SEQ ID NO: 7, wherein said at least a portion of the amino acid sequence encoded by said second nucleic acid has a biological activity comprising targeting of a fully-folded protein which contains a twin-arginine signal amino acid sequence to a cell membrane and translocating the fully-folded protein which contains twin-arginine signal amino acid sequence to a periplasm, and/or translocating the fully-folded protein which contains a twin-arginine signal amino acid sequence to an extracellular space;
b) operable fusing said nucleotide sequence of interest to said first nucleic acid sequence encoding a twin-arginine signal amino acid sequence to produce a fused polynucleotide sequence; and
c) introducing said fused polynucleotide sequence and said second nucleic acid sequence into said host cell under conditions such that said at least a portion of an amino acid sequence of SEQ ID NO: 47, and at least a portion of an amino acid sequence of SEQ ID NO: 7 are expressed, and said fused polynucleotide sequence is expressed to produce a fused polypeptide sequence comprising said twin-arginine signal amino acid sequence and said amino acid sequence of interest.

2. The method of claim 1, wherein said expressed amino acid sequence of interest is contained in periplasm of said host cell.

3. The method of claim 1, wherein said expressed amino acid sequence of interest is soluble.

4. The method of claim 1, wherein said host cell is cultured in medium, and wherein said expressed amino acid sequence of interest is contained in said medium.

5. The method of claim 4, wherein said expressed amino acid sequence of interest is soluble.

6. The method of claim 1, wherein said second nucleic acid sequence encodes an amino acid sequence comprising SEQ ID NO:47 and SEQ ID NO:7.

7. A method for expressing a nucleotide sequence of interest in a host cell to produce a soluble polypeptide sequence, said nucleotide sequence of interest when expressed in the absence of an operably linked nucleic acid sequence encoding a twin-arginine signal amino acid sequence produces an insoluble polypeptide, comprising:
a) providing:
i) said nucleotide sequence of interest encoding said insoluble polypeptide, wherein said insoluble polypeptide comprises a cofactor;
ii) said nucleic acid sequence encoding said twin-arginine signal amino acid sequence; and
iii) said host cell, wherein said host cell comprises at least a portion of an amino acid sequence of SEQ ID NO:47 and at least a portion of an amino acid sequence of SEQ ID NO:7, wherein both said at least a portion of said amino acid sequences together have a biological activity comprising targeting of a fully-folded protein which contains a twin-arginine signal amino acid sequence to a cell membrane and translocating the fully-folded protein which contains twin-arginine signal amino acid sequence to a periplasm, and/or translocating the fully-folded protein which contains a twin-arginine signal amino acid sequence to an extracellular space;
b) operably linking said nucleotide sequence of interest to said nucleic acid sequence encoding said twin-arginine signal amino acid sequence to produce a linked polynucleotide sequence; and
c) introducing said linked polynucleotide sequence into said host cell under conditions such that said fused polynucleotide sequence is expressed and said soluble polypeptide is produced.

8. The method of claim 7, wherein said insoluble polypeptide is included in an inclusion body.

9. The method of claim 7, wherein said cofactor is selected from the group consisting of iron-sulfur clusters, molybdopterin, polynuclear copper, tryptophan, tryptophylquinone, and flavin adenine dinucleotide.

10. The method of claim 7, wherein said soluble polypeptide is in the periplasm of said host cell.

11. The method of claim 7, wherein said host cell is cultured in medium, and wherein said soluble polypeptide is contained in said medium.

12. The method of claim 7, wherein said cell is *Escherichia coli*.

13. The method of claim 7, wherein said twin-arginine signal amino acid sequence is selected from the group consisting of SEQ ID NO:41 and SEQ ID NO:42.

## Patentansprüche

1. Methode zur Expression einer Nukleotidsequenz von Interesse, die eine Aminosäurensequenz von Interesse kodiert, in eine Wirtszelle, wobei die Methode Folgendes umfasst:
a) Bereitstellung:
i) der besagten Wirtszelle;
ii) der besagten Nukleotidsequenz von Interesse;
iii) einer ersten Nukleinsäurensequenz, die eine Twin-Arginin-Signalsäurensequenz kodiert; und
iv) einer zweiten Nukleinsäurensequenz, die mindestens einen Anteil einer Aminosäurensequenz der SEQ ID-NR: 47 und mindestens einen Anteil einer Aminosäurensequenz der SEQ ID-Nr: 7 kodiert, **dadurch gekennzeichnet, dass** der besagte mindestens eine Anteil der Aminosäurensequenz, der von der besagten zweiten Nukleinsäure kodiert wird, eine biologische Aktivität aufweist, welche die Zielsteuerung (Targeting) eines vollständig gefalteten Proteins umfasst, das eine Twin-Arginin-Signalaminosäurensequenz enthält, auf eine Zellmembran und Transport des vollständig gefalteten Proteins, welches die Twin-Arginin-Signalaminosäurensequenz enthält, an einen periplasmischen Raum und/oder Transport des vollständig gefalteten Proteins, welches eine Twin-Arginin-Signalaminosäurensequenz enthält, an einen extrazellulären Raum;
b) funktionelle Fusion der besagten Nukleotidsequenz von Interesse an die besagte erste Nukleinsäurensequeriz, die eine Twin-Arginin-Signalaminosäurensequenz kodiert, um eine fusionierte Polynukleotidsequenz zu produzieren; und
c) Einführung der besagten fusionierten Polynukleotidsequenz und der besagten zweiten Nukleinsäurensequenz in die besagte Wirtszelle unter Bedingungen, bei denen der besagte mindestens eine Teil einer Aminosäurensequenz der SEQ ID-NR: 47 und mindestens eine Teil einer Aminosäurensequenz der SEQ ID-Nr: 7 exprimiert werden und die besagte fusionierte Polynukleotidsequenz exprimiert wird, um eine fusionierte Polypeptidsequenz zu produzieren, welche die besagte Twin-Arginin-Signalaminosäurensequenz und die besagte Aminosäurensequenz von Interesse umfasst.

2. Methode nach Anspruch 1, **dadurch gekennzeichnet, dass** die exprimierte Aminosäurensequenz von Interesse im periplasmischen Raum der besagten Wirtszelle enthalten ist.

3. Methode nach Anspruch 1, **dadurch gekennzeichnet, dass** die exprimierte Aminosäurensequenz von Interesse löslich ist.

4. Methode nach Anspruch 1, **dadurch gekennzeichnet, dass** die besagte Wirtszelle in Medium kultiviert wird und die besagte Aminosäurensequenz von Interesse in dem besagten Medium enthalten ist.

5. Methode nach Anspruch 4, **dadurch gekennzeichnet, dass** die exprimierte Aminosäurensequenz von Interesse löslich ist.

6. Methode nach Anspruch 1, **dadurch gekennzeichnet, dass** die besagte zweite Nukleinsäurensequenz eine Aminosäurensequenz kodiert, welche die SEQ ID-NR:47 und SEQ ID-NR:7 umfasst.

7. Methode zur Expression einer Nukleotidsequenz von Interesse in eine Wirtszelle, um eine lösliche Polypeptidsequenz zu produzieren, und die besagte Nukleotidsequenz von Interesse bei einer Expression in Abwesenheit einer funktionell verknüpften Nukleinsäurensequenz, die eine Twin-Arginin-Signalaminosäurensequenz kodiert, ein unlösliches Polypeptid produziert, und wobei die Methode Folgendes umfasst:
a) Bereitstellung:
i) der besagten Nukleotidsequenz von Interesse, die das besagte unlösliche Polypeptid kodiert, **dadurch gekennzeichnet, dass** das besagte unlösliche Polypeptid einen Cofaktor umfasst;
ii) der besagten Nukleinsäurensequenz, die eine Twin-Arginin-Signalsäurensequenz kodiert; und
iii) der besagten Wirtszelle, **dadurch gekennzeichnet, dass** die besagte Wirtszelle
mindestens einen Anteil einer Aminosäurensequenz der SEQ ID-NR: 47 und mindestens einen Anteil einer Aminosäurensequenz der SEQ ID-NR: 7 umfasst, **dadurch gekennzeichnet, dass** beide besagten mindestens eine Anteile der besagten Aminosäurensequenzen zusammen eine biologische Aktivität aufweisen, welche die Zielsteuerung (Targeting) eines vollständig gefalteten Proteins umfasst, das eine Twin-Arginin-Signalaminosäurensequenz enthält, auf eine Zellmembran und Transport des vollständig gefalteten Proteins, welches die Twin-Arginin-Signalaminosäurensequenz enthält, an einen periplasmischen Raum und/oder Transport des vollständig gefalteten Proteins, welches eine Twin-Arginin-Signalaminosäurensequenz enthält, an einen extrazellulären Raum;
b) funktionelle Verknüpfung der besagten Nukleotidsequenz von Interesse mit der besagten ersten Nukleinsäurensequenz, die eine Twin-Arginin-Signalaminosäurensequenz kodiert, um eine verknüpfte Polynukleotidsequenz zu produzieren; und
c) Einführung der besagten verknüpften Polynukleotidsequenz in die besagte Wirtszelle unter Bedingungen, unter denen die besagte fusionierte Polynukleotidsequenz exprimiert und das besagte lösliche Polypeptid produziert wird.

8. Methode nach Anspruch 7, **dadurch gekennzeichnet, dass** das besagte unlösliche Polypeptid in einem Inklusionskörper enthalten ist.

9. Methode nach Anspruch 7, **dadurch gekennzeichnet, dass** der besagte Cofaktor aus einer Gruppe ausgewählt wird, die Eisen-Schwefel-Cluster, Molybdopterin, polynukleares Kupfer, Tryptophan, Tryptophylquinon und Flavinadenindinukleotid umfasst.

10. Methode nach Anspruch 7, **dadurch gekennzeichnet, dass** sich das besagte lösliche Polypeptid im periplasmischen Raum der besagten Wirtszelle befindet.

11. Methode nach Anspruch 7, **dadurch gekennzeichnet, dass** die besagte Wirtszelle in Medium kultiviert wird und das besagte lösliche Polypeptid in dem besagten Medium enthalten ist.

12. Methode nach Anspruch 7, **dadurch gekennzeichnet, dass** es sich bei der besagten Zelle um *Escherichia coli* handelt.

13. Methode nach Anspruch 7, **dadurch gekennzeichnet, dass** die besagte Twin-Arginin-Signalaminosäurensequenz aus einer Gruppe ausgewählt wird, welche die SEQ ID-NR: 41 und SEQ ID-NR: 42 umfasst.

## Revendications

1. Procédé pour l'expression d'une séquence nucléotidique d'intérêt codant pour une séquence d'acides aminés d'intérêt dans une cellule hôte, comprenant les étapes consistant à :
a) fournir :
i) ladite cellule hôte ;
ii) ladite séquence nucléotidique d'intérêt ;
iii) une première séquence d'acides nucléiques codant pour une séquence d'acides aminés signal de twin-arginine ;
et
iv) une deuxième séquence d'acides nucléiques codant pour au moins une portion d'une séquence d'acides aminés de SEQ ID NO:47 et au moins une portion de séquence d'acides aminés de SEQ ID NO:7, en ce qu'au moins une portion de la séquence d'acides aminés codée par ledit deuxième acide nucléique présente une activité biologique comprenant le ciblage d'une protéine complètement repliée qui contient une séquence d'acides aminés signal de twin-arginine vers une membrane de cellule et la translocation de la protéine complètement repliée qui contient une séquence d'acides aminés signal de twin-arginine vers un périplasme et/ou la translocation de la protéine complètement repliée qui contient une séquence d'acides aminés signal de twin-arginine vers un espace extracellulaire ;
b) fusionner fonctionnellement ladite séquence nucléotidique d'intérêt à ladite première séquence d'acides nucléiques codant pour une séquence d'acides aminés signal de twin-arginine afin de produire une séquence polynucléotidique fusionnée ; et
c) introduire ladite séquence polynucléotidique fusionnée et ladite deuxième séquence d'acides nucléiques dans ladite cellule hôte dans des conditions telles que, au moins une portion d'une séquence d'acides aminés de SEQ ID NO:47 et au moins une portion d'une séquence d'acides aminés de SEQ ID NO:7 sont exprimées et ladite séquence polynucléotidique fusionnée est exprimée pour produire une séquence polypeptidique fusionnée comprenant ladite séquence d'acides aminés signal de twin-arginine et ladite séquence d'acides aminés d'intérêt.

2. Procédé selon la revendication 1, **caractérisé en ce que** ladite séquence d'acides aminés d'intérêt exprimée est contenue dans le périplasme de ladite cellule hôte.

3. Procédé selon la revendication 1, **caractérisé en ce que** ladite séquence d'acides aminés d'intérêt exprimée est soluble.

4. Procédé selon la revendication 1, **caractérisé en ce que** ladite cellule hôte est cultivée dans un milieu et **en ce que** ladite séquence d'acides aminés d'intérêt exprimée est contenue dans ledit milieu.

5. Procédé selon la revendication 4, **caractérisé en ce que** ladite séquence d'acides aminés d'intérêt exprimée est soluble.

6. Procédé selon la revendication 1, **caractérisé en ce que** ladite deuxième séquence d'acides nucléiques code pour une séquence d'acides aminés comprenant SEQ ID NO:47 et SEQ ID NO:7.

7. Procédé pour l'expression d'une séquence nucléotidique d'intérêt dans une cellule hôte pour produire une séquence polypeptidique soluble, ladite séquence nucléotidique d'intérêt, lorsqu'elle est exprimée en l'absence d'une séquence d'acide nucléique fonctionnellement liée codant pour une séquence d'acides aminés signal de twin-arginine, produisant un polypeptide insoluble, comprenant les étapes consistant à :
a) fournir :
i) ladite séquence nucléotidique d'intérêt codant pour ledit polypeptide insoluble, ledit polypeptide insoluble comprenant un cofacteur ;
ii) ladite séquence d'acide nucléique codant pour ladite séquence d'acides aminés signal de twin-arginine ; et
iii) ladite cellule hôte, celle-ci comprenant au moins une portion d'une séquence d'acides aminés de SEQ ID NO:47 et au moins une portion d'une séquence d'acides aminés de SEQ ID NO:7, lesdites deux portions desdites séquences d'acides aminés ayant ensemble une activité biologique comprenant le ciblage d'une protéine complètement repliée qui contient une séquence d'acides aminés signal de twin-arginine vers une membrane de cellule et la translocation de la protéine complètement repliée qui contient une séquence d'acides aminés signal de twin-arginine vers un périplasme et/ou la translocation de la protéine complètement repliée qui contient une séquence d'acides aminés signal de twin-arginine vers un espace extracellulaire ;
b) lier fonctionnellement ladite séquence nucléotidique d'intérêt à ladite séquence d'acides nucléiques codant pour ladite séquence d'acides aminés signal de twin-arginine pour produire une séquence polynucléotidique liée ; et
c) introduire ladite séquence polynucléotidique liée dans ladite cellule hôte dans des conditions telles que ladite séquence polynucléotidique fusionnée est exprimée et ledit polypeptide soluble est produit.

8. Procédé selon la revendication 7, **caractérisé en ce que** ledit polypeptide insoluble est inclus dans un corps d'inclusion.

9. Procédé selon la revendication 7, **caractérisé en ce que** ledit cofacteur est sélectionné dans le groupe constitué d'agrégats fer-soufre, de molybdoptérine, de cuivre polynucléaire, de tryptophane, de tryptophylquinone et de flavine adénine dinucléotide.

10. Procédé selon la revendication 7, **caractérisé en ce que** ledit polypeptide soluble est dans le périplasme de ladite cellule hôte.

11. Procédé selon la revendication 7, **caractérisé en ce que** ladite cellule hôte est cultivée dans un milieu et **en ce que** ledit polypeptide soluble est contenu dans ledit milieu.

12. Procédé selon la revendication 7, **caractérisé en ce que** ladite cellule est *Escherichia coli*.

13. Procédé selon la revendication 7, **caractérisé en ce que** ladite séquence d'acides aminés signaux de twin-arginine est sélectionnée dans le groupe constitué de SEQ ID NO:41 et SEQ ID NO:42.


### SEQUENCE LISTING

<110> Weiner, Joel H.
   Turner, Raymond J.
<120> Compositions and Methods for Protein Secretion
<130> UALB-03697
<140> PCT/CA99/00272
   <141> 1999-03-29
<150> 09/053,197
   <151> 1998-04-01
<150> 09/085,761
   <151> 1998-05-28
<160> 49
<170> PatentIn Ver. 2.0
<210> 1
   <211> 277
   <212> PRT
   <213> Escherichia coli
<400> 1
   Met Arg Leu Cys Leu Ile Ile Ile Tyr His Arg Gly Thr Cys Met Gly
     1               5                  10                  15
   Gly Ile Ser Ile Trp Gln Leu Leu Ile Ile Ala Val Ile Val Val Leu
                20                  25                  30
   Leu Phe Gly Thr Lys Lys Leu Gly Ser Ile Gly Ser Asp Leu Gly Ala
            35                  40                  45
   Ser Ile Lys Gly Phe Lys Lys Ala Met Ser Asp Asp Glu Pro Lys Gln
        50                  55                  60
   Asp Lys Thr Ser Gln Asp Ala Asp Phe Thr Ala Lys Thr Ile Ala Asp
    65                  70                  75                  80
   Lys Gln Ala Asp Thr Asn Gln Glu Gln Ala Lys Thr Glu Asp Ala Lys
                    85                  90                  95
   Arg His Asp Lys Glu Gln Gly Val Asn Pro Cys Leu Ile Ser Val Leu
               100                 105                 110
   Ala Asn Leu Leu Leu Val Phe Ile Ile Gly Leu Val Val Leu Gly Pro
           115                 120                 125
   Gln Arg Leu Pro Val Ala Val Lys Thr Val Ala Gly Trp Ile Arg Ala
       130                 135                 140
   Leu Arg Ser Leu Ala Thr Thr Val Gln Asn Glu Leu Thr Gln Glu Leu
   145 150 155 160
   Lys Leu Gln Glu Phe Gln Asp Ser Leu Lys Lys Val Glu Lys Ala Ser
   165 170 175
   Leu Thr Asn Leu Thr Pro Glu Leu Lys Ala Ser Met Asp Glu Leu Arg
   180 185 190
   Gln Ala Ala Glu Ser Met Lys Arg Ser Tyr Val Ala Asn Asp Pro Glu
   195 200 205
   Lys Ala Ser Asp Glu Ala His Thr Ile His Asn Pro Val Val Lys Asp
   210 215 220
   Asn Glu Ala Ala His Glu Gly Val Thr Pro Ala Ala Ala Gln Thr Gln
   225 230 235 240
   Ala Ser Ser Pro Glu Gln Lys Pro Glu Thr Thr Pro Glu Pro Val Val
   245 250 255
   Lys Pro Ala Ala Asp Ala Glu Pro Lys Thr Ala Ala Pro Ser Pro Ser
   260 265 270
   Ser Ser Asp Lys Pro
   275
<210> 2
   <211> 284
   <212> PRT
   <213> Haemophilus influenzae
<400> 2
   Met Ala Lys Lys Ser Ile Phe Arg Ala Lys Phe Phe Leu Phe Tyr Arg
   1 5 10 15
   Thr Glu Phe Ile Met Phe Gly Leu Ser Pro Ala Gln Leu Ile Ile Leu
   20 25 30
   Leu Val Val Ile Leu Leu Ile Phe Gly Thr Lys Lys Leu Arg Asn Ala
   35 40 45
   Gly Ser Asp Leu Gly Ala Ala Val Lys Gly Phe Lys Lys Ala Met Lys
   50 55 60
   Glu Asp Glu Lys Val Lys Asp Ala Glu Phe Lys Ser Ile Asp Asn Glu
   65 70 75 80
   Thr Ala Ser Ala Lys Lys Gly Lys Tyr Lys Arg Glu Arg Asn Arg Leu
   85 90 95
   Asn Pro Cys Leu Ile Leu Val Phe Gln Asn Leu Phe Tyr Xaa Met Val
   100 105 110
   Leu Gly Leu Val Val Leu Gly Pro Lys Arg Leu Pro Ile Ala Ile Arg
   115 120 125
   Thr Val Met Asp Trp Val Lys Thr Ile Arg Gly Leu Ala Ala Asn Val
   130 135 140
   Gln Asn Glu Leu Lys Gln Glu Leu Lys Leu Gln Glu Leu Gln Asp Ser
   145 150 155 160
   Ile Lys Lys Ala Glu Ser Leu Asn Leu Gln Ala Leu Ser Pro Glu Leu
   165 170 175
   Ser Lys Thr Val Glu Glu Leu Lys Ala Gln Ala Asp Lys Met Lys Ala
   180 185 190
   Glu Leu Glu Asp Lys Ala Ala Gln Ala Gly Thr Thr Val Glu Asp Gln
   195 200 205
   Ile Lys Glu Ile Lys Ser Ala Ala Glu Asn Ala Glu Lys Ser Gln Asn
   210 215 220
   Ala Ile Ser Val Glu Glu Ala Ala Glu Thr Leu Ser Glu Ala Glu Arg
   225 230 235 240
   Thr Pro Thr Asp Leu Thr Ala Leu Glu Thr His Glu Lys Val Glu Leu
   245 250 255
   Asn Thr His Leu Ser Ser Tyr Tyr Pro Pro Asp Asp Ile Glu Ile Ala
   260 265 270
   Pro Ala Ser Lys Ser Gln Ser Ser Lys Thr Lys Ser
   275 280
<210> 3
   <211> 22108
   <212> DNA
   <213> Escherichia coli
<400> 3
<210> 4
   <211> 831
   <212> ENA
   <213> Escherichia coli
<400> 4
   atgcggcttt gtttaatcat catctaccac agaggaacat gtatgggtgg tatcagtatt 60
   tggcagttat tgattattgc cgtcatcgtt gtactgcttt ttggcaccaa aaagctcggc 120
   tccatcggtt ccgatcttgg tgcgtcgatc aaaggcttta aaaaagcaat gagcgatgat 180
   gaaccaaagc aggataaaac cagtcaggat gctgatttta ctgcgaaaac tatcgccgat 240
   aagcaggcgg atacgaatca ggaacaggct aaaacagaag acgcgaagcg ccacgataaa 300
   gagcaggtga atccgtgttt gatatcggtt ttagcgaact tgctattggt gttcatcatc 360
   ggcctcgtcg ttctggggcc gcaacgactg cctgtggcgg taaaaacggt agcgggctgg 420
   attcgcgcgt tgcgttcact ggcgacaacg gtgcagaacg aactgaccca ggagttaaaa 480
   ctccaggagt ttcaggacag tctgaaaaag gttgaaaagg cgagcctcac taacctgacg 540
   cccgaactga aagcgtcgat ggatgaacta cgccaggccg cggagtcgat gaagcgttcc 600
   tacgttgcaa acgatcctga aaaggcgagc gatgaagcgc acaccatcca taacccggtg 660
   gtgaaagata atgaagctgc gcatgagggc gtaacgcctg ccgctgcaca aacgcaggcc 720
   agttcgccgg aacagaagcc agaaaccacg ccagagccgg tggtaaaacc tgctgcggac 780
   gctgaaccga aaaccgctgc accttcccct tcgtcgagtg ataaaccgta a 831
<210> 5
   <211> 778
   <212> DNA
   <213> Escherichia coli
<400> 5
   atgtctgtag aagatactca accgcttatc acgcatctga ttgagctgcg taagcgtctg 60
   ctgaactgca ttatcgcggt gatcgtgata ttcctgtgtc tggtctattt cgccaatgac 120
   atctatcacc tggtatccgc gccattgatc aagcagttgc cgcaaggttc aacgatgatc 180
   gccaccgacg tggcctcgcc gttctttacg ccgatcaagc tgacctttat ggtgtcgctg 240
   attctgtcag cgccggtgat tctctatcag gtgtgggcat ttatcgcccc agcgctgtat 300
   aagcatgaac gtcgcctggt ggtgccgctg ctggtttcca gctctctgct gttttatatc 360
   ggcatggcat tcgcctactt tgtggtcttt ccgctggcat ttggcttcct tgccaatacc 420
   gcgccggaag gggtgcaggt atccaccgac atcgccagct atttaagctt cgttatggcg 480
   ctgtttatgg cgtttggtgt ctcctttgaa gtgccggtag caattgtgct gctgtgctgg 540
   atggggatta cctcgccaga agacttacgc aaaaaacgcc cgtatgtgct ggttggtgca 600
   ttcgttgtcg ggatgttgct gacgccgccg gatgtcttct cgcaaacgct gttggcgatc 660
   ccgatgtact gtctgtttga aatcggtgtc ttcttctcac gcttttacgt tggtaaaggg 720
   cgaaatcggg aagaggaaaa cgacgctgaa gcagaaagcg aaaaaactga agaataaa 778
<210> 6
   <211> 795
   <212> DNA
   <213> Escherichia coli
<400> 6
   atggagtaca ggatgtttga tatcggcgtt aatttgacca gttcgcaatt tgcgaaagac 60
   cgtgatgatg ttgtagcgtg cgcttttgac gcgggagtta atgggctact catcaccggc 120
   actaacctgc gtgaaagcca gcaggcgcaa aagctggcgc gtcagtattc gtcctgttgg 180
   tcaacggcgg gcgtacatcc tcacgacagc agccagtggc aagctgcgac tgaagaagcg 240
   attattgagc tggccgcgca gccagaagtg gtggcgattg gtgaatgtgg tctcgacttt 300
   aaccgcaact tttcgacgcc ggaagagcag gaacgcgctt ttgttgccca gctacgcatt 360
   gccgcagatt taaacatgcc ggtatttatg cactgtcgcg atgcccacga gcggtttatg 420
   acattgctgg agccgtggct ggataaactg cctggtgcgg ttcttcattg ctttaccggc 480
   acacgcgaag agatgcaggc gtgcgtggcg catggaattt atatcggcat taccggttgg 540
   gtttgcgatg aacgacgcgg actggagctg cgggaacttt tgccgttgat tccggcggaa 600
   aaattactga tcgaaactga tgcgccgtat ctgctccctc gcgatctcac gccaaagcca 660
   tcatcccggc gcaacgagcc agcccatctg ccccatattt tgcaacgtat tgcgcactgg 720
   cgtggagaag atgccgcatg gctggctgcc accacggatg ctaatgtcaa aacactgttt 780
   gggattgcgt tttag 795
<210> 7
   <211> 258
   <212> PRT
   <213> Escherichia coli
<400> 7
   Met Ser Val Glu Asp Thr Gln Pro Leu Ile Thr His Leu Ile Glu Leu
    1               5                  10                  15
   Arg Lys Arg Leu Leu Asn Cys Ile Ile Ala Val Ile Val Ile Phe Leu
               20                  25                  30
   Cys Leu Val Tyr Phe Ala Asn Asp Ile Tyr His Leu Val Ser Ala Pro
           35                  40                  45
   Leu Ile Lys Gln Leu Pro Gln Gly Ser Thr Met Ile Xaa Xaa Asp Val
       50                  55                  60
   Ala Ser Pro Phe Phe Thr Pro Ile Lys Leu Thr Phe Met Val Ser Leu
   65                  70                  75                  80
   Ile Leu Ser Ala Pro Val Ile Leu Tyr Gln Val Trp Ala Phe Ile Ala
                   85                  90                  95
   Pro Ala Leu Tyr Lys His Glu Arg Arg Leu Val Val Pro Leu Leu Val
               100                 105                 110
   Ser Ser Ser Leu Leu Phe Leu Tyr Arg His Ala Phe Ala Tyr Phe Val
           115                 120                 125
   Val Phe Pro Leu Ala Phe Gly Phe Leu Ala Asn Thr Ala Pro Glu Gly
       130                 135                 140
   Val Gln Val Ser Thr Asp Ile Ala Ser Tyr Leu Ser Phe Val Met Ala
   145                 150                 155                 160
   Leu Phe Met Ala Phe Gly Val Ser Phe Glu Val Pro Val Ala Ile Val
                   165                 170                 175
   Leu Leu Cys Trp Met Gly Ile Thr Ser Pro Glu Asp Leu Arg Lys Lys
               180                 185                 190
   Arg Pro Tyr Val Leu Val Gly Ala Phe Val Val Gly Met Leu Leu Thr
           195                 200                 205
   Pro Pro Asp Val Phe Ser Gln Thr Leu Leu Ala Ile Pro Met Tyr Cys
       210                 215                 220
   Leu Phe Glu Ile Gly Val Phe Phe Ser Arg Phe Tyr Val Gly Lys Gly
   225                 230                 235                 240
   Arg Asn Arg Glu Glu Glu Asn Asp Ala Glu Ala Glu Ser Glu Lys Thr
                   245                 250                 255
   Glu Glu
<210> 8
   <211> 264
   <212> PRT
   <213> Escherichia coli
<400> 8
   Met Glu Tyr Arg Met Phe Asp Ile Gly Val Asn Leu Thr Ser Ser Gln
    1               5                  10                  15
   Phe Ala Lys Asp Arg Asp Asp Val Val Ala Cys Ala Phe Asp Ala Gly
               20                  25                  30
   Val Asn Gly Leu Leu Ile Thr Gly Thr Asn Leu Arg Glu Ser Gln Gln
           35                  40                  45
   Ala Gln Lys Leu Ala Arg Gln Tyr Ser Ser Cys Trp Ser Thr Ala Gly
       50                  55                  60
   Val His Pro His Asp Ser Ser Gln Trp Gln Ala Ala Thr Glu Glu Ala
   65                  70                  75                  80
   Ile Ile Glu Leu Ala Ala Gln Pro Glu Val Val Ala Ile Gly Glu Cys
                   85                  90                  95
   Gly Leu Asp Phe Asn Arg Asn Phe Ser Thr Pro Glu Glu Gln Glu Arg
               100                 105                 110
   Ala Phe Val Ala Gln Leu Arg Ile Ala Ala Asp Leu Asn Met Pro Val
           115                 120                 125
   Phe Met His Cys Arg Asp Ala His Glu Arg Phe Met Thr Leu Leu Glu
   130 135 140
   Pro Trp Leu Asp Lys Leu Pro Gly Ala Val Leu His Cys Phe Thr Gly
   145 150 155 160
   Thr Arg Glu Glu Met Gln Ala Cys Val Ala His Gly Ile Tyr Ile Gly
   165 170 175
   Ile Thr Gly Trp Val Cys Asp Glu Arg Arg Gly Leu Glu Leu Arg Glu
   180 185 190
   Leu Leu Pro Leu Ile Pro Ala Glu Lys Leu Leu Ile Glu Thr Asp Ala
   195 200 205
   Pro Tyr Leu Leu Pro Arg Asp Leu Thr Pro Lys Pro Ser Ser Arg Arg
   210 215 220
   Asn Glu Pro Ala His Leu Pro His Ile Leu Gln Arg Ile Ala His Trp
   225 230 235 240
   Arg Gly Glu Asp Ala Ala Trp Leu Ala Ala Thr Thr Asp Ala Asn Val
   245 250 255
   Lys Thr Leu Phe Gly Ile Ala Phe
   260
<210> 9
   <211> 243
   <212> PRT
   <213> Zea mays
<400> 9
   Met Thr Pro Thr Ala Asn Leu Leu Leu Pro Ala Pro Pro Phe Val Pro
   1               5                   10                  15
   Ile Ser Asp Val Arg Arg Leu Gln Leu Pro Pro Arg Val Arg His Gln
               20                  25                  30
   Pro Arg Pro Cys Trp Lys Gly Val Glu Trp Gly Ser Ile Gln Thr Arg
           35                  40                  45
   Met Val Ser Ser Phe Val Ala Val Gly Ser Arg Thr Arg Arg Arg Asn
       50                  55                  60
   Val Ile Cys Ala Ser Leu Phe Gly Val Gly Ala Pro Glu Ala Leu Val
   65                  70                  75                  80
   Ile Gly Val Val Ala Leu Leu Val Phe Gly Pro Lys Gly Leu Ala Glu
                   85                  90                  95
   Val Ala Arg Asn Leu Gly Lys Thr Leu Arg Ala Phe Gln Pro Thr Ile
               100                 105                 110
   Arg Glu Leu Gln Asp Val Ser Arg Glu Phe Arg Ser Thr Leu Glu Arg
           115                 120                 125
   Glu Ile Gly Ile Asp Glu Val Ser Gln Ser Thr Asn Tyr Arg Pro Thr
       130                 135                 140
   Thr Met Asn Asn Asn Gln Gln Pro Ala Ala Asp Pro Asn Val Lys Pro
   145                 150                 155                 160
   Glu Pro Ala Pro Tyr Thr Ser Glu Glu Leu Met Lys Val Thr Glu Glu
                   165                 170                 175
   Gln Ile Ala Ala Ser Ala Ala Ala Ala Trp Asn Pro Gln Gln Pro Ala
               180                 185                 190
   Thr Ser Gln Gln Gln Glu Glu Ala Pro Thr Thr Pro Arg Ser Glu Asp
           195                 200                 205
   Ala Pro Thr Ser Gly Gly Ser Asp Gly Pro Ala Ala Pro Ala Arg Ala
       210                 215                 220
   Val Ser Asp Ser Asp Pro Asn Gln Val Asn Lys Ser Gln Lys Ala Glu
   225                 230                 235                 240
   Gly Glu Arg
<210> 10
   <211> 67
   <212> PRT
   <213> Escherichia coli
<400> 10
   Met Gly Glu Ile Ser Ile Thr Lys Leu Leu Val Val Ala Ala Leu Val
    1               5                  10                  15
   Val Leu Leu Phe Gly Thr Lys Lys Leu Arg Thr Leu Gly Gly Asp Leu
               20                  25                  30
   Gly Ala Ala Ile Lys Gly Phe Lys Lys Ala Met Asn Asp Asp Asp Ala
           35                  40                  45
   Ala Ala Lys Lys Gly Ala Asp Val Asp Leu Gln Ala Glu Lys Leu Ser
       50                  55                  60
   His Lys Glu
   65
<210> 11
   <211> 126
   <212> PRT
   <213> Mycobacterium tuberculosis
<400> 11
   Met Ala Leu Thr Leu Val Met Gly Ala Ile Ala Ser Pro Trp Val Ser
    1               5                  10                  15
   Val Gly Thr Lys Leu Cys Tyr Ser Arg Leu Asn Glu Ser Phe Tyr Pro
               20                  25                  30
   Ser Asn Pro Leu Thr Ala Pro Asn Pro Met Asn Ile Phe Gly Ile Gly
           35                  40                  45
   Leu Pro Glu Leu Gly Leu Ile Phe Val Ile Ala Leu Leu Val Phe Gly
       50                  55                  60
   Pro Lys Lys Leu Pro Glu Val Gly Arg Ser Leu Gly Lys Ala Leu Arg
   65                  70                  75                  80
   Gly Phe Gln Glu Ala Ser Lys Glu Phe Glu Thr Glu Leu Lys Arg Glu
                   85                  90                  95
   Ala Gln Asn Leu Glu Lys Ser Val Gln Ile Lys Ala Glu Leu Glu Glu
               100                 105                 110
   Ser Lys Thr Pro Glu Ser Ser Ser Ser Ser Glu Lys Ala Ser
           115                 120                 125
<210> 12
   <211> 98
   <212> PRT
   <213> Rhodococcus erythropolis
<400> 12
   Met Gly Ala Met Ser Pro Trp His Trp Ala Ile Val Ala Leu Val Val
   1               5                   10                  15
   Val Ile Leu Phe Gly Ser Lys Lys Leu Pro Asp Ala Ala Arg Gly Leu
               20                  25                  30
   Gly Arg Ser Leu Arg Ile Phe Lys Ser Glu Val Lys Glu Met Gln Asn
           35                  40                  45
   Asp Asn Ser Thr Pro Ala Pro Thr Ala Gln Ser Ala Pro Pro Pro Gln
       50                  55                  60
   Ser Ala Pro Ala Glu Leu Pro Val Ala Asp Thr Thr Thr Ala Pro Val
   65                  70                  75                  80
   Thr Pro Pro Ala Pro Val Gln Pro Gln Ser Gln His Thr Glu Pro Lys
                   85                  90                  95
   Ser Ala
<210> 13
   <211> 58
   <212> PRT
   <213> Pseudomonas stutzeri
<400> 13
   Met Met Gly Ile Ser Val Trp Gln Leu Leu Ile Ile Leu Leu Ile Val
   1               5                   10                  15
   Val Met Leu Phe Gly Thr Lys Arg Leu Arg Gly Leu Gly Ser Asp Leu
               20                  25                  30
   Gly Ser Ala Ile Asn Gly Phe Arg Lys Ser Val Ser Asp Gly Glu Thr
           35                  40                  45
   Thr Thr Gln Ala Glu Ala Ser Ser Arg Ser
       50                  55
<210> 14
   <211> 88
   <212> PRT
   <213> Mycobacterium leprae
<400> 14
   Met Gly Ser Leu Ser Pro Trp His Trp Val Val Leu Val Val Val Val
   1 5 10 15
   Val Leu Leu Phe Gly Ala Lys Lys Leu Pro Asp Ala Ala Arg Ser Leu
   20 25 30
   Gly Lys Ser Met Arg Ile Phe Lys Ser Glu Leu Arg Glu Met Gln Thr
   35 40 45
   Glu Asn Gln Ala Gln Ala Ser Ala Leu Glu Thr Pro Met Gln Asn Pro
   50 55 60
   Thr Val Val Gln Ser Gln Arg Val Val Pro Pro Trp Ser Thr Glu Gln
   65 70 75 80
   Asp His Thr Glu Ala Arg Pro Ala
   85
<210> 15
   <211> 79
   <212> PRT
   <213> Helicobacter pylori
<400> 15
   Met Gly Gly Phe Thr Ser Ile Trp His Trp Val Ile Val Leu Leu Val
   1 5 10 15
   Ile Val Leu Leu Phe Gly Ala Lys Lys Ile Pro Glu Leu Ala Lys Gly
   20 25 30
   Leu Gly Ser Gly Ile Lys Asn Phe Lys Lys Ala Val Lys Asp Asp Glu
   35 40 45
   Glu Glu Ala Lys Asn Glu Pro Lys Thr Leu Asp Ala Gln Ala Thr Gln
   50 55 60
   Thr Lys Val His Glu Ser Ser Glu Ile Lys Ser Lys Gln Glu Ser
   65 70 75
<210> 16
   <211> 109
   <212> PRT
   <213> Haemophilus influenzae
<400> 16
   Met Ala Lys Lys Ser Ile Phe Arg Ala Lys Phe Phe Leu Phe Tyr Arg
   1 5 10 15
   Thr Glu Phe Ile Met Phe Gly Leu Ser Pro Ala Gln Leu Ile Ile Leu
   20 25 30
   Leu Val Val Ile Leu Leu Ile Phe Gly Thr Lys Lys Leu Arg Asn Ala
   35 40 45
   Gly Ser Asp Leu Gly Ala Ala Val Lys Gly Phe Lys Lys Ala Met Lys
   50 55 60
   Glu Asp Glu Lys Val Lys Asp Ala Glu Phe Lys Ser Ile Asp Asn Glu
   65 70 75 80
   Thr Ala Ser Ala Lys Lys Gly Lys Tyr Lys Arg Glu Arg Asn Arg Leu
   85 90 95
   Asn Pro Cys Leu Ile Leu Val Phe Gln Asn Leu Phe Tyr
   100 105
<210> 17
   <211> 57
   <212> PRT
   <213> Bacillus subtilis
<400> 17
   Met Pro Ile Gly Pro Gly Ser Leu Ala Val Ile Ala Ile Val Ala Leu
   1 5 10 15
   Ile Ile Phe Gly Pro Lys Lys Leu Pro Glu Leu Gly Lys Ala Ala Gly
   20 25 30
   Asp Thr Leu Arg Glu Phe Lys Asn Ala Thr Lys Gly Leu Thr Ser Asp
   35 40 45
   Glu Glu Glu Lys Lys Lys Glu Asp Gln
   50 55
<210> 18
   <211> 192
   <212> PRT
   <213> Azotobacter chroococcum
<400> 18
   Met Gly Phe Gly Gly Ile Ser Ile Trp Gln Leu Leu Ile Ile Leu Leu
   1               5                   10                  15
   Ile Val Val Met Leu Phe Gly Thr Lys Arg Leu Lys Ser Leu Gly Ser
               20                  25                  30
   Asp Leu Gly Asp Ala Ile Lys Gly Phe Arg Lys Ser Met Asp Asn Glu
           35                  40                  45
   Glu Asn Lys Ala Pro Pro Val Glu Glu Gln Lys Gly Gln Asp His Arg
       50                  55                  60
   Gly Pro Gly Pro Gln Gly Arg Gly Thr Gly Gln Glu Arg Leu Ser Met
   65                  70                  75                  80
   Phe Asp Ile Gly Phe Ser Glu Leu Leu Leu Val Gly Leu Val Ala Leu
                   85                  90                  95
   Leu Val Leu Gly Pro Glu Arg Leu Pro Val Ala Ala Arg Met Ala Gly
               100                 105                 110
   Leu Trp Ile Gly Arg Leu Lys Arg Ser Phe Asn Thr Leu Lys Thr Glu
           115                 120                 125
   Val Glu Arg Glu Ile Gly Ala Asp Glu Ile Arg Arg Gln Leu His Asn
       130                 135                 140
   Glu Arg Ile Leu Glu Leu Glu Arg Glu Met Lys Gln Ser Leu Gln Pro
   145                 150                 155                 160
   Pro Ala Pro Ser Ala Pro Asp Glu Thr Ala Ala Ser Pro Ala Thr Pro
                   165                 170                 175
   Pro Gln Pro Ala Ser Pro Ala Ala His Ser Asp Lys Thr Pro Ser Pro
               180                 185                 190
<210> 19
   <211> 158
   <212> PRT
   <213> Proteus vulgaris
<400> 19
   Thr Glu His Leu Glu Glu Leu Arg Gln Arg Thr Val Phe Val Phe Ile
   1               5                   10                  15
   Phe Phe Leu Leu Ala Ala Thr Ile Ser Phe Thr Gln Ile Lys Ile Ile
               20                  25                  30
   Val Glu Ile Phe Gln Ala Pro Ala Ile Gly Ile Lys Phe Leu Gln Leu
           35                  40                  45
   Ala Pro Gly Glu Tyr Phe Phe Ser Ser Ile Lys Ile Ala Ile Tyr Cys
       50                  55                  60
   Gly Ile Val Ala Thr Thr Pro Phe Gly Val Tyr Gln Val Ile Leu Tyr
   65                  70                  75                  80
   Ile Leu Pro Gly Leu Thr Asn Lys Glu Arg Lys Val Ile Leu Pro Ile
                   85                  90                  95
   Leu Ile Gly Ser Ile Val Leu Phe Ile Val Gly Gly Ile Phe Ala Tyr
               100                 105                 110
   Phe Val Leu Ala Pro Ala Ala Leu Asn Phe Leu Ile Ser Tyr Gly Ala
           115                 120                 125
   Asp Ile Val Glu Pro Leu Trp Ser Phe Glu Gln Tyr Phe Asp Phe Ile
       130                 135                 140
   Leu Leu Leu Leu Phe Ser Thr Gly Leu Ala Phe Glu Ile Pro
   145                 150                 155
<210> 20
   <211> 168
   <212> PRT
   <213> Marchantia polymorpha
<400> 20
   Lys Thr Ile Leu Glu Glu Val Arg Ile Arg Val Phe Trp Ile Leu Ile
   1 5 10 15
   Cys Phe Ser Phe Thr Trp Phe Thr Cys Tyr Trp Phe Ser Glu Glu Phe
   20 25 30
   Ile Phe Leu Leu Ala Lys Pro Phe Leu Thr Leu Pro Tyr Leu Asp Ser
   35 40 45
   Ser Phe Ile Cys Thr Gln Leu Thr Glu Ala Leu Ser Thr Tyr Val Thr
   50 55 60
   Thr Ser Leu Ile Ser Cys Phe Tyr Phe Leu Phe Pro Phe Leu Ser Tyr
   65 70 75 80
   Gln Ile Trp Cys Phe Leu Met Pro Ser Cys Tyr Glu Glu Gln Arg Lys
   85 90 95
   Lys Tyr Asn Lys Leu Phe Tyr Leu Ser Gly Phe Cys Phe Phe Leu Phe
   100 105 110
   Phe Phe Val Thr Phe Val Trp Ile Val Pro Asn Val Trp His Phe Leu
   115 120 125
   Tyr Lys Leu Ser Thr Thr Ser Thr Asn Leu Leu Ile Ile Lys Leu Gln
   130 135 140
   Pro Lys Ile Phe Asp Tyr Ile Met Leu Thr Val Arg Ile Leu Phe Ile
   145 150 155 160
   Ser Ser Ile Cys Ser Gln Val Pro
   165
<210> 21
   <211> 167
   <212> PRT
   <213> Arabidopsis thaliana
<400> 21
   Glu Thr Ile Leu Gly Glu Val Arg Ile Arg Ser Val Arg Ile Leu Ile
   1 5 10 15
   Gly Leu Gly Leu Thr Trp Phe Thr Cys Tyr Trp Phe Pro Glu Glu Leu
   20 25 30
   Ile Ser Pro Leu Ala Ser Pro Phe Leu Thr Leu Pro Phe Asp Ser Tyr
   35 40 45
   Phe Val Cys Thr Gln Leu Thr Glu Ala Phe Ser Thr Phe Val Ala Thr
   50 55 60
   Ser Ser Ile Ala Cys Ser Tyr Phe Val Phe Pro Leu Ile Ser Tyr Gln
   65 70 75 80
   Ile Trp Cys Phe Leu Ile Pro Ser Cys Tyr Gly Glu Gln Arg Thr Lys
   85 90 95
   Tyr Asn Arg Phe Leu His Leu Ser Gly Ser Arg Phe Phe Leu Phe Leu
   100 105 110
   Phe Leu Thr Pro Pro Arg Val Val Pro Asn Val Trp His Phe Pro Tyr
   115 120 125
   Phe Val Gly Ala Thr Ser Thr Asn Ser Leu Met Ile Lys Leu Gln Pro
   130 135 140
   Lys Ile Tyr Asp His Ile Met Leu Thr Val Arg Ile Ser Phe Ile Pro
   145 150 155 160
   Ser Val Cys Ser Gln Val Pro
   165
<210> 22
   <211> 163
   <212> PRT
   <213> Reclinomonas americana
<400> 22
   Leu Thr His Leu Tyr Glu Ile Arg Leu Arg Ile Ile Tyr Leu Leu Tyr
   1 5 10 15
   Ser Ile Phe Leu Thr Cys Phe Cys Ser Tyr Gln Tyr Lys Glu Glu Ile
   20 25 30
   Phe Tyr Leu Leu Phe Ile Pro Leu Ser Lys Asn Phe Ile Tyr Thr Asp
   35 40 45
   Leu Ile Glu Ala Phe Ile Thr Tyr Ile Lys Leu Ser Ile Ile Val Gly
   50 55 60
   Ile Tyr Leu Ser Tyr Pro Ile Phe Leu Tyr Gln Ile Trp Ser Phe Leu
   65 70 75 80
   Ile Pro Gly Phe Phe Leu Tyr Glu Lys Lys Leu Phe Arg Leu Leu Cys
   85 90 95
   Leu Thr Ser Ile Phe Leu Tyr Phe Leu Gly Ser Cys Ile Gly Tyr Tyr
   100 105 110
   Leu Leu Phe Pro Ile Ala Phe Thr Phe Phe Leu Gly Phe Gln Lys Leu
   115 120 125
   Gly Lys Asp Gln Leu Phe Thr Ile Glu Leu Gln Ala Lys Ile His Glu
   130 135 140
   Tyr Leu Ile Leu Asn Thr Lys Leu Ile Phe Ser Leu Ser Ile Cys Phe
   145 150 155 160
   Gln Leu Pro
<210> 23
   <211> 158
   <212> PRT
   <213> Synechocystis sp.
<400> 23
   Phe Asp His Leu Asp Glu Leu Arg Thr Arg Ile Phe Leu Ser Leu Gly
   1               5                   10                  15
   Ala Val Leu Val Gly Val Val Ala Cys Phe Ile Phe Val Lys Pro Leu
               20                  25                  30
   Val Gln Trp Leu Gln Val Pro Ala Gly Thr Val Lys Phe Leu Gln Leu
           35                  40                  45
   Ser Pro Gly Glu Phe Phe Phe Val Ser Val Lys Val Ala Gly Tyr Ser
       50                  55                  60
   Gly Ile Leu Val Met Ser Pro Phe Ile Leu Tyr Gln Ile Ile Gln Phe
   65                  70                  75                  80
   Val Leu Pro Gly Leu Thr Arg Arg Glu Arg Arg Leu Leu Gly Pro Val
                   85                  90                  95
   Val Leu Gly Ser Ser Val Leu Phe Phe Ala Gly Leu Gly Phe Ala Tyr
               100                 105                 110
   Tyr Ala Leu Ile Pro Ala Ala Leu Lys Phe Phe Val Ser Tyr Gly Ala
           115                 120                 125
   Asp Val Val Glu Gln Leu Trp Ser Ile Asp Lys Tyr Phe Glu Phe Val
       130                 135                 140
   Leu Leu Leu Met Phe Ser Thr Gly Leu Ala Phe Gln Ile Pro
   145                 150                 155
<210> 24
   <211> 178
   <212> PRT
   <213> Mycobacterium tuberculosis
<400> 24
   Val Asp His Leu Thr Glu Leu Arg Thr Arg Leu Leu Ile Ser Leu Ala
    1               5                  10                  15
   Ala Ile Leu Val Thr Thr Ile Phe Gly Phe Val Trp Tyr Ser His Ser
               20                  25                  30
   Ile Phe Gly Leu Asp Ser Leu Gly Glu Trp Leu Arg His Pro Tyr Cys
           35                  40                  45
   Ala Leu Pro Gln Ser Ala Arg Ala Asp Ile Ser Ala Asp Gly Glu Cys
       50                  55                  60
   Arg Leu Leu Ala Thr Ala Pro Phe Asp Gln Phe Met Leu Arg Leu Lys
   65                  70                  75                  80
   Val Gly Met Ala Ala Gly Ile Val Leu Ala Cys Pro Val Trp Phe Tyr
                   85                  90                  95
   Gln Leu Trp Ala Phe Ile Thr Pro Gly Leu Tyr Gln Arg Glu Arg Arg
               100                 105                 110
   Phe Ala Val Ala Phe Val Ile Pro Ala Ala Val Leu Phe Val Ala Gly
           115                 120                 125
   Ala Val Leu Ala Tyr Leu Val Leu Ser Lys Ala Leu Gly Phe Leu Leu
       130                 135                 140
   Thr Val Gly Ser Asp Val Gln Val Thr Ala Leu Ser Gly Asp Arg Tyr
   145                 150                 155                 160
   Phe Gly Phe Leu Leu Asn Leu Leu Val Val Phe Gly Val Ser Phe Glu
                   165                 170                 175
   Phe Pro
<210> 25
   <211> 155
   <212> PRT
   <213> Helicobacter pylori
<400> 25
   His Leu Gln Glu Leu Arg Lys Arg Leu Met Val Ser Val Gly Thr Ile
   1 5 10 15
   Leu Val Ala Phe Leu Gly Cys Phe His Phe Trp Lys Ser Ile Phe Glu
   20 25 30
   Phe Val Lys Asn Ser Tyr Lys Gly Thr Leu Ile Gln Leu Ser Pro Ile
   35 40 45
   Glu Gly Val Met Val Ala Val Lys Ile Ser Phe Ser Ala Ala Ile Val
   50 55 60
   Ile Ser Met Pro Ile Ile Phe Trp Gln Leu Trp Leu Phe Ile Ala Pro
   65 70 75 80
   Gly Leu Tyr Lys Asn Glu Lys Lys Val Ile Leu Pro Phe Val Phe Phe
   85 90 95
   Gly Ser Gly Met Phe Leu Ile Gly Ala Ala Phe Ser Tyr Tyr Val Val
   100 105 110
   Phe Pro Phe Ile Ile Glu Tyr Leu Ala Thr Phe Gly Ser Asp Val Phe
   115 120 125
   Ala Ala Asn Ile Ser Ala Ser Ser Tyr Val Ser Phe Phe Thr Arg Leu
   130 135 140
   Ile Leu Gly Phe Gly Val Ala Phe Glu Leu Pro
   145 150 155
<210> 26
   <211> 163
   <212> PRT
   <213> Haemophilus influenzae
<400> 26
   Ile Thr His Leu Val Glu Leu Arg Asn Arg Leu Leu Arg Cys Val Ile
   1 5 10 15
   Cys Val Val Leu Val Phe Val Ala Leu Val Tyr Phe Ser Asn Asp Ile
   20 25 30
   Tyr His Phe Val Ala Ala Pro Leu Thr Ala Val Met Pro Lys Gly Ala
   35 40 45
   Thr Met Ile Ala Thr Asn Ile Gln Thr Pro Phe Phe Thr Pro Ile Lys
   50 55 60
   Leu Thr Ala Ile Val Ala Ile Phe Ile Ser Val Pro Tyr Leu Leu Tyr
   65 70 75 80
   Gln Ile Trp Ala Phe Ile Ala Pro Ala Leu Tyr Gln His Glu Lys Arg
                   85                       90                       95
   Met Ile Tyr Pro Leu Leu Phe Ser Ser Thr Ile Leu Phe Tyr Cys Gly
               100                 105                 110
   Val Ala Phe Ala Tyr Tyr Ile Val Phe Pro Leu Val Phe Ser Phe Phe
           115                 120                 125
   Thr Gln Thr Ala Pro Glu Gly Val Thr Ile Ala Thr Asp Ile Ser Ser
       130                 135                 140
   Tyr Leu Asp Phe Ala Leu Ala Leu Phe Leu Ala Phe Gly Val Cys Phe
   145                 150                 155                 160
   Glu Val Pro
<210> 27
   <211> 161
   <212> PRT
   <213> Bacillus subtilis
<400> 27
   Leu Glu His Ile Ala Glu Leu Arg Lys Arg Leu Leu Ile Val Ala Leu
   1               5                   10                  15
   Ala Phe Val Val Phe Phe Ile Ala Gly Phe Phe Leu Ala Lys Pro Ile
               20                  25                  30
   Ile Val Tyr Leu Gln Glu Thr Asp Glu Ala Lys Gln Leu Thr Leu Asn
           35                  40                  45
   Ala Phe Asn Leu Thr Asp Pro Leu Tyr Val Phe Met Gln Phe Ala Phe
       50                  55                  60
   Ile Ile Gly Ile Val Leu Thr Ser Pro Val Ile Leu Tyr Gln Leu Trp
   65                  70                  75                  80
   Ala Phe Val Ser Pro Gly Leu Tyr Glu Lys Glu Arg Lys Val Thr Leu
                   85                  90                  95
   Ser Tyr Ile Pro Val Ser Ile Leu Leu Phe Leu Ala Gly Leu Ser Phe
               100                 105                 110
   Ser Tyr Tyr Ile Leu Phe Pro Phe Val Val Asp Phe Met Lys Arg Ile
           115                 120                 125
   Ser Gln Asp Leu Asn Val Asn Gln Val Ile Gly Ile Asn Glu Tyr Phe
       130                 135                 140
   His Phe Leu Leu Gln Leu Thr Ile Pro Phe Gly Leu Leu Phe Gln Met
   145                 150                 155                 160
   Pro
<210> 28
   <211> 163
   <212> PRT
   <213> Azotobacter chroococcum
<400> 28
   Val Ala His Leu Thr Glu Leu Arg Ser Arg Leu Leu Arg Ser Val Ala
   1 5 10 15
   Ala Val Leu Leu Ile Phe Ala Ala Leu Phe Tyr Phe Ala Gln Asp Ile
   20 25 30
   Tyr Ala Leu Val Ser Ala Pro Leu Arg Ala Tyr Leu Pro Glu Gly Ala
   35 40 45
   Thr Met Ile Ala Thr Gly Val Ala Ser Pro Phe Leu Ala Pro Phe Lys
   50 55 60
   Leu Thr Leu Met Ile Ser Leu Phe Leu Ala Met Pro Val Val Leu His
   65 70 75 80
   Gln Val Trp Gly Phe Ile Ala Pro Gly Leu Tyr Gln His Glu Lys Arg
   85 90 95
   Ile Ala Met Pro Leu Met Ala Ser Ser Val Leu Leu Phe Tyr Ala Gly
   100 105 110
   Met Ala Phe Ala Tyr Phe Val Val Phe Pro Ile Met Phe Gly Phe Phe
   115 120 125
   Ala Ser Val Thr Pro Glu Gly Val Ala Met Met Thr Asp Ile Gly Gln
   130 135 140
   Tyr Leu Asp Phe Val Leu Thr Leu Phe Phe Ala Phe Gly Val Ala Phe
   145 150 155 160
   Glu Val Pro
<210> 29
   <211> 204
   <212> PRT
   <213> Archaeoglobus fulgidus
<400> 29
   Ile Ala Leu Ile Val Ile Val Val Ser Ser Leu Phe Phe Thr Phe Gly
   1 5 10 15
   Ala Asn Ile Val Val Gly Lys Ile Ile Gly Asp Leu Phe Pro Gly Glu
   20 25 30
   Ala Val Ile Glu Asn Arg Asp Lys Ile Leu Ala Ile Ala Glu Glu Leu
   35 40 45
   Lys Lys Ile Ala Ser Asp Leu Glu Asn Tyr Ala Tyr His Pro Ser Glu
   50 55 60
   Ala Asn Arg Ser Ile Ala Phe Ala Ala Ser Lys Ser Leu Val Arg Ile
   65 70 75 80
   Ala Met Gln Leu Ser Thr Ser Pro Val Leu Leu Thr Pro Leu Glu Gly
   85 90 95
   Leu Leu Leu Tyr Leu Lys Ile Ser Leu Ala Val Gly Ile Ala Ala Ala
   100 105 110
   Leu Pro Tyr Ile Phe His Leu Val Leu Thr Ala Leu Arg Glu Arg Gly
   115 120 125
   Val Ile Thr Phe Ser Phe Arg Lys Thr Ser Ala Phe Lys Tyr Gly Met
   130 135 140
   Ala Ala Ile Phe Leu Phe Ala Leu Gly Ile Phe Tyr Gly Tyr Asn Met
   145 150 155 160
   Met Lys Phe Phe Ile Lys Phe Leu Tyr Leu Met Ala Val Ser Gln Gly
   165 170 175
   Ala Ile Pro Leu Tyr Ser Leu Ser Glu Phe Val Asn Phe Val Ala Leu
   180 185 190
   Met Leu Val Leu Phe Gly Ile Val Phe Glu Leu Pro
   195 200
<210> 30
   <211> 136
   <212> PRT
   <213> Escherichia coli
<400> 30
   Asp Val Glu Asp Leu Arg Arg Leu Ala Ala Glu Glu Gly Val Val Ala
     1               5                  10                  15
   Leu Gly Glu Thr Gly Leu Asp Tyr Tyr Tyr Thr Pro Glu Thr Lys Val
                20                  25                  30
   Arg Gln Gln Glu Ser Phe Ile His His Ile Gln Ile Gly Arg Glu Leu
            35                  40                  45
   Asn Lys Pro Val Ile Val His Thr Arg Asp Ala Arg Ala Asp Thr Leu
        50                  55                  60
   Ala Ile Leu Arg Glu Glu Lys Val Thr Asp Cys Gly Gly Val Leu His
    65                  70                  75                  80
   Cys Phe Thr Glu Asp Arg Glu Thr Ala Gly Lys Leu Leu Asp Leu Gly
                    85                  90                  95
   Phe Tyr Ile Ser Phe Ser Gly Ile Val Thr Phe Arg Asn Ala Glu Gln
                100                 105                 110
   Leu Arg Asp Ala Ala Arg Tyr Val Pro Leu Asp Arg Leu Leu Val Glu
           115                 120                 125
   Thr Asp Ser Pro Tyr Leu Ala Pro
       130                 135
<210> 31
   <211> 137
   <212> PRT
   <213> Escherichia coli
<400> 31
   Ser Leu Glu Gln Leu Gln Gln Ala Leu Glu Arg Arg Pro Ala Lys Val
   1               5                   10                  15
   Val Ala Val Gly Glu Ile Gly Leu Asp Leu Phe Gly Asp Asp Pro Gln
               20                  25                  30
   Phe Glu Arg Gln Gln Trp Leu Leu Asp Glu Gln Leu Lys Leu Ala Lys
           35                  40                  45
   Arg Tyr Asp Leu Pro Val Ile Leu His Ser Arg Arg Thr His Asp Lys
       50                  55                  60
   Leu Ala Met His Leu Lys Arg His Asp Leu Pro Arg Thr Gly Val Val
   65                  70                  75                  80
   His Gly Phe Ser Gly Ser Leu Gln Gln Ala Glu Arg Phe Val Gln Leu
                   85                  90                  95
   Gly Tyr Lys Ile Gly Val Gly Gly Thr Ile Thr Tyr Pro Arg Ala Ser
               100                 105                 110
   Lys Thr Arg Asp Val Ile Ala Lys Leu Pro Leu Ala Ser Leu Leu Leu
           115                 120                 125
   Glu Thr Asp Ala Pro Asp Met Pro Leu
       130                 135
<210> 32
   <211> 135
   <212> PRT
   <213> Methanobacterium thermoautotrophicum
<400> 32
   Leu Ile Gly Glu Val Val Ser Gln Ile Glu Ser Asn Ile Asp Leu Ile
   1               5                   10                  15
   Val Ala Val Gly Glu Thr Gly Met Asp Phe His His Thr Arg Asp Glu
               20                  25                  30
   Glu Gly Arg Arg Arg Gln Glu Glu Thr Phe Arg Val Phe Val Glu Leu
   35 40 45
   Ala Ala Glu His Glu Met Pro Leu Val Val His Ala Arg Asp Ala Glu
   50 55 60
   Glu Arg Ala Leu Glu Thr Val Leu Glu Tyr Arg Val Pro Glu Val Ile
   65 70 75 80
   Phe His Cys Tyr Gly Gly Ser Ile Glu Thr Ala Arg Arg Ile Leu Asp
   85 90 95
   Glu Gly Tyr Tyr Ile Ser Ile Ser Thr Leu Val Ala Phe Ser Glu His
   100 105 110
   His Met Glu Leu Val Arg Ala Ile Pro Leu Glu Gly Met Leu Thr Glu
   115 120 125
   Thr Asp Ser Pro Tyr Leu Ser
   130 135
<210> 33
   <211> 142
   <212> PRT
   <213> Mycoplasma pneumoniae
<400> 33
   Ala Gln Ala Thr Leu Lys Lys Leu Val Ser Thr His Arg Ser Phe Ile
   1 5 10 15
   Ser Cys Ile Gly Glu Tyr Gly Phe Asp Tyr His Tyr Thr Lys Asp Tyr
   20 25 30
   Ile Thr Gln Gln Glu Gln Phe Phe Leu Met Gln Phe Gln Leu Ala Glu
   35 40 45
   Gln Tyr Gln Leu Val His Met Leu His Val Arg Asp Val His Glu Arg
   50 55 60
   Ile Tyr Glu Val Leu Lys Arg Leu Lys Pro Lys Gln Pro Val Val Phe
   65 70 75 80
   His Cys Phe Ser Glu Asp Thr Asn Thr Ala Leu Lys Leu Leu Thr Leu
   85 90 95
   Arg Glu Val Gly Leu Lys Val Tyr Phe Ser Ile Pro Gly Ile Val Thr
   100 105 110
   Phe Lys Asn Ala Lys Asn Leu Gln Ala Ala Leu Ser Val Ile Pro Thr
   115 120 125
   Glu Leu Leu Leu Ser Glu Thr Asp Ser Pro Tyr Leu Ala Pro
   130 135 140
<210> 34
   <211> 140
   <212> PRT
   <213> Mycobacterium tuberculosis
<400> 34
   Ala Arg Ala Glu Leu Glu Arg Leu Val Ala His Pro Arg Val Val Ala
   1               5                   10                  15
   Val Gly Glu Thr Gly Ile Asp Met Tyr Trp Pro Gly Arg Leu Asp Gly
               20                  25                  30
   Cys Ala Glu Pro His Val Gln Arg Glu Ala Phe Ala Trp His Ile Asp
           35                  40                  45
   Leu Ala Lys Arg Thr Gly Lys Pro Leu Met Ile His Asn Arg Gln Ala
       50                  55                  60
   Asp Arg Asp Val Leu Asp Val Leu Arg Ala Glu Gly Ala Pro Asp Thr
   65                  70                  75                  80
   Val Ile Leu His Cys Phe Ser Ser Asp Ala Ala Met Ala Arg Thr Cys
                   85                  90                  95
   Val Asp Ala Gly Trp Leu Leu Ser Leu Ser Gly Thr Val Ser Phe Arg
               100                 105                 110
   Thr Ala Arg Glu Leu Arg Glu Ala Val Pro Leu Met Pro Val Glu Gln
           115                 120                 125
   Leu Leu Val Glu Thr Asp Ala Pro Tyr Leu Thr Pro
       130                 135                 140
<210> 35
   <211> 138
   <212> PRT
   <213> Helicobacter pylori
<400> 35
   Asp Glu Ser Leu Phe Glu Lys Phe Val Gly His Gln Lys Cys Val Ala
   1               5                   10                  15
   Ile Gly Glu Cys Gly Leu Asp Tyr Tyr Arg Leu Pro Glu Leu Asn Glu
               20                  25                  30
   Arg Glu Asn Tyr Lys Ser Lys Gln Lys Glu Ile Phe Thr Lys Gln Ile
           35                  40                  45
   Glu Phe Ser Ile Gln His Asn Lys Pro Leu Ile Ile His Ile Arg Glu
       50                  55                  60
   Ala Ser Phe Asp Ser Leu Asn Leu Leu Lys Asn Tyr Pro Lys Ala Phe
   65                  70                  75                  80
   Gly Val Leu His Cys Phe Asn Ala Asp Gly Met Leu Leu Glu Leu Ser
                   85                  90                  95
   Asp Arg Phe Tyr Tyr Gly Ile Gly Gly Val Ser Thr Phe Lys Asn Ala
               100                 105                 110
   Lys Arg Leu Val Glu Ile Leu Pro Lys Ile Pro Lys Asn Arg Leu Leu
           115                 120                 125
   Leu Glu Thr Asp Ser Pro Tyr Leu Thr Pro
       130                 135
<210> 36
   <211> 136
   <212> PRT
   <213> Haemophilus influenzae
<400> 36
   Asp Ala Glu Arg Leu Leu Arg Leu Ala Gln Asp Pro Lys Val Ile Ala
   1 5 10 15
   Ile Gly Glu Ile Gly Leu Asp Tyr Tyr Tyr Ser Ala Asp Asn Lys Ala
   20 25 30
   Ala Gln Gln Ala Val Phe Gly Ser Gln Ile Asp Ile Ala Asn Gln Leu
   35 40 45
   Asp Lys Pro Val Ile Ile His Thr Arg Ser Ala Gly Asp Asp Thr Ile
   50 55 60
   Ala Met Leu Arg Gln His Arg Ala Glu Lys Cys Gly Gly Val Ile His
   65 70 75 80
   Cys Phe Thr Glu Thr Met Glu Phe Xaa Lys Lys Ala Leu Asp Leu Gly
   85 90 95
   Phe Tyr Ile Ser Cys Ser Gly Ile Val Thr Phe Lys Asn Ala Glu Ala
   100 105 110
   Ile Arg Glu Val Ile Arg Tyr Val Pro Met Glu Arg Leu Leu Val Glu
   115 120 125
   Thr Asp Ser Pro Tyr Leu Ala Pro
   130 135
<210> 37
   <211> 136
   <212> PRT
   <213> Bacillus subtilis
<400> 37
   Asp Leu Ala Trp Ile Lys Glu Leu Ser Ala His Glu Lys Val Val Ala
   1 5 10 15
   Ile Gly Glu Met Gly Leu Asp Tyr His Trp Asp Lys Ser Pro Lys Asp
   20 25 30
   Ile Gln Lys Glu Val Phe Arg Asn Gln Ile Ala Leu Ala Lys Glu Val
   35 40 45
   Asn Leu Pro Ile Ile Ile His Asn Arg Asp Ala Thr Glu Asp Val Val
   50 55 60
   Thr Ile Leu Lys Glu Glu Gly Ala Glu Ala Val Gly Gly Ile Met His
   65 70 75 80
   Cys Phe Thr Gly Ser Ala Glu Val Ala Arg Glu Cys Met Lys Met Asn
   85 90 95
   Phe Tyr Leu Ser Phe Gly Gly Pro Val Thr Phe Lys Asn Ala Lys Lys
   100 105 110
   Pro Lys Glu Val Val Lys Glu Ile Pro Asn Asp Arg Leu Leu Ile Glu
   115 120 125
   Thr Asp Cys Pro Phe Leu Thr Pro
   130 135
<210> 38
   <211> 135
   <212> PRT
   <213> Schizosaccharomyces pombe
<400> 38
   Glu Ala Leu Ala Asn Lys Gly Lys Ala Ser Gly Lys Val Val Ala Phe
   1               5                   10                  15
   Gly Glu Phe Gly Leu Asp Tyr Asp Arg Leu His Tyr Ala Pro Ala Asp
               20                  25                  30
   Val Gln Lys Met Tyr Phe Glu Glu Gln Leu Lys Val Ala Val Arg Val
           35                  40                  45
   Gln Leu Pro Leu Phe Leu His Ser Arg Asn Ala Glu Asn Asp Phe Phe
       50                  55                  60
   Ala Ile Leu Glu Lys Tyr Leu Pro Glu Leu Pro Lys Lys Gly Val Val
   65                  70                  75                  80
   His Ser Phe Thr Gly Ser Ile Asp Glu Met Arg Arg Cys Ile Glu His
                   85                  90                  95
   Gly Leu Tyr Val Gly Val Asn Gly Cys Ser Leu Lys Thr Glu Glu Asn
               100                 105                 110
   Leu Glu Val Val Arg Ala Ile Pro Leu Glu Lys Met Leu Leu Glu Thr
           115                 120                 125
   Asp Ala Pro Trp Cys Glu Val
       130                 135
<210> 39
   <211> 149
   <212> PRT
   <213> Caenorhabditis elegans
<400> 39
   His Ile Ser Lys Met Glu Gln Phe Phe Val Glu His Glu Arg Asp Ile
   1 5 10 15
   Ile Cys Val Gly Glu Cys Gly Leu Asp His Thr Ile Ser Gln Phe Lys
   20 25 30
   Leu Thr Thr Glu Asp Phe Glu Glu Gln Glu Thr Val Phe Lys Trp Gln
   35 40 45
   Ile Asp Leu Ala Lys His Phe Glu Lys Pro Leu Ile Leu Glu Ile Pro
   50 55 60
   Asp Ile Ser Arg Asn Val His Ser Arg Ser Ala Ala Arg Arg Thr Ile
   65 70 75 80
   Glu Ile Leu Leu Glu Cys His Val Ala Pro Asp Gln Val Val Leu His
   85 90 95
   Ala Phe Asp Gly Thr Pro Gly Asp Leu Lys Leu Gly Leu Glu Ala Gly
   100 105 110
   Tyr Leu Phe Ser Ile Pro Pro Ser Phe Gly Lys Ser Glu Glu Thr Thr
   115 120 125
   Gln Leu Ile Glu Ser Ile Pro Leu Ser Gln Leu Leu Leu Glu Thr Asp
   130 135 140
   Ser Pro Ala Leu Gly
   145
<210> 40
   <211> 139
   <212> PRT
   <213> Homo sapiens
<400> 40
   Gln Glu Arg Asn Leu Leu Gln Ala Leu Arg His Pro Lys Ala Val Ala
   1 5 10 15
   Phe Gly Glu Met Gly Leu Asp Tyr Ser Tyr Lys Cys Thr Thr Pro Val
   20 25 30
   Pro Glu Gln His Lys Val Phe Glu Arg Gln Leu Gln Leu Ala Val Ser
   35 40 45
   Leu Lys Lys Pro Leu Val Ile His Cys Arg Glu Ala Asp Glu Asp Leu
   50 55 60
   Leu Glu Ile Met Lys Lys Phe Val Pro Pro Asp Tyr Lys Ile His Arg
   65 70 75 80
   His Cys Phe Thr Gly Ser Tyr Pro Val Ile Glu Pro Leu Leu Lys Tyr
   85 90 95
   Phe Pro Asn Met Ser Val Gly Phe Thr Ala Val Leu Thr Tyr Ser Ser
   100 105 110
   Ala Trp Glu Ala Arg Glu Ala Leu Arg Gln Ile Pro Leu Glu Arg Ile
   115 120 125
   Ile Val Glu Thr Asp Ala Pro Tyr Phe Leu Pro
   130 135
<210> 41
   <211> 7
   <212> PRT
   <213> Artificial Sequence
<220>
   <223> Description of Artificial Sequence: synthetic - generic organism.
<400> 41
   Ser Arg Arg Ser Phe Leu Lys
   1 5
<210> 42
   <211> 7
   <212> PRT
   <213> Artificial Sequence
<220>
   <223> Description of Artificial Sequence: synthetic - generic organism
<400> 42
   Thr Arg Arg Ser Phe Leu Lys
   1 5
<210> 43
   <211> 50
   <212> PRT
   <213> Artificial Sequence
<220>
   <223> Description of Artificial Sequence: synthetic
<400> 43
   Met Lys Thr Lys Ile Pro Asp Ala Val Leu Ala Ala Glu Val Ser Arg
   1 5 10 15
   Arg Gly Leu Val Lys Thr Thr Ile Ala Phe Phe Leu Ala Met Ala Ser
   20 25 30
   Ser Ala Leu Thr Leu Pro Phe Ser Arg Ile Ala His Ala Val Asp Ser
   35 40 45
   Ala Ile
   50
<210> 44
   <211> 30
   <212> DNA
   <213> Artificial Sequence
<220>
   <223> Description of Artificial Sequence: synthetic
<400> 44
   ttagtcggat taatcacaat gtcgatagcg 30
<210> 45
   <211> 3120
   <212> DNA
   <213> Artificial Sequence
<220>
   <223> Description of Artificial Sequence: synthetic
<400> 45
   attctggctg ggtgccacca gataccaacg ttgaagagtt cgaatttgcc attcgtacgg 60
   tctgtgaacc tatctttgag aaaccgctgg ccgaaatttc gtttggacat gtactgttaa 120
   atctgtttaa tacggcgcgt cgcttcaata tggaagtgca gccgcaactg gtgttactcc 180
   agaaaaccct gctctacgtc gaaggggtag gacgccagct ttatccgcaa ctcgatttat 240
   ggaaaacggc gaagcctttc ctggagtcgt ggattaaaga tcaggtcggt attcctgcgc 300
   tggtgagagc atttaaagaa aaagcgccgt tctgggtcga aaaaatgcca gaactgcctg 360
   aattggttta cgacagtttg cgccagggca agtatttaca gcacagtgtt gataagattg 420
   cccgcgagct tcagtcaaat catgtacgtc agggacaatc gcgttatttt ctcggaattg 480
   gcgctacgtt agtattaagt ggcacattct tgttggtcag ccgacctgaa tgggggctga 540
   tgcccggctg gttaatggca ggtggtctga tcgcctggtt tgtcggttgg cgcaaaacac 600
   gctgattttt tcatcgctca aggcgggccg tgtaacgtat aatgcggctt tgtttaatca 660
   tcatctacca cagaggaaca tgtatgggtg gtatcagtat ttggcagtta ttgattattg 720
   ccgtcatcgt tgtactgctt tttggcacca aaaagctcgg ctccatcggt tccgatcttg 780
   gtgcgtcgat caaaggcttt aaaaaagcaa tgagcgatga tgaaccaaag caggataaaa 840
   ccagtcagga tgctgatttt actgcgaaaa ctatcgccga taagcaggcg gatacgaatc 900
   aggaacaggc taaaacagaa gacgcgaagc gccacgataa agagcaggtg taatccgtgt 960
   ttgatatcgg ttttagcgaa ctgctattgg tgttcatcat cggcctcgtc gttctggggc 1020
   cgcaacgact gcctgtggcg gtaaaaacgg tagcgggctg gattcgcgcg ttgcgttcac 1080
   tggcgacaac ggtgcagaac gaactgaccc aggagttaaa actccaggag tttcaggaca 1140
   gtctgaaaaa ggttgaaaag gcgagcctca ctaacctgac gcccgaactg aaagcgtcga 1200
   tggatgaact acgccaggcc gcggagtcga tgaagcgttc ctacgttgca aacgatcctg 1260
   aaaaggcgag cgatgaagcg cacaccatcc ataacccggt ggtgaaagat aatgaagctg 1320
   cgcatgaggg cgtaacgcct gccgctgcac aaacgcaggc cagttcgccg gaacagaagc 1380
   cagaaaccac gccagagccg gtggtaaaac ctgctgcgga cgctgaaccg aaaaccgctg 1440
   caccttcccc ttcgtcgagt gataaaccgt aaacatgtct gtagaagata ctcaaccgct 1500
   tatcacgcat ctgattgagc tgcgtaagcg tctgctgaac tgcattatcg cggtgatcgt 1560
   gatattcctg tgtctggtct atttcgccaa tgacatctat cacctggtat ccgcgccatt 1620
   gatcaagcag ttgccgcaag gttcaacgat gatcgccacc gacgtggcct cgccgttctt 1680
   tacgccgatc aagctgacct ttatggtgtc gctgattctg tcagcgccgg tgattctcta 1740
   tcaggtgtgg gcatttatcg ccccagcgct gtataagcat gaacgtcgcc tggtggtgcc 1800
   gctgctggtt tccagctctc tgctgtttta tatcggcatg gcattcgcct actttgtggt 1860
   ctttccgctg gcatttggct tccttgccaa taccgcgccg gaaggggtgc aggtatccac 1920
   cgacatcgcc agctatttaa gcttcgttat ggcgctgttt atggcgtttg gtgtctcctt 1980
   tgaagtgccg gtagcaattg tgctgctgtg ctggatgggg attacctcgc cagaagactt 2040
   acgcaaaaaa cgcccgtatg tgctggttgg tgcattcgtt gtcgggatgt tgctgacgcc 2100
   gccggatgtc ttctcgcaaa cgctgttggc gatcccgatg tactgtctgt ttgaaatcgg 2160
   tgtcttcttc tcacgctttt acgttggtaa agggcgaaat cgggaagagg aaaacgacgc 2220
   tgaagcagaa agcgaaaaaa ctgaagaata aattcaaccg cccgtcaggg cggttgtcat 2280
   atggagtaca ggatgtttga tatcggcgtt aatttgacca gttcgcaatt tgcgaaagac 2340
   cgtgatgatg ttgtagcgtg cgcttttgac gcgggagtta atgggctact catcaccggc 2400
   actaacctgc gtgaaagcca gcaggcgcaa aagctggcgc gtcagtattc gtcctgttgg 2460
   tcaacggcgg gcgtacatcc tcacgacagc agccagtggc aagctgcgac tgaagaagcg 2520
   attattgagc tggccgcgca gccagaagtg gtggcgattg gtgaatgtgg tctcgacttt 2580
   aaccgcaact tttcgacgcc ggaagagcag gaacgcgctt ttgttgccca gctacgcatt 2640
   gccgcagatt taaacatgcc ggtatttatg cactgtcgcg atgcccacga gcggtttatg 2700
   acattgctgg agccgtggct ggataaactg cctggtgcgg ttcttcattg ctttaccggc 2760
   acacgcgaag agatgcaggc gtgcgtggcg catggaattt atatcggcat taccggttgg 2820
   gtttgcgatg aacgacgcgg actggagctg cgggaacttt tgccgttgat tccggcggaa 2880
   aaattactga tcgaaactga tgcgccgtat ctgctccctc gcgatctcac gccaaagcca 2940
   tcatcccggc gcaacgagcc agcccatctg ccccatattt tgcaacgtat tgcgcactgg 3000
   cgtggagaag atgccgcatg gctggctgcc accacggatg ctaatgccaa aacactgttt 3060
   gggattgcgt tttagagttt gcggaactcg gtattcttca cactgtgctt aatctcttta 3120
<210> 46
   <211> 312
   <212> DNA
   <213> Artificial Sequence
<220>
   <223> Description of Artificial Sequence: synthetic
<400> 46
   atgcggcttt gtttaatcat catctaccac agaggaacat gtatgggtgg tatcagtatt 60
   tggcagttat tgattattgc cgtcatcgtt gtactgcttt ttggcaccaa aaagctcggc 120
   tccatcggtt ccgatcttgg tgcgtcgatc aaaggcttta aaaaagcaat gagcgatgat 180
   gaaccaaagc aggataaaac cagtcaggat gctgatttta ctgcgaaaac tatcgccgat 240
   aagcaggcgg atacgaatca ggaacaggct aaaacagaag acgcgaagcg ccacgataaa 300
   gagcaggtgt aa                                                     312
<210> 47
   <211> 103
   <212> PRT
   <213> Artificial Sequence
<220>
   <223> Description of Artificial Sequence: synthetic
<400> 47
   Met Arg Leu Cys Leu Ile Ile Ile Tyr His Arg Gly Thr Cys Met Gly
    1               5                  10                  15
   Gly Ile Ser Ile Trp Gln Leu Leu Ile Ile Ala Val Ile Val Val Leu
               20                  25                  30
   Leu Phe Gly Thr Lys Lys Leu Gly Ser Ile Gly Ser Asp Leu Gly Ala
           35                  40                  45
   Ser Ile Lys Gly Phe Lys Lys Ala Met Ser Asp Asp Glu Pro Lys Gln
       50                  55                  60
   Asp Lys Thr Ser Gln Asp Ala Asp Phe Thr Ala Lys Thr Ile Ala Asp
   65                  70                  75                  80
   Lys Gln Ala Asp Thr Asn Gln Glu Gln Ala Lys Thr Glu Asp Ala Lys
                   85                  90                  95
   Arg His Asp Lys Glu Gln Val
               100
<210> 48
   <211> 515
   <212> DNA
   <213> Artificial Sequence
<220>
   <223> Description of Artificial Sequence: synthetic
<400> 48
   tgtttgatat cggttttagc gaactgctat tggtgttcat catcggcctc gtcgttctgg 60
   ggccgcaacg actgcctgtg gcggtaaaaa cggtagcggg ctggattcgc gcgttgcgtt 120
   cactggcgac aacggtgcag aacgaactga cccaggagtt aaaactccag gagtttcagg 180
   acagtctgaa aaaggttgaa aaggcgagcc tcactaacct gacgcccgaa ctgaaagcgt 240
   cgatggatga actacgccag gccgcggagt cgatgaagcg ttcctacgtt gcaaacgatc 300
   ctgaaaaggc gagcgatgaa gcgcacacca tccataaccc ggtggtgaaa gataatgaag 360
   ctgcgcatga gggcgtaacg cctgccgctg cacaaacgca ggccagttcg ccggaacaga 420
   agccagaaac cacgccagag ccggtggtaa aacctgctgc ggacgctgaa ccgaaaaccg 480
   ctgcaccttc cccttcgtcg agtgataaac cgtaa                            515
<210> 49
   <211> 161
   <212> PRT
   <213> Artificial Sequence
<220>
   <223> Description of Artificial Sequence: synthetic
<400> 49
   Val Phe Asp Ile Gly Phe Ser Glu Leu Leu Leu Val Phe Ile Ile Gly
    1               5                  10                  15
   Leu Val Val Leu Gly Pro Gln Arg Leu Pro Val Ala Val Lys Thr Val
               20                  25                  30
   Ala Gly Trp Ile Arg Ala Leu Arg Ser Leu Ala Thr Thr Val Gln Asn
           35                  40                  45
   Glu Leu Thr Gln Glu Leu Lys Leu Gln Glu Phe Gln Asp Ser Leu Lys
       50                  55                  60
   Lys Val Glu Lys Ala Ser Leu Thr Asn Leu Thr Pro Glu Leu Lys Ala
   65 70 75 80
   Ser Met Asp Glu Leu Arg Gln Ala Ala Glu Ser Met Lys Arg Ser Tyr
   85 90 95
   Val Ala Asn Asp Pro Glu Lys Ala Ser Asp Glu Ala His Thr Ile His
   100 105 110
   Asn Pro Val Val Lys Asp Asn Glu Ala Ala His Glu Gly Val Thr Pro
   115 120 125
   Ala Ala Ala Gln Thr Gln Ala Ser Ser Pro Glu Gln Lys Pro Glu Thr
   130 135 140
   Thr Pro Glu Pro Val Val Lys Pro Ala Ala Asp Ala Glu Pro Lys Thr
   145 150 155 160
   Ala